(19) Europäisches Patentamt
European Patent Office
Office européen des brevets

(11) **EP 0 970 199 B1**

(12) **EUROPEAN PATENT SPECIFICATION**

(45) Date of publication and mention
of the grant of the patent:
**22.10.2008 Bulletin 2008/43**

(51) Int Cl.:
*C12N 15/00* (2006.01)    *C12N 15/09* (2006.01)
*C07H 21/02* (2006.01)    *C07H 21/04* (2006.01)

(21) Application number: **98909178.0**

(22) Date of filing: **13.03.1998**

(86) International application number:
**PCT/US1998/004978**

(87) International publication number:
**WO 1998/040477 (17.09.1998 Gazette 1998/37)**

(54) **FLUORESCENT PROTEIN SENSORS FOR DETECTION OF ANALYTES**

FLUORESZENZPROTEIN-SENSOREN ZUR ERKENNUNG VON ANALYTEN

DETECTEURS DE PROTEINES FLUORESCENTES PERMETTANT DE DETECTER DES
SUBSTANCES A ANALYSER

(84) Designated Contracting States:
**AT BE CH DE DK ES FI FR GB GR IE IT LI LU MC
NL PT SE**

(30) Priority: **14.03.1997 US 818252
14.03.1997 US 818253
27.08.1997 US 919143**

(43) Date of publication of application:
**12.01.2000 Bulletin 2000/02**

(73) Proprietor: **The Regents of the University
of California
Oakland, CA 94607-5200 (US)**

(72) Inventors:
• **TSIEN, Roger, Y.
La Jolla, CA 92037 (US)**

• **MIYAWAKI, Atsushi
San Diego, CA 92122 (US)**

(74) Representative: **Vossius, Volker et al
Dr. Volker Vossius
Patent- und Rechtsanwaltskanzlei
Geibelstrasse 6
81679 München (DE)**

(56) References cited:
WO-A-98/02571      WO-A-98/06737
US-A- 4 868 103      US-A- 5 134 232
US-A- 5 439 797

• DATABASE EMBL/GENBANK/DDBJ [Online]
CORMACK, B.P. ET AL.: "Cloning vector pEGFP-
N2 with enhanced green fluorescent protein
gene, complete sequence." retrieved from EBI
Database accession no. CV57608 XP002243579

EP 0 970 199 B1

SEQUENCE LISTING

    (1) GENERAL INFORMATION:

        (i) APPLICANT: The Regents of the University of California

        (ii) TITLE OF INVENTION: Fluorescent Protein Sensors for Detection of Analyates

        (iii) NUMBER OF SEQUENCES: 8

        (iv) CORRESPONDENCE ADDRESS:

            (A) ADDRESSEE: Fish & Richardson P.C.
            (B) STREET: 4225 Executive Square, Suite 1400
            (C) CITY: La Jolla
            (D) STATE: CA
            (E) COUNTRY: USA
            (F) ZIP: 92037

        (v) COMPUTER READABLE FORM:

            (A) MEDIUM TYPE: Floppy disk
            (B) COMPUTER: IBM PC compatible
            (C) OPERATING SYSTEM: PC-DOS/MS-DOS
            (D) SOFTWARE: PatentIn Release #1.0, Version #1.30

        (vii) PRIOR APPLICATION DATA:

            (A) APPLICATION NUMBER: US 08/818,252
            (B) FILING DATE: 14 March 1997
            (C) CLASSIFICATION:

        (vii) PRIOR APPLICATION DATA:

            (A) APPLICATION NUMBER US 08/818,253
            (B) FILING DATE: 14 MARCH 1997
            (C) CLASSIFICATION:

        (vii) PRIOR APPLICATION DATA:

            (A) APPLICATION NUMBER: US 08/919,143
            (B) FILING DATE: 27 AUGUST 1997
            (C) CLASSIFICATION:

(viii) ATTORNEY/AGENT INFORMATION:

    (A) NAME: Haile, Lisa A.
    (B) REGISTRATION NUMBER: 38,347
    (C) REFERENCE/DOCKET NUMBER: 07257/058WO1

(ix) TELECOMMUNICATION INFORMATION:

    (A) TELEPHONE: 619/678-5070
    (B) TELEFAX: 619/678-5099

(2) INFORMATION FOR SEQ ID NO:1:

    (i) SEQUENCE CHARACTERISTICS

        (A) LENGTH: 1929 BASE PAIRS
        (B) TYPE: NUCLEIC ACID
        (C) STRANDEDNESS: SINGLE
        (D) TOPOLOGY: LINEAR

    (ii) MOLECULE TYPE: cDNA

    (xi) SEQUENCE DESCRIPTION: SEQ ID NO:1:

```
ATG GTG AGC AAG GGC GAG GAG CTG TTC ACC GGG GTG GTG CCC ATC CTG   48
Met Val Ser Lys Gly Glu Glu Leu Phe Thr Gly Val Val Pro Ile Leu
                    5                   10                  15
```

```
GTC GAG CTG GAC GGC GAC GTA AAC GGC CAC AAG TTC AGC GTG TCC GGC      96
Val Glu Leu Asp Gly Asp Val Asn Gly His Lys Phe Ser Val Ser Gly
            20                    25                    30

GAG GGC GAG GGC GAT GCC ACC TAC GGC AAG CTG ACC CTG AAG TTC ATC     144
Glu Gly Glu Gly Asp Ala Thr Tyr Gly Lys Leu Thr Leu Lys Phe Ile
            35                    40                    45

TGC ACC ACC GGC AAG CTG CCC GTG CCC TGG CCC ACC CTC GTG ACC ACC     192
Cys Thr Thr Gly Lys Leu Pro Val Pro Trp Pro Thr Leu Val Thr Thr
            50                    55                    60

CTG ACC CAT GGC GTG CAG TGC TTC AGC CGC TAC CCC GAC CAC ATG AAG     240
Leu Thr His Gly Val Gln Cys Phe Ser Arg Tyr Pro Asp His Met Lys
65                    70                    75                    80

CAG CAC GAC TTC TTC AAG TCC GCC ATG CCC GAA GGC TAC GTC CAG GAG     288
Gln His Asp Phe Phe Lys Ser Ala Met Pro Glu Gly Tyr Val Gln Glu
                  85                    90                    95

CGC ACC ATC TTC TTC AAG GAC GAC GGC AAC TAC AAG ACC CGC GCC GAG     336
Arg Thr Ile Phe Phe Lys Asp Asp Gly Asn Tyr Lys Thr Arg Ala Glu
                  100                   105                   110

GTG AAG TTC GAG GGC GAC ACC CTG GTG AAC CGC ATC GAG CTG AAG GGC     384
Val Lys Phe Glu Gly Asp Thr Leu Val Asn Arg Ile Glu Leu Lys Gly
            115                   120                   125

ATC GAC TTC AAG GAG GAC GGC AAC ATC CTG GGG CAC AAG CTG GAG TAC     432
Ile Asp Phe Lys Glu Asp Gly Asn Ile Leu Gly His Lys Leu Glu Tyr
            130                   135                   140

AAC TTC AAC AGC CAC AAC GTC TAT ATC ATG GCC GAC AAG CAG AAG AAC     480
Asn Phe Asn Ser His Asn Val Tyr Ile Met Ala Asp Lys Gln Lys Asn
145                   150                   155                   160

GGC ATC AAG GTG AAC TTC AAG ATC CGC CAC AAC ATC GAG GAC GGC AGC     528
Gly Ile Lys Val Asn Phe Lys Ile Arg His Asn Ile Glu Asp Gly Ser
                  165                   170                   175

GTG CAG CTC GCC GAC CAC TAC CAG CAG AAC ACC CCC ATC GGC GAC GGC     576
Val Gln Leu Ala Asp His Tyr Gln Gln Asn Thr Pro Ile Gly Asp Gly
            180                   185                   190

CCC GTG CTG CTG CCC GAC AAC CAC TAC CTG AGC ACC CAG TCC GCC CTG     624
Pro Val Leu Leu Pro Asp Asn His Tyr Leu Ser Thr Gln Ser Ala Leu
            195                   200                   205

AGC AAA GAC CCC AAC GAG AAG CGC GAT CAC ATG GTC CTG CTG GAG TTC     672
Ser Lys Asp Pro Asn Glu Lys Arg Asp His Met Val Leu Leu Glu Phe
            210                   215                   220

GTG ACC GCC GCC CGC ATG CAT GAC CAA CTG ACA GAA GAG CAG ATT GCA     720
Val Thr Ala Ala Arg Met His Asp Gln Leu Thr Glu Glu Gln Ile Ala
225                   230                   235                   240

GAG TTC AAA GAA GCC TTC TCA TTA TTC GAC AAG GAT GGG GAC GGC ACC     768
Glu Phe Lys Glu Ala Phe Ser Leu Phe Asp Lys Asp Gly Asp Gly Thr
                  245                   250                   255

ATC ACC ACA AAG GAA CTT GGC ACC GTT ATG AGG TCG CTT GGA CAA AAC     816
Ile Thr Thr Lys Glu Leu Gly Thr Val Met Arg Ser Leu Gly Gln Asn
            260                   265                   270
```

```
CCA ACG GAA GCA GAA TTG CAG GAT ATG ATC AAT GAA GTC GAT GCT GAT    864
Pro Thr Glu Ala Glu Leu Gln Asp Met Ile Asn Glu Val Asp Ala Asp
        275                 280                 285

GGC AAT GGA ACG ATT TAC TTT CCT GAA TTT CTT ACT ATG ATG GCT AGA    912
Gly Asn Gly Thr Ile Tyr Phe Pro Glu Phe Leu Thr Met Met Ala Arg
        290                 295                 300

AAA ATG AAG GAC ACA GAC AGC GAA GAG GAA ATC CGA GAA GCA TTC CGT    960
Lys Met Lys Asp Thr Asp Ser Glu Glu Glu Ile Arg Glu Ala Phe Arg
305                 310                 315                 320

GTT TTT GAC AAG GAT GGG AAC GGC TAC ATC AGC GCT GCT GAA TTA CGT   1008
Val Phe Asp Lys Asp Gly Asn Gly Tyr Ile Ser Ala Ala Glu Leu Arg
                325                 330                 335

CAC GTC ATG ACA AAC CTC GGG GAG AAG TTA ACA GAT GAA GAA GTT GAT   1056
His Val Met Thr Asn Leu Gly Glu Lys Leu Thr Asp Glu Glu Val Asp
                340                 345                 350

GAA ATG ATA AGG GAA GCA GAT ATC GAT GGT GAT GGC CAA GTA AAC TAT   1104
Glu Met Ile Arg Glu Ala Asp Ile Asp Gly Asp Gly Gln Val Asn Tyr
                355                 360                 365

GAA GAG TTT GTA CAA ATG ATG ACA GCA AAG GGG GGG AAG AGG CGC TGG   1152
Glu Glu Phe Val Gln Met Met Thr Ala Lys Gly Gly Lys Arg Arg Trp
        370                 375                 380

AAG AAA AAC TTC ATT GCC GTC AGC GCT GCC AAC CGG TTC AAG AAG ATC   1200
Lys Lys Asn Phe Ile Ala Val Ser Ala Ala Asn Arg Phe Lys Lys Ile
385                 390                 395                 400

TCC GAG CTC ATG GTG AGC AAG GGC GAG GAG CTG TTC ACC GGG GTG GTG   1248
Ser Glu Leu Met Val Ser Lys Gly Glu Glu Leu Phe Thr Gly Val Val
                405                 410                 415

CCC ATC CTG GTC GAG CTG GAC GGC GAC GTA AAC GGC CAC AAG TTC AGC   1296
Pro Ile Leu Val Glu Leu Asp Gly Asp Val Asn Gly His Lys Phe Ser
                420                 425                 430

GTG TCC GGC GAG GGC GAG GGC GAT GCC ACC TAC GGC AAG CTG ACC CTG   1344
Val Ser Gly Glu Gly Glu Gly Asp Ala Thr Tyr Gly Lys Leu Thr Leu
                435                 440                 445

AAG TTC ATC TGC ACC ACC GGC AAG CTG CCC GTG CCC TGG CCC ACC CTC   1392
Lys Phe Ile Cys Thr Thr Gly Lys Leu Pro Val Pro Trp Pro Thr Leu
        450                 455                 460

GTG ACC ACC CTG ACC TAC GGC GTG CAG TGC TTC AGC CGC TAC CCC GAC   1440
Val Thr Thr Leu Thr Tyr Gly Val Gln Cys Phe Ser Arg Tyr Pro Asp
465                 470                 475                 480

CAC ATG AAG CAG CAC GAC TTC TTC AAG TCC GCC ATG CCC GAA GGC TAC   1488
His Met Lys Gln His Asp Phe Phe Lys Ser Ala Met Pro Glu Gly Tyr
                485                 490                 495

GTC CAG GAG CGC ACC ATC TTC TTC AAG GAC GAC GGC AAC TAC AAG ACC   1536
Val Gln Glu Arg Thr Ile Phe Phe Lys Asp Asp Gly Asn Tyr Lys Thr
                500                 505                 510

CGC GCC GAG GTG AAG TTC GAG GGC GAC ACC CTG GTG AAC CGC ATC GAG   1584
Arg Ala Glu Val Lys Phe Glu Gly Asp Thr Leu Val Asn Arg Ile Glu
                515                 520                 525
```

21

```
CTG AAG GGC ATC GAC TTC AAG GAG GAC GGC AAC ATC CTG GGG CAC AAG   1632
Leu Lys Gly Ile Asp Phe Lys Glu Asp Gly Asn Ile Leu Gly His Lys
    530                 535                 540

CTG GAG TAC AAC TAC AAC AGC CAC AAC GTC TAT ATC ATG GCC GAC AAG   1680
Leu Glu Tyr Asn Tyr Asn Ser His Asn Val Tyr Ile Met Ala Asp Lys
545                 550                 555                 560

CAG AAG AAC GGC ATC AAG GTG AAC TTC AAG ATC CGC CAC AAC ATC GAG   1728
Gln Lys Asn Gly Ile Lys Val Asn Phe Lys Ile Arg His Asn Ile Glu
                565                 570                 575

GAC GGC AGC GTG CAG CTC GCC GAC CAC TAC CAG CAG AAC ACC CCC ATC   1776
Asp Gly Ser Val Gln Leu Ala Asp His Tyr Gln Gln Asn Thr Pro Ile
            580                 585                 590

GGC GAC GGC CCC GTG CTG CTG CCC GAC AAC CAC TAC CTG AGC ACC CAG   1824
Gly Asp Gly Pro Val Leu Leu Pro Asp Asn His Tyr Leu Ser Thr Gln
            595                 600                 605

TCC GCC CTG AGC AAA GAC CCC AAC GAG AAG CGC GAT CAC ATG GTC CTG   1872
Ser Ala Leu Ser Lys Asp Pro Asn Glu Lys Arg Asp His Met Val Leu
    610                 615                 620

CTG GAG TTC GTG ACC GCC GCC GGG ATC ACT CTC GGC ATG GAC GAG CTG   1920
Leu Glu Phe Val Thr Ala Ala Gly Ile Thr Leu Gly Met Asp Glu Leu
625                 630                 635                 640

TAC AAG TAA                                                       1929
Tyr Lys
```

(3) INFORMATION FOR SEQ ID NO:2:

   (i) SEQUENCE CHARACTERISTICS

      (A) LENGTH: 642 AMINO ACIDS
      (B) TYPE: AMINO ACID
      (C) STRANDEDNESS:
      (D) TOPOLOGY: LINEAR

   (ii) MOLECULE TYPE: PROTEIN
   (xi) SEQUENCE DESCRIPTION: SEQ ID NO:2:

```
Met Val Ser Lys Gly Glu Glu Leu Phe Thr Gly Val Val Pro Ile Leu
                5                  10                  15
Val Glu Leu Asp Gly Asp Val Asn Gly His Lys Phe Ser Val Ser Gly
            20                  25                  30
Glu Gly Glu Gly Asp Ala Thr Tyr Gly Lys Leu Thr Leu Lys Phe Ile
        35                  40                  45
Cys Thr Thr Gly Lys Leu Pro Val Pro Trp Pro Thr Leu Val Thr Thr
    50                  55                  60
Leu Thr His Gly Val Gln Cys Phe Ser Arg Tyr Pro Asp His Met Lys
65                  70                  75                  80

Gln His Asp Phe Phe Lys Ser Ala Met Pro Glu Gly Tyr Val Gln Glu
                85                  90                  95

Arg Thr Ile Phe Phe Lys Asp Asp Gly Asn Tyr Lys Thr Arg Ala Glu
            100                 105                 110

Val Lys Phe Glu Gly Asp Thr Leu Val Asn Arg Ile Glu Leu Lys Gly
        115                 120                 125
```

```
Ile Asp Phe Lys Glu Asp Gly Asn Ile Leu Gly His Lys Leu Glu Tyr
    130                 135                 140

Asn Phe Asn Ser His Asn Val Tyr Ile Met Ala Asp Lys Gln Lys Asn
145                 150                 155                 160

Gly Ile Lys Val Asn Phe Lys Ile Arg His Asn Ile Glu Asp Gly Ser
                165                 170                 175

Val Gln Leu Ala Asp His Tyr Gln Gln Asn Thr Pro Ile Gly Asp Gly
                180                 185                 190

Pro Val Leu Leu Pro Asp Asn His Tyr Leu Ser Thr Gln Ser Ala Leu
                195                 200                 205

Ser Lys Asp Pro Asn Glu Lys Arg Asp His Met Val Leu Leu Glu Phe
    210                 215                 220

Val Thr Ala Ala Arg Met His Asp Gln Leu Thr Glu Glu Gln Ile Ala
225                 230                 235                 240

Glu Phe Lys Glu Ala Phe Ser Leu Phe Asp Lys Asp Gly Asp Gly Thr
                245                 250                 255

Ile Thr Thr Lys Glu Leu Gly Thr Val Met Arg Ser Leu Gly Gln Asn
                260                 265                 270

Pro Thr Glu Ala Glu Leu Gln Asp Met Ile Asn Glu Val Asp Ala Asp
                275                 280                 285

Gly Asn Gly Thr Ile Tyr Phe Pro Glu Phe Leu Thr Met Met Ala Arg
    290                 295                 300

Lys Met Lys Asp Thr Asp Ser Glu Glu Glu Ile Arg Glu Ala Phe Arg
305                 310                 315                 320

Val Phe Asp Lys Asp Gly Asn Gly Tyr Ile Ser Ala Ala Glu Leu Arg
                325                 330                 335

His Val Met Thr Asn Leu Gly Glu Lys Leu Thr Asp Glu Glu Val Asp
                340                 345                 350

Glu Met Ile Arg Glu Ala Asp Ile Asp Gly Asp Gly Gln Val Asn Tyr
                355                 360                 365

Glu Glu Phe Val Gln Met Met Thr Ala Lys Gly Gly Lys Arg Arg Trp
    370                 375                 380

Lys Lys Asn Phe Ile Ala Val Ser Ala Ala Asn Arg Phe Lys Lys Ile
385                 390                 395                 400

Ser Glu Leu Met Val Ser Lys Gly Glu Glu Leu Phe Thr Gly Val Val
                405                 410                 415

Pro Ile Leu Val Glu Leu Asp Gly Asp Val Asn Gly His Lys Phe Ser
                420                 425                 430

Val Ser Gly Glu Gly Glu Gly Asp Ala Thr Tyr Gly Lys Leu Thr Leu
    435                 440                 445

Lys Phe Ile Cys Thr Thr Gly Lys Leu Pro Val Pro Trp Pro Thr Leu
    450                 455                 460

Val Thr Thr Leu Thr Tyr Gly Val Gln Cys Phe Ser Arg Tyr Pro Asp
465                 470                 475                 480
```

23

```
His Met Lys Gln His Asp Phe Phe Lys Ser Ala Met Pro Glu Gly Tyr
                485             490                 495

Val Gln Glu Arg Thr Ile Phe Phe Lys Asp Asp Gly Asn Tyr Lys Thr
            500             505                 510

Arg Ala Glu Val Lys Phe Glu Gly Asp Thr Leu Val Asn Arg Ile Glu
            515             520                 525

Leu Lys Gly Ile Asp Phe Lys Glu Asp Gly Asn Ile Leu Gly His Lys
        530             535                 540

Leu Glu Tyr Asn Tyr Asn Ser His Asn Val Tyr Ile Met Ala Asp Lys
545                 550                 555                 560

Gln Lys Asn Gly Ile Lys Val Asn Phe Lys Ile Arg His Asn Ile Glu
                565             570                 575

Asp Gly Ser Val Gln Leu Ala Asp His Tyr Gln Gln Asn Thr Pro Ile
            580             585                 590

Gly Asp Gly Pro Val Leu Leu Pro Asp Asn His Tyr Leu Ser Thr Gln
        595             600                 605

Ser Ala Leu Ser Lys Asp Pro Asn Glu Lys Arg Asp His Met Val Leu
    610             615                 620

Leu Glu Phe Val Thr Ala Ala Gly Ile Thr Leu Gly Met Asp Glu Leu
625             630                 635                 640

Tyr Lys
```

(4) INFORMATION FOR SEQ ID NO: 3:

(i) SEQUENCE CHARACTERISTICS

(A) LENGTH: 1959 BASE PAIRS
(B) TYPE: NUCLEIC ACID
(C) STRANDEDNESS: SINGLE
(D) TOPOLOGY: LINEAR

(ii) MOLECULE TYPE: cDNA
(xi) SEQUENCE DESCRIPTION: SEQ ID NO:3:

```
ATG GTG AGC AAG GGC GAG GAG CTG TTC ACC GGG GTG GTG CCC ATC CTG   48
Met Val Ser Lys Gly Glu Glu Leu Phe Thr Gly Val Val Pro Ile Leu
                5                   10                  15

GTC GAG CTG GAC GGC GAC GTA AAC GGC CAC AAG TTC AGC GTG TCC GGC   96
Val Glu Leu Asp Gly Asp Val Asn Gly His Lys Phe Ser Val Ser Gly
            20                  25                  30

GAG GGC GAG GGC GAT GCC ACC TAC GGC AAG CTG ACC CTG AAG TTC ATC
    144
Glu Gly Glu Gly Asp Ala Thr Tyr Gly Lys Leu Thr Leu Lys Phe Ile
        35                  40                  45

TGC ACC ACC GGC AAG CTG CCC GTG CCC TGG CCC ACC CTC GTG ACC ACC   192
Cys Thr Thr Gly Lys Leu Pro Val Pro Trp Pro Thr Leu Val Thr Thr
    50                  55                  60
```

```
CTG ACC CAT GGC GTG CAG TGC TTC AGC CGC TAC CCC GAC CAC ATG AAG   240
Leu Thr His Gly Val Gln Cys Phe Ser Arg Tyr Pro Asp His Met Lys
 65              70                  75                  80

CAG CAC GAC TTC TTC AAG TCC GCC ATG CCC GAA GGC TAC GTC CAG GAG   288
Gln His Asp Phe Phe Lys Ser Ala Met Pro Glu Gly Tyr Val Gln Glu
                85                  90                  95

CGC ACC ATC TTC TTC AAG GAC GAC GGC AAC TAC AAG ACC CGC GCC GAG   336
Arg Thr Ile Phe Phe Lys Asp Asp Gly Asn Tyr Lys Thr Arg Ala Glu
            100                 105                 110

GTG AAG TTC GAG GGC GAC ACC CTG GTG AAC CGC ATC GAG CTG AAG GGC   384
Val Lys Phe Glu Gly Asp Thr Leu Val Asn Arg Ile Glu Leu Lys Gly
        115                 120                 125

ATC GAC TTC AAG GAG GAC GGC AAC ATC CTG GGG CAC AAG CTG GAG TAC   432
Ile Asp Phe Lys Glu Asp Gly Asn Ile Leu Gly His Lys Leu Glu Tyr
        130                 135                 140

AAC TTC AAC AGC CAC AAC GTC TAT ATC ATG GCC GAC AAG CAG AAG AAC   480
Asn Phe Asn Ser His Asn Val Tyr Ile Met Ala Asp Lys Gln Lys Asn
145                 150                 155                 160

GGC ATC AAG GTG AAC TTC AAG ATC CGC CAC AAC ATC GAG GAC GGC AGC   528
Gly Ile Lys Val Asn Phe Lys Ile Arg His Asn Ile Glu Asp Gly Ser
                165                 170                 175

GTG CAG CTC GCC GAC CAC TAC CAG CAG AAC ACC CCC ATC GGC GAC GGC   576
Val Gln Leu Ala Asp His Tyr Gln Gln Asn Thr Pro Ile Gly Asp Gly
            180                 185                 190

CCC GTG CTG CTG CCC GAC AAC CAC TAC CTG AGC ACC CAG TCC GCC CTG   624
Pro Val Leu Leu Pro Asp Asn His Tyr Leu Ser Thr Gln Ser Ala Leu
        195                 200                 205

AGC AAA GAC CCC AAC GAG AAG CGC GAT CAC ATG GTC CTG CTG GAG TTC   672
Ser Lys Asp Pro Asn Glu Lys Arg Asp His Met Val Leu Leu Glu Phe
        210                 215                 220

GTG ACC GCC GCC CGC ATG CAT GAC CAA CTG ACA GAA GAG CAG ATT GCA   720
Val Thr Ala Ala Arg Met His Asp Gln Leu Thr Glu Glu Gln Ile Ala
225                 230                 235                 240

GAG TTC AAA GAA GCC TTC TCA TTA TTC GAC AAG GAT GGG GAC GGC ACC   768
Glu Phe Lys Glu Ala Phe Ser Leu Phe Asp Lys Asp Gly Asp Gly Thr
                245                 250                 255

ATC ACC ACA AAG GAA CTT GGC ACC GTT ATG AGG TCG CTT GGA CAA AAC   816
Ile Thr Thr Lys Glu Leu Gly Thr Val Met Arg Ser Leu Gly Gln Asn
            260                 265                 270

CCA ACG GAA GCA GAA TTG CAG GAT ATG ATC AAT GAA GTC GAT GCT GAT   864
Pro Thr Glu Ala Glu Leu Gln Asp Met Ile Asn Glu Val Asp Ala Asp
        275                 280                 285

GGC AAT GGA ACG ATT TAC TTT CCT GAA TTT CTT ACT ATG ATG GCT AGA   912
Gly Asn Gly Thr Ile Tyr Phe Pro Glu Phe Leu Thr Met Met Ala Arg
        290                 295                 300

AAA ATG AAG GAC ACA GAC AGC GAA GAG GAA ATC CGA GAA GCA TTC CGT   960
Lys Met Lys Asp Thr Asp Ser Glu Glu Glu Ile Arg Glu Ala Phe Arg
305                 310                 315                 320
```

```
GTT TTT GAC AAG GAT GGG AAC GGC TAC ATC AGC GCT GCT GAA TTA CGT   1008
Val Phe Asp Lys Asp Gly Asn Gly Tyr Ile Ser Ala Ala Glu Leu Arg
                325                 330                 335

CAC GTC ATG ACA AAC CTC GGG GAG AAG TTA ACA GAT GAA GAA GTT GAT   1056
His Val Met Thr Asn Leu Gly Glu Lys Leu Thr Asp Glu Glu Val Asp
                340                 345                 350

GAA ATG ATA AGG GAA GCA GAT ATC GAT GGT GAT GGC CAA GTA AAC TAT   1104
Glu Met Ile Arg Glu Ala Asp Ile Asp Gly Asp Gly Gln Val Asn Tyr
                355                 360                 365

GAA GAG TTT GTA CAA ATG ATG ACA GCA AAG GGG GGG AAG AGG CGC TGG   1152
Glu Glu Phe Val Gln Met Met Thr Ala Lys Gly Gly Lys Arg Arg Trp
                370                 375                 380

AAG AAA AAC TTC ATT GCC GTC AGC GCT GCC AAC CGG TTC AAG AAG ATC   1200
Lys Lys Asn Phe Ile Ala Val Ser Ala Ala Asn Arg Phe Lys Lys Ile
385                 390                 395                 400

TCC GAG CTC ATG GTG AGC AAG GGC GAG GAG CTG TTC ACC GGG GTG GTG   1248
Ser Glu Leu Met Val Ser Lys Gly Glu Glu Leu Phe Thr Gly Val Val
                405                 410                 415

CCC ATC CTG GTC GAG CTG GAC GGC GAC GTA AAC GGC CAC AAG TTC AGC   1296
Pro Ile Leu Val Glu Leu Asp Gly Asp Val Asn Gly His Lys Phe Ser
                420                 425                 430

GTG TCC GGC GAG GGC GAG GGC GAT GCC ACC TAC GGC AAG CTG ACC CTG   1344
Val Ser Gly Glu Gly Glu Gly Asp Ala Thr Tyr Gly Lys Leu Thr Leu
                435                 440                 445

AAG TTC ATC TGC ACC ACC GGC AAG CTG CCC GTG CCC TGG CCC ACC CTC   1392
Lys Phe Ile Cys Thr Thr Gly Lys Leu Pro Val Pro Trp Pro Thr Leu
                450                 455                 460

GTG ACC ACC CTG ACC TAC GGC GTG CAG TGC TTC AGC CGC TAC CCC GAC   1440
Val Thr Thr Leu Thr Tyr Gly Val Gln Cys Phe Ser Arg Tyr Pro Asp
465                 470                 475                 480

CAC ATG AAG CAG CAC GAC TTC TTC AAG TCC GCC ATG CCC GAA GGC TAC   1488
His Met Lys Gln His Asp Phe Phe Lys Ser Ala Met Pro Glu Gly Tyr
                485                 490                 495

GTC CAG GAG CGC ACC ATC TTC TTC AAG GAC GAC GGC AAC TAC AAG ACC   1536
Val Gln Glu Arg Thr Ile Phe Phe Lys Asp Asp Gly Asn Tyr Lys Thr
                500                 505                 510

CGC GCC GAG GTG AAG TTC GAG GGC GAC ACC CTG GTG AAC CGC ATC GAG   1584
Arg Ala Glu Val Lys Phe Glu Gly Asp Thr Leu Val Asn Arg Ile Glu
                515                 520                 525

CTG AAG GGC ATC GAC TTC AAG GAG GAC GGC AAC ATC CTG GGG CAC AAG   1632
Leu Lys Gly Ile Asp Phe Lys Glu Asp Gly Asn Ile Leu Gly His Lys
                530                 535                 540

CTG GAG TAC AAC TAC AAC AGC CAC AAC GTC TAT ATC ATG GCC GAC AAG   1680
Leu Glu Tyr Asn Tyr Asn Ser His Asn Val Tyr Ile Met Ala Asp Lys
545                 550                 555                 560

CAG AAG AAC GGC ATC AAG GTG AAC TTC AAG ATC CGC CAC AAC ATC GAG   1728
Gln Lys Asn Gly Ile Lys Val Asn Phe Lys Ile Arg His Asn Ile Glu
                565                 570                 575
```

```
GAC GGC AGC GTG CAG CTC GCC GAC CAC TAC CAG CAG AAC ACC CCC ATC   1776
Asp Gly Ser Val Gln Leu Ala Asp His Tyr Gln Gln Asn Thr Pro Ile
            580                 585                 590

GGC GAC GGC CCC GTG CTG CTG CCC GAC AAC CAC TAC CTG AGC ACC CAG   1824
Gly Asp Gly Pro Val Leu Leu Pro Asp Asn His Tyr Leu Ser Thr Gln
            595                 600                 605

TCC GCC CTG AGC AAA GAC CCC AAC GAG AAG CGC GAT CAC ATG GTC CTG   1872
Ser Ala Leu Ser Lys Asp Pro Asn Glu Lys Arg Asp His Met Val Leu
    610                 615                 620

CTG GAG TTC GTG ACC GCC GCC GGG ATC ACT CTC GGC ATG GAC GAG CTG   1920
Leu Glu Phe Val Thr Ala Ala Gly Ile Thr Leu Gly Met Asp Glu Leu
625                 630                 635                 640

TAC AAG CCA AAA AAG AAG AGA AAG GTG GAA GAC GCT TAA                1959
Tyr Lys Pro Lys Lys Lys Arg Lys Val Glu Asp Ala
    645                 650
```

(5) INFORMATION FOR SEQ ID NO:4:

    (i) SEQUENCE CHARACTERISTICS

        (A) LENGTH: 652 AMINO ACIDS
        (B) TYPE: AMINO ACID
        (C) STRANDEDNESS:
        (D) TOPOLOGY: LINEAR

    (ii) MOLECULE TYPE: PROTEIN
    (xi) SEQUENCE DESCRIPTION: SEQ ID NO:4:

```
Met Val Ser Lys Gly Glu Glu Leu Phe Thr Gly Val Val Pro Ile Leu
              5                  10              15

Val Glu Leu Asp Gly Asp Val Asn Gly His Lys Phe Ser Val Ser Gly
              20              25              30

Glu Gly Glu Gly Asp Ala Thr Tyr Gly Lys Leu Thr Leu Lys Phe Ile
          35                  40                  45

Cys Thr Thr Gly Lys Leu Pro Val Pro Trp Pro Thr Leu Val Thr Thr
        50                  55              60

Leu Thr His Gly Val Gln Cys Phe Ser Arg Tyr Pro Asp His Met Lys
65                  70              75                  80

Gln His Asp Phe Phe Lys Ser Ala Met Pro Glu Gly Tyr Val Gln Glu
                  85                  90              95

Arg Thr Ile Phe Phe Lys Asp Asp Gly Asn Tyr Lys Thr Arg Ala Glu
              100                 105                 110

Val Lys Phe Glu Gly Asp Thr Leu Val Asn Arg Ile Glu Leu Lys Gly
          115                 120                 125

Ile Asp Phe Lys Glu Asp Gly Asn Ile Leu Gly His Lys Leu Glu Tyr
    130                 135                 140

Asn Phe Asn Ser His Asn Val Tyr Ile Met Ala Asp Lys Gln Lys Asn
145                 150                 155                 160
```

```
Gly Ile Lys Val Asn Phe Lys Ile Arg His Asn Ile Glu Asp Gly Ser
            165                 170                 175

Val Gln Leu Ala Asp His Tyr Gln Gln Asn Thr Pro Ile Gly Asp Gly
                180             185                 190

Pro Val Leu Leu Pro Asp Asn His Tyr Leu Ser Thr Gln Ser Ala Leu
            195                 200                 205

Ser Lys Asp Pro Asn Glu Lys Arg Asp His Met Val Leu Leu Glu Phe
    210                 215                 220

Val Thr Ala Ala Arg Met His Asp Gln Leu Thr Glu Glu Gln Ile Ala
225                 230                 235                 240

Glu Phe Lys Glu Ala Phe Ser Leu Phe Asp Lys Asp Gly Asp Gly Thr
                245                 250                 255

Ile Thr Thr Lys Glu Leu Gly Thr Val Met Arg Ser Leu Gly Gln Asn
            260                 265                 270

Pro Thr Glu Ala Glu Leu Gln Asp Met Ile Asn Glu Val Asp Ala Asp
        275                 280                 285

Gly Asn Gly Thr Ile Tyr Phe Pro Glu Phe Leu Thr Met Met Ala Arg
    290                 295                 300

Lys Met Lys Asp Thr Asp Ser Glu Glu Glu Ile Arg Glu Ala Phe Arg
305                 310                 315                 320

Val Phe Asp Lys Asp Gly Asn Gly Tyr Ile Ser Ala Ala Glu Leu Arg
                325                 330                 335

His Val Met Thr Asn Leu Gly Glu Lys Leu Thr Asp Glu Glu Val Asp
            340                 345                 350

Glu Met Ile Arg Glu Ala Asp Ile Asp Gly Asp Gly Gln Val Asn Tyr
            355                 360                 365

Glu Glu Phe Val Gln Met Met Thr Ala Lys Gly Gly Lys Arg Arg Trp
    370                 375                 380

Lys Lys Asn Phe Ile Ala Val Ser Ala Ala Asn Arg Phe Lys Lys Ile
385                 390                 395                 400

Ser Glu Leu Met Val Ser Lys Gly Glu Glu Leu Phe Thr Gly Val Val
            405                 410                 415

Pro Ile Leu Val Glu Leu Asp Gly Asp Val Asn Gly His Lys Phe Ser
            420                 425                 430

Val Ser Gly Glu Gly Glu Gly Asp Ala Thr Tyr Gly Lys Leu Thr Leu
            435                 440                 445

Lys Phe Ile Cys Thr Thr Gly Lys Leu Pro Val Pro Trp Pro Thr Leu
            450                 455                 460

Val Thr Thr Leu Thr Tyr Gly Val Gln Cys Phe Ser Arg Tyr Pro Asp
465                 470                 475                 480

His Met Lys Gln His Asp Phe Phe Lys Ser Ala Met Pro Glu Gly Tyr
                485                 490                 495
```

```
Val Gln Glu Arg Thr Ile Phe Phe Lys Asp Asp Gly Asn Tyr Lys Thr
        500             505             510

Arg Ala Glu Val Lys Phe Glu Gly Asp Thr Leu Val Asn Arg Ile Glu
        515             520             525

Leu Lys Gly Ile Asp Phe Lys Glu Asp Gly Asn Ile Leu Gly His Lys
        530             535             540

Leu Glu Tyr Asn Tyr Asn Ser His Asn Val Tyr Ile Met Ala Asp Lys
545             550             555             560

Gln Lys Asn Gly Ile Lys Val Asn Phe Lys Ile Arg His Asn Ile Glu
        565             570             575

Asp Gly Ser Val Gln Leu Ala Asp His Tyr Gln Gln Asn Thr Pro Ile
        580             585             590

Gly Asp Gly Pro Val Leu Leu Pro Asp Asn His Tyr Leu Ser Thr Gln
        595             600             605

Ser Ala Leu Ser Lys Asp Pro Asn Glu Lys Arg Asp His Met Val Leu
        610             615             620

Leu Glu Phe Val Thr Ala Ala Gly Ile Thr Leu Gly Met Asp Glu Leu
625             630             635             640

Tyr Lys Pro Lys Lys Lys Arg Lys Val Glu Asp Ala
        645             650
```

(6) INFORMATION FOR SEQ ID NO:5:

    (i) SEQUENCE CHARACTERISTICS

        (A) LENGTH: 1929 BASE PAIRS
        (B) TYPE: NUCLEIC ACID
        (C) STRANDEDNESS: SINGLE
        (D) TOPOLOGY: LINEAR

    (ii) MOLECULE TYPE: cDNA
    (xi) SEQUENCE DESCRIPTION: SEQ ID NO:5:

```
ATG GTG AGC AAG GGC GAG GAG CTG TTC ACC GGG GTG GTG CCC ATC CTG   48
Met Val Ser Lys Gly Glu Glu Leu Phe Thr Gly Val Val Pro Ile Leu
                5               10              15

GTC GAG CTG GAC GGC GAC GTA AAC GGC CAC AAG TTC AGC GTG TCC GGC   96
Val Glu Leu Asp Gly Asp Val Asn Gly His Lys Phe Ser Val Ser Gly
                20              25              30

GAG GGC GAG GGC GAT GCC ACC TAC GGC AAG CTG ACC CTG AAG TTC ATC  144
Glu Gly Glu Gly Asp Ala Thr Tyr Gly Lys Leu Thr Leu Lys Phe Ile
                35              40              45

TGC ACC ACC GGC AAG CTG CCC GTG CCC TGG CCC ACC CTC GTG ACC ACC  192
Cys Thr Thr Gly Lys Leu Pro Val Pro Trp Pro Thr Leu Val Thr Thr
        50              55              60
```

```
CTG ACC CAT GGC GTG CAG TGC TTC AGC CGC TAC CCC GAC CAC ATG AAG   240
Leu Thr His Gly Val Gln Cys Phe Ser Arg Tyr Pro Asp His Met Lys
65              70              75              80

CAG CAC GAC TTC TTC AAG TCC GCC ATG CCC GAA GGC TAC GTC CAG GAG   288
Gln His Asp Phe Phe Lys Ser Ala Met Pro Glu Gly Tyr Val Gln Glu
                85              90              95

CGC ACC ATC TTC TTC AAG GAC GAC GGC AAC TAC AAG ACC CGC GCC GAG   336
Arg Thr Ile Phe Phe Lys Asp Asp Gly Asn Tyr Lys Thr Arg Ala Glu
            100             105             110

GTG AAG TTC GAG GGC GAC ACC CTG GTG AAC CGC ATC GAG CTG AAG GGC   384
Val Lys Phe Glu Gly Asp Thr Leu Val Asn Arg Ile Glu Leu Lys Gly
        115             120             125

ATC GAC TTC AAG GAG GAC GGC AAC ATC CTG GGG CAC AAG CTG GAG TAC   432
Ile Asp Phe Lys Glu Asp Gly Asn Ile Leu Gly His Lys Leu Glu Tyr
        130             135             140

AAC TTC AAC AGC CAC AAC GTC TAT ATC ATG GCC GAC AAG CAG AAG AAC   480
Asn Phe Asn Ser His Asn Val Tyr Ile Met Ala Asp Lys Gln Lys Asn
145             150             155             160

GGC ATC AAG GTG AAC TTC AAG ATC CGC CAC AAC ATC GAG GAC GGC AGC   528
Gly Ile Lys Val Asn Phe Lys Ile Arg His Asn Ile Glu Asp Gly Ser
            165             170             175

GTG CAG CTC GCC GAC CAC TAC CAG CAG AAC ACC CCC ATC GGC GAC GGC   576
Val Gln Leu Ala Asp His Tyr Gln Gln Asn Thr Pro Ile Gly Asp Gly
            180             185             190

CCC GTG CTG CTG CCC GAC AAC CAC TAC CTG AGC ACC CAG TCC GCC CTG   624
Pro Val Leu Leu Pro Asp Asn His Tyr Leu Ser Thr Gln Ser Ala Leu
            195             200             205

AGC AAA GAC CCC AAC GAG AAG CGC GAT CAC ATG GTC CTG CTG GAG TTC   672
Ser Lys Asp Pro Asn Glu Lys Arg Asp His Met Val Leu Leu Glu Phe
    210             215             220

GTG ACC GCC GCC CGC ATG CAT GAC CAA CTG ACA GAA GAG CAG ATT GCA   720
Val Thr Ala Ala Arg Met His Asp Gln Leu Thr Glu Glu Gln Ile Ala
225             230             235             240

GAG TTC AAA GAA GCC TTC TCA TTA TTC GAC AAG GAT GGG GAC GGC ACC   768
Glu Phe Lys Glu Ala Phe Ser Leu Phe Asp Lys Asp Gly Asp Gly Thr
            245             250             255

ATC ACC ACA AAG GAA CTT GGC ACC GTT ATG AGG TCG CTT GGA CAA AAC   816
Ile Thr Thr Lys Glu Leu Gly Thr Val Met Arg Ser Leu Gly Gln Asn
            260             265             270

CCA ACG GAA GCA GAA TTG CAG GAT ATG ATC AAT GAA GTC GAT GCT GAT   864
Pro Thr Glu Ala Glu Leu Gln Asp Met Ile Asn Glu Val Asp Ala Asp
        275             280             285

GGC AAT GGA ACG ATT TAC TTT CCT GAA TTT CTT ACT ATG ATG GCT AGA   912
Gly Asn Gly Thr Ile Tyr Phe Pro Glu Phe Leu Thr Met Met Ala Arg
    290             295             300

AAA ATG AAG GAC ACA GAC AGC GAA GAG GAA ATC CGA GAA GCA TTC CGT   960
Lys Met Lys Asp Thr Asp Ser Glu Glu Glu Ile Arg Glu Ala Phe Arg
305             310             315             320
```

```
GTT TTT GAC AAG GAT GGG AAC GGC TAC ATC AGC GCT GCT CAG TTA CGT    1008
Val Phe Asp Lys Asp Gly Asn Gly Tyr Ile Ser Ala Ala Gln Leu Arg
            325                 330                 335

CAC GTC ATG ACA AAC CTC GGG GAG AAG TTA ACA GAT GAA GAA GTT GAT    1056
His Val Met Thr Asn Leu Gly Glu Lys Leu Thr Asp Glu Glu Val Asp
            340                 345                 350

GAA ATG ATA AGG GAA GCA GAT ATC GAT GGT GAT GGC CAA GTA AAC TAT    1104
Glu Met Ile Arg Glu Ala Asp Ile Asp Gly Asp Gly Gln Val Asn Tyr
            355                 360                 365

GAA GAG TTT GTA CAA ATG ATG ACA GCA AAG GGG GGG AAG AGG CGC TGG    1152
Glu Glu Phe Val Gln Met Met Thr Ala Lys Gly Gly Lys Arg Arg Trp
            370                 375                 380

AAG AAA AAC TTC ATT GCC GTC AGC GCT GCC AAC CGG TTC AAG AAG ATC    1200
Lys Lys Asn Phe Ile Ala Val Ser Ala Ala Asn Arg Phe Lys Lys Ile
385                 390                 395                 400

TCC GAG CTC ATG GTG AGC AAG GGC GAG GAG CTG TTC ACC GGG GTG GTG    1248
Ser Glu Leu Met Val Ser Lys Gly Glu Glu Leu Phe Thr Gly Val Val
            405                 410                 415

CCC ATC CTG GTC GAG CTG GAC GGC GAC GTA AAC GGC CAC AAG TTC AGC    1296
Pro Ile Leu Val Glu Leu Asp Gly Asp Val Asn Gly His Lys Phe Ser
            420                 425                 430

GTG TCC GGC GAG GGC GAG GGC GAT GCC ACC TAC GGC AAG CTG ACC CTG    1344
Val Ser Gly Glu Gly Glu Gly Asp Ala Thr Tyr Gly Lys Leu Thr Leu
            435                 440                 445

AAG TTC ATC TGC ACC ACC GGC AAG CTG CCC GTG CCC TGG CCC ACC CTC    1392
Lys Phe Ile Cys Thr Thr Gly Lys Leu Pro Val Pro Trp Pro Thr Leu
            450                 455                 460

GTG ACC ACC CTG ACC TAC GGC GTG CAG TGC TTC AGC CGC TAC CCC GAC    1440
Val Thr Thr Leu Thr Tyr Gly Val Gln Cys Phe Ser Arg Tyr Pro Asp
465                 470                 475                 480

CAC ATG AAG CAG CAC GAC TTC TTC AAG TCC GCC ATG CCC GAA GGC TAC    1488
His Met Lys Gln His Asp Phe Phe Lys Ser Ala Met Pro Glu Gly Tyr
            485                 490                 495

GTC CAG GAG CGC ACC ATC TTC TTC AAG GAC GAC GGC AAC TAC AAG ACC    1536
Val Gln Glu Arg Thr Ile Phe Phe Lys Asp Asp Gly Asn Tyr Lys Thr
            500                 505                 510

CGC GCC GAG GTG AAG TTC GAG GGC GAC ACC CTG GTG AAC CGC ATC GAG    1584
Arg Ala Glu Val Lys Phe Glu Gly Asp Thr Leu Val Asn Arg Ile Glu
            515                 520                 525

CTG AAG GGC ATC GAC TTC AAG GAG GAC GGC AAC ATC CTG GGG CAC AAG    1632
Leu Lys Gly Ile Asp Phe Lys Glu Asp Gly Asn Ile Leu Gly His Lys
            530                 535                 540

CTG GAG TAC AAC TAC AAC AGC CAC AAC GTC TAT ATC ATG GCC GAC AAG    1680
Leu Glu-Tyr Asn Tyr Asn Ser His Asn Val Tyr Ile Met Ala Asp Lys
545                 550                 555                 560

CAG AAG AAC GGC ATC AAG GTG AAC TTC AAG ATC CGC CAC AAC ATC GAG    1728
Gln Lys Asn Gly Ile Lys Val Asn Phe Lys Ile Arg His Asn Ile Glu
            565                 570                 575
```

32

```
GAC GGC AGC GTG CAG CTC GCC GAC CAC TAC CAG CAG AAC ACC CCC ATC   1776
Asp Gly Ser Val Gln Leu Ala Asp His Tyr Gln Gln Asn Thr Pro Ile
            580                 585                 590

GGC GAC GGC CCC GTG CTG CTG CCC GAC AAC CAC TAC CTG AGC ACC CAG   1824
Gly Asp Gly Pro Val Leu Leu Pro Asp Asn His Tyr Leu Ser Thr Gln
            595                 600                 605

TCC GCC CTG AGC AAA GAC CCC AAC GAG AAG CGC GAT CAC ATG GTC CTG   1872
Ser Ala Leu Ser Lys Asp Pro Asn Glu Lys Arg Asp His Met Val Leu
    610                 615                 620

CTG GAG TTC GTG ACC GCC GCC GGG ATC ACT CTC GGC ATG GAC GAG CTG   1920
Leu Glu Phe Val Thr Ala Ala Gly Ile Thr Leu Gly Met Asp Glu Leu
625                 630                 635                 640

TAC AAG TAA
        1929
Tyr Lys
```

(7) INFORMATION FOR SEQ ID NO:6:

    (i) SEQUENCE CHARACTERISTICS

        (A) LENGTH: 642 AMINO ACIDS
        (B) TYPE: AMINO ACID
        (C) STRANDEDNESS:
        (D) TOPOLOGY: LINEAR

    (ii) MOLECULE TYPE: PROTEIN
    (xi) SEQUENCE DESCRIPTION: SEQ ID NO:6:

```
Met Val Ser Lys Gly Glu Glu Leu Phe Thr Gly Val Val Pro Ile Leu
                  5                  10                  15

Val Glu Leu Asp Gly Asp Val Asn Gly His Lys Phe Ser Val Ser Gly
                 20                  25                  30

Glu Gly Glu Gly Asp Ala Thr Tyr Gly Lys Leu Thr Leu Lys Phe Ile
             35                  40                  45

Cys Thr Thr Gly Lys Leu Pro Val Pro Trp Pro Thr Leu Val Thr Thr
         50                  55                  60

Leu Thr His Gly Val Gln Cys Phe Ser Arg Tyr Pro Asp His Met Lys
 65                  70                  75                  80

Gln His Asp Phe Phe Lys Ser Ala Met Pro Glu Gly Tyr Val Gln Glu
                 85                  90                  95

Arg Thr Ile Phe Phe Lys Asp Asp Gly Asn Tyr Lys Thr Arg Ala Glu
                100                 105                 110

Val Lys Phe Glu Gly Asp Thr Leu Val Asn Arg Ile Glu Leu Lys Gly
         115                 120                 125

Ile Asp Phe Lys Glu Asp Gly Asn Ile Leu Gly His Lys Leu Glu Tyr
     130                 135                 140

Asn Phe Asn Ser His Asn Val Tyr Ile Met Ala Asp Lys Gln Lys Asn
145                 150                 155                 160

Gly Ile Lys Val Asn Phe Lys Ile Arg His Asn Ile Glu Asp Gly Ser
                 165                 170                 175
```

34

Val Gln Leu Ala Asp His Tyr Gln Gln Asn Thr Pro Ile Gly Asp Gly
            180                 185                 190

Pro Val Leu Leu Pro Asp Asn His Tyr Leu Ser Thr Gln Ser Ala Leu
            195                 200                 205

Ser Lys Asp Pro Asn Glu Lys Arg Asp His Met Val Leu Leu Glu Phe
    210                 215                 220

Val Thr Ala Ala Arg Met His Asp Gln Leu Thr Glu Glu Gln Ile Ala
225                 230                 235                 240

Glu Phe Lys Glu Ala Phe Ser Leu Phe Asp Lys Asp Gly Asp Gly Thr
                245                 250                 255

Ile Thr Thr Lys Glu Leu Gly Thr Val Met Arg Ser Leu Gly Gln Asn
            260                 265                 270

Pro Thr Glu Ala Glu Leu Gln Asp Met Ile Asn Glu Val Asp Ala Asp
        275                 280                 285

Gly Asn Gly Thr Ile Tyr Phe Pro Glu Phe Leu Thr Met Met Ala Arg
    290                 295                 300

Lys Met Lys Asp Thr Asp Ser Glu Glu Glu Ile Arg Glu Ala Phe Arg
305                 310                 315                 320

Val Phe Asp Lys Asp Gly Asn Gly Tyr Ile Ser Ala Ala Gln Leu Arg
                325                 330                 335

His Val Met Thr Asn Leu Gly Glu Lys Leu Thr Asp Glu Glu Val Asp
            340                 345                 350

Glu Met Ile Arg Glu Ala Asp Ile Asp Gly Asp Gly Gln Val Asn Tyr
        355                 360                 365

Glu Glu Phe Val Gln Met Met Thr Ala Lys Gly Gly Lys Arg Arg Trp
    370                 375                 380

Lys Lys Asn Phe Ile Ala Val Ser Ala Ala Asn Arg Phe Lys Lys Ile
385                 390                 395                 400

Ser Glu Leu Met Val Ser Lys Gly Glu Glu Leu Phe Thr Gly Val Val
                405                 410                 415

Pro Ile Leu Val Glu Leu Asp Gly Asp Val Asn Gly His Lys Phe Ser
            420                 425                 430

Val Ser Gly Glu Gly Glu Gly Asp Ala Thr Tyr Gly Lys Leu Thr Leu
    435                 440                 445

Lys Phe Ile Cys Thr Thr Gly Lys Leu Pro Val Pro Trp Pro Thr Leu
    450                 455                 460

Val Thr Thr Leu Thr Tyr Gly Val Gln Cys Phe Ser Arg Tyr Pro Asp
465                 470                 475                 480

His Met Lys Gln His Asp Phe Phe Lys Ser Ala Met Pro Glu Gly Tyr
            485                 490                 495

Val Gln Glu Arg Thr Ile Phe Phe Lys Asp Asp Gly Asn Tyr Lys Thr
            500                 505                 510

Arg Ala Glu Val Lys Phe Glu Gly Asp Thr Leu Val Asn Arg Ile Glu
        515                 520                 525

```
Leu Lys Gly Ile Asp Phe Lys Glu Asp Gly Asn Ile Leu Gly His Lys
    530                 535             540
Leu Glu Tyr Asn Tyr Asn Ser His Asn Val Tyr Ile Met Ala Asp Lys
545             550                 555                 560
Gln Lys Asn Gly Ile Lys Val Asn Phe Lys Ile Arg His Asn Ile Glu
            565                 570                 575
Asp Gly Ser Val Gln Leu Ala Asp His Tyr Gln Gln Asn Thr Pro Ile
            580                 585                 590
Gly Asp Gly Pro Val Leu Leu Pro Asp Asn His Tyr Leu Ser Thr Gln
        595                 600                 605
Ser Ala Leu Ser Lys Asp Pro Asn Glu Lys Arg Asp His Met Val Leu
    610                 615                 620
Leu Glu Phe Val Thr Ala Ala Gly Ile Thr Leu Gly Met Asp Glu Leu
625                 630                 635                 640
Tyr Lys
```

(8) INFORMATION FOR SEQ ID NO: 7:

(i) SEQUENCE CHARACTERISTICS

(A) LENGTH: 1971 BASE PAIRS
(B) TYPE: NUCLEIC ACID
(C) STRANDEDNESS: SINGLE
(D) TOPOLOGY: LINEAR

(ii) MOLECULE TYPE: cDNA
(xi) SEQUENCE DESCRIPTION: SEQ ID NO: 7:

```
ATG CTG CTG CCC GTC CCC CTG CTG CTG GGC CTG CTG GGC GCC GCC GCC   48
Met Leu Leu Pro Val Pro Leu Leu Leu Gly Leu Leu Gly Ala Ala Ala
              5                 10                  15
GAC GTG AGC AAG GGC GAG GAG CTG TTC ACC GGG GTG GTG CCC ATC CTG   96
Asp Val Ser Lys Gly Glu Glu Leu Phe Thr Gly Val Val Pro Ile Leu
              20                25                  30
GTC GAG CTG GAC GGC GAC GTA AAC GGC CAC AAG TTC AGC GTG TCC GGC  144
Val Glu Leu Asp Gly Asp Val Asn Gly His Lys Phe Ser Val Ser Gly
          35                  40                  45
GAG GGC GAG GGC GAT GCC ACC TAC GGC AAG CTG ACC CTG AAG TTC ATC  192
Glu Gly Glu Gly Asp Ala Thr Tyr Gly Lys Leu Thr Leu Lys Phe Ile
      50                  55                  60
TGC ACC ACC GGC AAG CTG CCC GTG CCC TGG CCC ACC CTC GTG ACC ACC  240
Cys Thr Thr Gly Lys Leu Pro Val Pro Trp Pro Thr Leu Val Thr Thr
65                  70                  75                  80
CTG ACC CAT GGC GTG CAG TGC TTC AGC CGC TAC CCC GAC CAC ATG AAG  288
Leu Thr His Gly Val Gln Cys Phe Ser Arg Tyr Pro Asp His Met Lys
              85                  90                  95
CAG CAC GAC TTC TTC AAG TCC GCC ATG CCC GAA GGC TAC GTC CAG GAG  336
Gln His Asp Phe Phe Lys Ser Ala Met Pro Glu Gly Tyr Val Gln Glu
              100                 105                 110
```

```
CGC ACC ATC TTC TTC AAG GAC GAC GGC AAC TAC AAG ACC CGC GCC GAG   384
Arg Thr Ile Phe Phe Lys Asp Asp Gly Asn Tyr Lys Thr Arg Ala Glu
        115                 120                 125

GTG AAG TTC GAG GGC GAC ACC CTG GTG AAC CGC ATC GAG CTG AAG GGC   432
Val Lys Phe Glu Gly Asp Thr Leu Val Asn Arg Ile Glu Leu Lys Gly
        130                 135                 140

ATC GAC TTC AAG GAG GAC GGC AAC ATC CTG GGG CAC AAG CTG GAG TAC   480
Ile Asp Phe Lys Glu Asp Gly Asn Ile Leu Gly His Lys Leu Glu Tyr
145                 150                 155                 160

AAC TTC AAC AGC CAC AAC GTC TAT ATC ATG GCC GAC AAG CAG AAG AAC   528
Asn Phe Asn Ser His Asn Val Tyr Ile Met Ala Asp Lys Gln Lys Asn
                165                 170                 175

GGC ATC AAG GTG AAC TTC AAG ATC CGC CAC AAC ATC GAG GAC GGC AGC   576
Gly Ile Lys Val Asn Phe Lys Ile Arg His Asn Ile Glu Asp Gly Ser
                180                 185                 190

GTG CAG CTC GCC GAC CAC TAC CAG CAG AAC ACC CCC ATC GGC GAC GGC   624
Val Gln Leu Ala Asp His Tyr Gln Gln Asn Thr Pro Ile Gly Asp Gly
        195                 200                 205

CCC GTG CTG CTG CCC GAC AAC CAC TAC CTG AGC ACC CAG TCC GCC CTG   672
Pro Val Leu Leu Pro Asp Asn His Tyr Leu Ser Thr Gln Ser Ala Leu
        210                 215                 220

AGC AAA GAC CCC AAC GAG AAG CGC GAT CAC ATG GTC CTG CTG GAG TTC   720
Ser Lys Asp Pro Asn Glu Lys Arg Asp His Met Val Leu Leu Glu Phe
225                 230                 235                 240

GTG ACC GCC GCC CGC ATG CAT GAC CAA CTG ACA GAA GAG CAG ATT GCA   768
Val Thr Ala Ala Arg Met His Asp Gln Leu Thr Glu Glu Gln Ile Ala
                245                 250                 255

GAG TTC AAA GAA GCC TTC TCA TTA TTC GAC AAG GAT GGG GAC GGC ACC   816
Glu Phe Lys Glu Ala Phe Ser Leu Phe Asp Lys Asp Gly Asp Gly Thr
                260                 265                 270

ATC ACC ACA AAG GAA CTT GGC ACC GTT ATG AGG TCG CTT GGA CAA AAC   864
Ile Thr Thr Lys Glu Leu Gly Thr Val Met Arg Ser Leu Gly Gln Asn
        275                 280                 285

CCA ACG GAA GCA GAA TTG CAG GAT ATG ATC AAT GAA GTC GAT GCT GAT   912
Pro Thr Glu Ala Glu Leu Gln Asp Met Ile Asn Glu Val Asp Ala Asp
        290                 295                 300

GGC AAT GGA ACG ATT TAC TTT CCT GAA TTT CTT ACT ATG ATG GCT AGA   960
Gly Asn Gly Thr Ile Tyr Phe Pro Glu Phe Leu Thr Met Met Ala Arg
305                 310                 315                 320

AAA ATG AAG GAC ACA GAC AGC GAA GAG GAA ATC CGA GAA GCA TTC CGT   1008
Lys Met Lys Asp Thr Asp Ser Glu Glu Glu Ile Arg Glu Ala Phe Arg
                325                 330                 335

GTT TTT GAC AAG GAT GGG AAC GGC TAC ATC AGC GCT GCT CAG TTA CGT   1056
Val Phe Asp Lys Asp Gly Asn Gly Tyr Ile Ser Ala Ala Gln Leu Arg
                340                 345                 350

CAC GTC ATG ACA AAC CTC GGG GAG AAG TTA ACA GAT GAA GAA GTT GAT   1104
His Val Met Thr Asn Leu Gly Glu Lys Leu Thr Asp Glu Glu Val Asp
        355                 360                 365
```

EP 0 970 199 B1

```
GAA ATG ATA AGG GAA GCA GAT ATC GAT GGT GAT GGC CAA GTA AAC TAT   1152
Glu Met Ile Arg Glu Ala Asp Ile Asp Gly Asp Gly Gln Val Asn Tyr
    370                 375                 380

GAA GAG TTT GTA CAA ATG ATG ACA GCA AAG GGG GGG AAG AGG CGC TGG   1200
Glu Glu Phe Val Gln Met Met Thr Ala Lys Gly Gly Lys Arg Arg Trp
385                 390                 395                 400

AAG AAA AAC TTC ATT GCC GTC AGC GCT GCC AAC CGG TTC AAG AAG ATC   1248
Lys Lys Asn Phe Ile Ala Val Ser Ala Ala Asn Arg Phe Lys Lys Ile
                405                 410                 415

TCC GAG CTC ATG GTG AGC AAG GGC GAG GAG CTG TTC ACC GGG GTG GTG   1296
Ser Glu Leu Met Val Ser Lys Gly Glu Glu Leu Phe Thr Gly Val Val
            420                 425                 430

CCC ATC CTG GTC GAG CTG GAC GGC GAC GTA AAC GGC CAC AAG TTC AGC   1344
Pro Ile Leu Val Glu Leu Asp Gly Asp Val Asn Gly His Lys Phe Ser
            435                 440                 445

GTG TCC GGC GAG GGC GAG GGC GAT GCC ACC TAC GGC AAG CTG ACC CTG   1392
Val Ser Gly Glu Gly Glu Gly Asp Ala Thr Tyr Gly Lys Leu Thr Leu
    450                 455                 460

AAG TTC ATC TGC ACC ACC GGC AAG CTG CCC GTG CCC TGG CCC ACC CTC   1440
Lys Phe Ile Cys Thr Thr Gly Lys Leu Pro Val Pro Trp Pro Thr Leu
465                 470                 475                 480

GTG ACC ACC CTG ACC TAC GGC GTG CAG TGC TTC AGC CGC TAC CCC GAC   1488
Val Thr Thr Leu Thr Tyr Gly Val Gln Cys Phe Ser Arg Tyr Pro Asp
                485                 490                 495

CAC ATG AAG CAG CAC GAC TTC TTC AAG TCC GCC ATG CCC GAA GGC TAC   1536
His Met Lys Gln His Asp Phe Phe Lys Ser Ala Met Pro Glu Gly Tyr
            500                 505                 510

GTC CAG GAG CGC ACC ATC TTC TTC AAG GAC GAC GGC AAC TAC AAG ACC   1584
Val Gln Glu Arg Thr Ile Phe Phe Lys Asp Asp Gly Asn Tyr Lys Thr
    515                 520                 525

CGC GCC GAG GTG AAG TTC GAG GGC GAC ACC CTG GTG AAC CGC ATC GAG   1632
Arg Ala Glu Val Lys Phe Glu Gly Asp Thr Leu Val Asn Arg Ile Glu
    530                 535                 540

CTG AAG GGC ATC GAC TTC AAG GAG GAC GGC AAC ATC CTG GGG CAC AAG   1680
Leu Lys Gly Ile Asp Phe Lys Glu Asp Gly Asn Ile Leu Gly His Lys
545                 550                 555                 560

CTG GAG TAC AAC TAC AAC AGC CAC AAC GTC TAT ATC ATG GCC GAC AAG   1728
Leu Glu Tyr Asn Tyr Asn Ser His Asn Val Tyr Ile Met Ala Asp Lys
                565                 570                 575

CAG AAG AAC GGC ATC AAG GTG AAC TTC AAG ATC CGC CAC AAC ATC GAG   1776
Gln Lys Asn Gly Ile Lys Val Asn Phe Lys Ile Arg His Asn Ile Glu
            580                 585                 590

GAC GGC AGC GTG CAG CTC GCC GAC CAC TAC CAG CAG AAC ACC CCC ATC   1824
Asp Gly Ser Val Gln Leu Ala Asp His Tyr Gln Gln Asn Thr Pro Ile
            595                 600                 605

GGC GAC GGC CCC GTG CTG CTG CCC GAC AAC CAC TAC CTG AGC ACC CAG   1872
Gly Asp Gly Pro Val Leu Leu Pro Asp Asn His Tyr Leu Ser Thr Gln
            610                 615                 620
```

38

```
TCC GCC CTG AGC AAA GAC CCC AAC GAG AAG CGC GAT CAC ATG GTC CTG   1920
Ser Ala Leu Ser Lys Asp Pro Asn Glu Lys Arg Asp His Met Val Leu
625             630             635             640

CTG GAG TTC GTG ACC GCC GCC GGG ATC ACT CTC GGC AAG GAC GAG CTG   1968
Leu Glu Phe Val Thr Ala Ala Gly Ile Thr Leu Gly Lys Asp Glu Leu
            645             650             655

TAA                                                               1971
```

(9) INFORMATION FOR SEQ ID NO:8:

    (i) SEQUENCE CHARACTERISTICS

        (A) LENGTH: 656 AMINO ACIDS
        (B) TYPE: AMINO ACID
        (C) STRANDEDNESS:
        (D) TOPOLOGY: LINEAR

    (ii) MOLECULE TYPE: PROTEIN
    (xi) SEQUENCE DESCRIPTION: SEQ ID NO:8:

EP 0 970 199 B1

```
Met Leu Leu Pro Val Pro Leu Leu Leu Gly Leu Leu Gly Ala Ala Ala
              5                   10                  15

Asp Val Ser Lys Gly Glu Glu Leu Phe Thr Gly Val Val Pro Ile Leu
             20              25                   30

Val Glu Leu Asp Gly Asp Val Asn Gly His Lys Phe Ser Val Ser Gly
         35                   40                  45

Glu Gly Glu Gly Asp Ala Thr Tyr Gly Lys Leu Thr Leu Lys Phe Ile
     50                   55                   60

Cys Thr Thr Gly Lys Leu Pro Val Pro Trp Pro Thr Leu Val Thr Thr
65                   70                   75                   80

Leu Thr His Gly Val Gln Cys Phe Ser Arg Tyr Pro Asp His Met Lys
             85                   90                   95

Gln His Asp Phe Phe Lys Ser Ala Met Pro Glu Gly Tyr Val Gln Glu
             100              105                  110

Arg Thr Ile Phe Phe Lys Asp Asp Gly Asn Tyr Lys Thr Arg Ala Glu
         115                  120                  125

Val Lys Phe Glu Gly Asp Thr Leu Val Asn Arg Ile Glu Leu Lys Gly
     130                  135                  140

Ile Asp Phe Lys Glu Asp Gly Asn Ile Leu Gly His Lys Leu Glu Tyr
145                  150                  155                  160

Asn Phe Asn Ser His Asn Val Tyr Ile Met Ala Asp Lys Gln Lys Asn
             165                  170                  175

Gly Ile Lys Val Asn Phe Lys Ile Arg His Asn Ile Glu Asp Gly Ser
             180                  185                  190

Val Gln Leu Ala Asp His Tyr Gln Gln Asn Thr Pro Ile Gly Asp Gly
         195                  200                  205

Pro Val Leu Leu Pro Asp Asn His Tyr Leu Ser Thr Gln Ser Ala Leu
     210                  215                  220
```

40

```
Ser Lys Asp Pro Asn Glu Lys Arg Asp His Met Val Leu Leu Glu Phe
225             230             235             240

Val Thr Ala Ala Arg Met His Asp Gln Leu Thr Glu Glu Gln Ile Ala
            245             250             255

Glu Phe Lys Glu Ala Phe Ser Leu Phe Asp Lys Asp Gly Asp Gly Thr
        260             265             270

Ile Thr Thr Lys Glu Leu Gly Thr Val Met Arg Ser Leu Gly Gln Asn
        275             280             285

Pro Thr Glu Ala Glu Leu Gln Asp Met Ile Asn Glu Val Asp Ala Asp
        290             295             300

Gly Asn Gly Thr Ile Tyr Phe Pro Glu Phe Leu Thr Met Met Ala Arg
305             310             315             320

Lys Met Lys Asp Thr Asp Ser Glu Glu Glu Ile Arg Glu Ala Phe Arg
            325             330             335

Val Phe Asp Lys Asp Gly Asn Gly Tyr Ile Ser Ala Ala Gln Leu Arg
            340             345             350

His Val Met Thr Asn Leu Gly Glu Lys Leu Thr Asp Glu Glu Val Asp
            355             360             365

Glu Met Ile Arg Glu Ala Asp Ile Asp Gly Asp Gly Gln Val Asn Tyr
    370             375             380

Glu Glu Phe Val Gln Met Met Thr Ala Lys Gly Gly Lys Arg Arg Trp
385             390             395             400

Lys Lys Asn Phe Ile Ala Val Ser Ala Ala Asn Arg Phe Lys Lys Ile
            405             410             415

Ser Glu Leu Met Val Ser Lys Gly Glu Glu Leu Phe Thr Gly Val Val
            420             425             430

Pro Ile Leu Val Glu Leu Asp Gly Asp Val Asn Gly His Lys Phe Ser
            435             440             445

Val Ser Gly Glu Gly Glu Gly Asp Ala Thr Tyr Gly Lys Leu Thr Leu
    450             455             460

Lys Phe Ile Cys Thr Thr Gly Lys Leu Pro Val Pro Trp Pro Thr Leu
465             470             475             480

Val Thr Thr Leu Thr Tyr Gly Val Gln Cys Phe Ser Arg Tyr Pro Asp
            485             490             495

His Met Lys Gln His Asp Phe Phe Lys Ser Ala Met Pro Glu Gly Tyr
            500             505             510

Val Gln Glu Arg Thr Ile Phe Phe Lys Asp Asp Gly Asn Tyr Lys Thr
    515             520             525

Arg Ala Glu Val Lys Phe Glu Gly Asp Thr Leu Val Asn Arg Ile Glu
    530             535             540

Leu Lys Gly Ile Asp Phe Lys Glu Asp Gly Asn Ile Leu Gly His Lys
545             550             555             560
```

```
Leu Glu Tyr Asn Tyr Asn Ser His Asn Val Tyr Ile Met Ala Asp Lys
            565             570             575

Gln Lys Asn Gly Ile Lys Val Asn Phe Lys Ile Arg His Asn Ile Glu
            580             585             590

Asp Gly Ser Val Gln Leu Ala Asp His Tyr Gln Gln Asn Thr Pro Ile
            595             600             605

Gly Asp Gly Pro Val Leu Leu Pro Asp Asn His Tyr Leu Ser Thr Gln
    610             615             620

Ser Ala Leu Ser Lys Asp Pro Asn Glu Lys Arg Asp His Met Val Leu
625             630             635             640

Leu Glu Phe Val Thr Ala Ala Gly Ile Thr Leu Gly Lys Asp Glu Leu
            645             650             655
```

**Description**

<u>Background of the Invention</u>

The invention relates to fluorescent protein sensors for detecting and quantifying analytes.

Measurement of an analyte concentration *in vitro* or *in vivo* by non-invasive techniques can help elucidate the physiological function of the analyte. This can also aid in identifying changes that occur in a cell or organism in response to physiological stimuli. For example, cyclic AMP can be detected by fluorescence resonance energy transfer between separately labeled proteins that associate with each other but are not covalently attached to each other. See, U.S. Pat. No. 5,439,797.

For example, many effects of $Ca^{2+}$ in cells are mediated by $Ca^{2+}$ binding to calmodulin (CaM), which causes CaM to bind and activate target proteins or peptide sequences. Based on the NMR solution structure of CaM bound to the 26-residue M 13 $Ca^{2+}$ -binding peptide of myosin light-chain kinase, Porumb *et al.* fused the C-terminus of CaM via a Gly-Gly spacer to the M13. $Ca^{2+}$ binding switches the resulting hybrid protein (CaM-M13) from a dumbbell-like extended form to a compact globular form similar to the CaM-M13 intermolecular complex. See, Porumb, T., et al., Prot.Engineering 7:109-115 (1994).

Fluorescent $Ca^{2+}$ indicators such as fura-2, indo-1, fluo-3, and Calcium-Green have been the mainstay of intracellular $Ca^{2+}$ measurement and imaging. See, for example, U.S. Pat. No. 4,603,209 and U.S. Pat. No. 5,049,673. These relatively low molecular weight indicators can suffer from many technical problems relating to ester loading, leakage of the dyes from the cell, compartmentation in organelles, and perturbation of the indicators by cellular constituents. Although the $Ca^{2+}$-indicating photoprotein aequorin is targetable, the photoresponse to $Ca^{2+}$ is low since it is chemiluminescent. Moreover, aequorins need to incorporate exogenous coelenterazine.

U.S. Pat. No. 4,868,103 describes a method for detecting an analyte by complexing it to a binding entity comprising an analyte recognition segment and a first partner of an energy transfer system, wherein the formation of the complex induces or allows for the localization of a reporting entity comprising a component for rendering said analyte detectable and a second partner of the energy transfer system within a proximate distance of the first partner so that energy transfer may occur. The first and second partner of the energy transfer system each may be a fluorescent aromatic agent or a lanthanide metal.

<u>Summary of the Invention</u>

This invention provides fluorescent indicator fusion proteins and methods for using them to determine the concentration of an analyte which is a ligand of a binding protein both in vitro and in vivo. The fluorescent indicator of the invention comprises a binding protein moiety having the ligand binding region of the binding protein and which undergoes a conformational change upon the binding of the ligand; a donor fluorescent protein moiety wherein the donor fluorescent protein moiety is fused to the binding protein moiety; an acceptor fluorescent protein moiety wherein the acceptor fluorescent protein moiety is fused to the binding protein moiety; and wherein the donor fluorescent protein moiety has an emission spectrum which overlaps with the absorption spectrum of the acceptor fluorescent protein moiety and the excitation spectrum of the donor fluorescent moiety comprises a wavelength of excitation at which the donor fluorescent protein moiety can be excited effectively and selectively with respect to the acceptor fluorescent protein moiety; and the emission spectrum of the donor fluorescent protein moiety and the emission spectrum of the acceptor fluorescent protein moiety allow the two emissions to be distinguished, and fluorescence resonance energy transfer (FRET) between the donor fluorescent protein moiety and the acceptor fluorescent protein moiety can be detected when the donor fluorescent protein moiety is excited at the wavelength; and wherein the acceptor fluorescent protein moiety and the donor fluorescent protein moiety change position relative to each other when the ligand binds to the ligand-binding region, altering the FRET.

The donor fluorescent protein moiety and the acceptor fluorescent protein moiety can be *Aequorea*-related fluorescent protein moieties. Preferably, the donor fluorescent protein moiety is P4-3, EBFP, or W1B, and the acceptor fluorescent protein moiety is S65T, EGFP, or 10c.

In preferred embodiments, the indicator further includes a target peptide moiety and a linker moiety that covalently couples the binding protein and the target peptide moiety. The binding protein moiety further includes a peptide-binding region for binding the target peptide moiety. The binding protein moiety can be covalently coupled to the donor fluorescent protein moiety and the target peptide moiety can be covalently coupled to the acceptor fluorescent protein moiety.

In preferred embodiments, one of the donor fluorescent protein moiety or the acceptor fluorescent protein moiety is fused at the carboxy terminus of the fusion protein and the other of the donor fluorescent protein moiety or the acceptor fluorescent protein moiety is fused at the amino terminus of the fusion protein.

The indicator can include a localization sequence. The localization sequence can be a nuclear localization sequence, an endoplasmic reticulum localization sequence, a peroxisome localization sequence, a mitochondrial import sequence, a mitochondrial localization sequence, or a localized protein.

In preferred embodiments, the linker moiety is a peptide moiety. The linker moiety can include between about 1 amino acid residue and about 20 amino acid residues. The linker moiety can be -Gly-Gly-.

Preferably, the binding protein moiety is calmodulin, a calmodulin-related protein moiety, cGMP-dependent protein kinase, a steroid hormone receptor, a ligand binding domain of a steroid hormone receptor, protein kinase C, inositol-1,4,5-triphosphate receptor, or recoverin. A calmodulin-related protein moiety is derived from calmodulin that has been modified to have a different binding affinity for calcium or a target peptide moiety.

Most preferably, the binding protein moiety is calmodulin or a calmodulin-related protein moiety. In these embodiments, the target peptide moiety can be a subsequence of a calmodulin-binding domain of M 13, smMLCKp, CaMKII, Caldesmon, Calspermin, Calcineurin, PhK5, PhK13, C28W, 59-kDa PDE, 60-kDa PDE, NO-30, AC-28, *Bordetella pertussis* AC, Neuromodulin, Spectrin, MARCKS, F52, β-Adducin, HSP90a, HIV-1 gp160, BBMHBI, Dilute MHC, Mastoparan, Melittin, Glucagon, Secretin, VIP, GIP, or Model Peptide CBP2. Preferably, the target peptide moiety is M13.

In another aspect, the invention features a method for determining the concentration of an analyte which is a ligand of a binding protein in a sample. The method includes the steps of contacting the sample with the fluorescent indicator, exciting the donor fluorescent protein moiety, and determining the degree of fluorescence resonance energy transfer in the sample corresponding to the concentration of the analyte in the sample.

The step of determining the degree of fluorescence resonance energy transfer in the sample includes measuring light emitted by the acceptor fluorescent protein moiety.

In preferred embodiments, determining the degree of fluorescence resonance energy transfer in the sample includes measuring light emitted from the donor fluorescent protein moiety, measuring light emitted from the acceptor fluorescent protein moiety, and calculating a ratio of the light emitted from the donor fluorescent protein moiety and the light emitted from the acceptor fluorescent protein moiety.

In other preferred embodiments, determining the degree of fluorescence resonance energy transfer in the sample includes measuring the excited state lifetime of the donor moiety.

The method can further include the steps of determining the concentration of the analyte at a first time after contacting the sample with the fluorescence indicator, determining the concentration of the analyte at a second time after contacting the sample with the fluorescence indicator, and calculating the difference in the concentration of the analyte at the first time and the second time, whereby the difference in the concentration of the analyte in the sample reflects a change in concentration of the analyte present in the sample.

In other embodiments, the method can further include the step of contacting the sample with a compound between the first time and the second time, whereby a difference in the concentration of the analyte in the sample between the first time and the second time indicates that the compound alters the presence of the analyte.

In preferred embodiments, the sample includes an intact cell and the contacting step includes incorporating the fluorescent indicator into the cell. The step of incorporating the fluorescent indicator into the cell can include transfecting the cell with an expression vector comprising expression control sequences operably linked to a nucleic acid sequence coding for the expression of the fluorescent indicator. The preferred analyte is calcium.

In yet another aspect, the invention features an isolated nucleic acid sequence which encodes the fluorescent indicator. In preferred embodiments, an expression vector or a transgenic non-human animal includes the nucleic acid sequence.

In another aspect, the invention features an expression vector including expression control sequences operatively linked to a nucleic acid sequence coding for the expression of the fluorescent indicator. The expression vector can be adapted for function in a prokaryotic cell or a eukaryotic cell.

In another aspect of the invention, a host cell transfected with an expression vector can include an expression control sequence operatively linked to a sequence coding for the expression of the fluorescent indicator. The cell can be a prokaryote, such as E. *coli,* or a eukaryotic cell, such as a yeast cell or mammalian cell.

A transgenic non-human animal having a phenotype characterized by expression of the nucleic acid sequence coding for the expression of the fluorescent indicator can be produced by a method including the steps of: (a) introducing a transgene into a zygote of an animal, the transgene comprising a DNA construct encoding the fluorescent indicator; (b) transplanting the zygote into a pseudopregnant animal; (c) allowing the zygote to develop to term; and (d) identifying at least one transgenic offspring containing the transgene. The step of introducing of the transgene into the embryo can be by introducing an embryonic stem cell containing the transgene into the embryo, or infecting the embryo with a retrovirus containing the transgene. The phenotype is conferred by a transgene contained in the somatic and germ cells of the animal. The animal can be a mouse.

"Peptide" refers to a polymer in which the monomers are amino acid residues which are joined together through amide bonds, alternatively referred to as a polypeptide. A "single polypeptide" is a continuous peptide that constitutes the protein. When the amino acids are alpha-amino acids, either the L-optical isomer or the D-optical isomer can be used, the L-isomers being preferred. Additionally, unnatural amino acids such as beta-alanine, phenylglycine, and homoarginine are meant to be included. Commonly encountered amino acids which are not gene-encoded can also be used in the present invention, although preferred amino acids are those that are encodable. For a general review, see, for example, Spatola, A.F., in Chemistry and Biochemistry of Amino Acids, Peptides and Proteins, B. Weinstein, ed., Marcel Dekker, New York, p. 267 (1983).

"Fluorescent protein" refers to any protein capable of emitting light when excited with appropriate electromagnetic radiation. Fluorescent proteins include proteins having amino acid sequences that are either natural or engineered, such as the fluorescent proteins derived from *Aequorea*-related fluorescent proteins.

In FRET, the "donor fluorescent protein moiety" and the "acceptor fluorescent protein moiety" are selected so that the donor and acceptor moieties exhibit fluorescence resonance energy transfer when the donor moiety is excited. One factor to be considered in choosing the donor/acceptor fluorescent protein moiety pair is the efficiency of FRET between the two moieties. Preferably, the efficiency of FRET between the donor and acceptor moieties is at least 10%, more preferably at least 50%, and most preferably at least 80%. The efficiency of FRET can be tested empirically using the methods described herein and known in the art, particularly, using the conditions set forth in the Examples.

"Covalently coupled" refers to a covalent bond or other covalent linkage between two moieties. The covalent linkage can include a diradical moiety linking to two moieties.

"Binding protein" refers to a protein capable of binding an analyte. Preferred binding proteins change conformation upon binding the analyte. "Target peptide" refers to a peptide that can bind to the binding protein. The target peptide can be a subsequence of a peptide that binds to the binding protein.

"Analyte" refers to a molecule or ion in solution that binds to the binding protein, causing it to change conformation. Preferably, the analyte binds reversibly to the binding protein.

"Moiety" refers to a radical of a molecule that is attached to another radical of the indicator. Thus, a "fluorescent protein moiety" is the radical of a fluorescent protein coupled to a binding protein moiety or a linker moiety, a "binding protein moiety" is a radical of a binding protein coupled to a fluorescent protein moiety, a "target peptide moiety" is a radical of a target peptide of the binding protein, and a "linker moiety" refers to the radical of a molecular linker that is ultimately coupled to both the donor and acceptor fluorescent protein moieties.

"Operatively linked" refers to a juxtaposition wherein the components so described are in a relationship permitting them to function in their intended manner. A control sequence operatively linked to a coding sequence is ligated such that expression of the coding sequence is achieved under conditions compatible with the control sequences. "Control sequence" refers to polynucleotide sequences which are necessary to effect the expression of coding and non-coding sequences to which they are ligated. Control sequences generally includes promoter, ribosomal binding site, and transcription termination sequence. The term "control sequences" is intended to include, at a minimum, components whose presence can influence expression, and can also include additional components whose presence is advantageous, for example, leader sequences and fusion partner sequences.

"Polynucleotide" refers to a polymeric form of nucleotides of at least 10 bases in length. The nucleotides can be ribonucleotides, deoxynucleotides, or modified forms of either type of nucleotide. The term includes single and double stranded forms of DNA.

The invention can have one or more of the following advantages. Ligand-induced conformational changes can be monitored by FRET if, for example, the amino and carboxy termini of the binding protein are fused to a donor and acceptor GFP. This approach has several advantages over the usual covalent labeling with fluorescent probes. The fluorescent indicator can be generated *in situ* by gene transfer into the cells or organisms. This approach avoids the need to express and purify large quantities of soluble recombinant protein, purify and label it *in vitro,* microinject it back into cells. The fluorescent indicator can be targeted to cellular structures. The sites of fusion between the moieties of the fluorescent indicator are exactly defined, giving a molecularly homogenous product without relying on elaborate protein chemistry. In addition, the chromophore of GFP is fixed in the protein. See, Ormo, M., et al., Science 273: 1392-1395 (1996). If the GFP donor and acceptor are fused to a host protein rigidly, minor changes in the relative orientation of the ends of the latter would alter FRET. In contrast, most conventional fluorescent labels are attached by flexible linkers that at least partially decouple the fluorophore orientation from that of the protein to which it is attached, limiting the sensitivity of the FRET measurement.

Other features or advantages of the present invention will be apparent from the following detailed description of the invention, and also from the claims.

Brief Description of the Drawings

FIG. 1 is a schematic diagram depicting a fluorescent indicator that measures the concentration of an analyte by fluorescence resonance energy transfer.

FIG. 2a is a schematic diagram depicting the structure of chimera proteins containing P4-3, CaM-M13, and S65T, where HIS is the amino-terminal tag peptide containing the polyhistidine sequence and XCaM is Xenopus calmodulin.

FIG. 2b is a diagram depicting the amino acid and nucleotide sequences of the boundaries between P4-3 and CaM and between M13 and S65T in cameleon-1.

FIG. 3 is a graph depicting the emission spectra of cameleon-1 before and after addition of 2 mM CaCl$_2$ to give 1 mM free Ca$^{2+}$.

FIG. 4a is a graph depicting the change in emission spectrum of cameieon-1 on titration with Ca$^{2+}$ when excited at 380 nm.

FIG. 4b is a graph depicting Ca$^{2+}$ titration curves of camcleon-1 (open circles) and cameleon-1-/E104Q (solid circles).

FIG. 5 is a schematic diagram depicting the structures of cameleon-2 and cameleon-3, and their derivatives, cameleon-2nu and cameleon-3er, where Kz is Kozak's consensus sequence.

FIG. 6a is a digital fluorescence image depicting fluorescence of cameleon-2 localized to the cytosol of HeLa cells, where the bar is 10 Tm.

FIG. 6b is a graph depicting temporal changes in the emission ratio of cameleon-2 for each of the HeLa cells shown in FIG. 6a.

FIG. 6c is a digital fluorescence image depicting fluorescence of cameleon-2nu localized to the nuclei of HeLa cells, where the bar is 10 Tm.

FIG. 6d is a graph depicting temporal changes in the emission ratio of cameleon-2nu in the two nuclei shown in FIG. 6c.

FIG. 6e is a digital fluorescence image depicting fluorescence of cameleon-3er in transfected HeLa cells, where the bar is 10 Tm.

FIG. 6f is a graph depicting the time-course of emission ratio of cameleon-3er (average of four cells).

FIG. 7 depicts the nucleotide sequence (SEQ ID NO:1) and amino acid sequence (SEQ ID NO:2) of cameleon-2.

FIG. 8 depicts the nucleotide sequence (SEQ ID NO:3) and amino acid sequence (SEQ ID NO:4) of cameleon-2nu.

FIG. 9 depicts the nucleotide sequence (SEQ ID NO:5) and amino acid sequence (SEQ ID NO:6) of cameleon-3.

FIG. 10 is a list depicting the nucleotide sequence (SEQ ID NO:7) and amino acid sequence of cameleon-3er (SEQ ID NO:8).

Detailed Description

A fluorescent indicator that utilizes fluorescent resonance energy transfer ("FRET") to measure the concentration of an analyte includes two fluorescent protein moieties having emission and excitation spectra that render one a donor fluorescent protein moiety and the other an acceptor fluorescent protein moiety. The fluorescent protein moieties are chosen such that the excitation spectrum of one of the moieties (the acceptor fluorescent protein moiety) overlaps with the emission spectrum of the excited protein moiety (the donor fluorescent protein moiety). The donor and acceptor fluorescent protein moieties are bound to a binding protein moiety that changes conformation upon binding the analyte. The change in conformation leads to a change in relative position and orientation of the donor and acceptor fluorescent protein moieties, thereby altering the relative amounts of fluorescence from the two fluorescent protein moieties when the donor is excited by irradiation. In particular, binding of the analyte changes the ratio of the amount of light emitted by the donor and acceptor fluorescent protein moieties. The ratio between the two emission wavelengths provides a measure of the concentration of the analyte in the sample, which is based in part on the binding affinity of the binding protein moiety and the analyte.

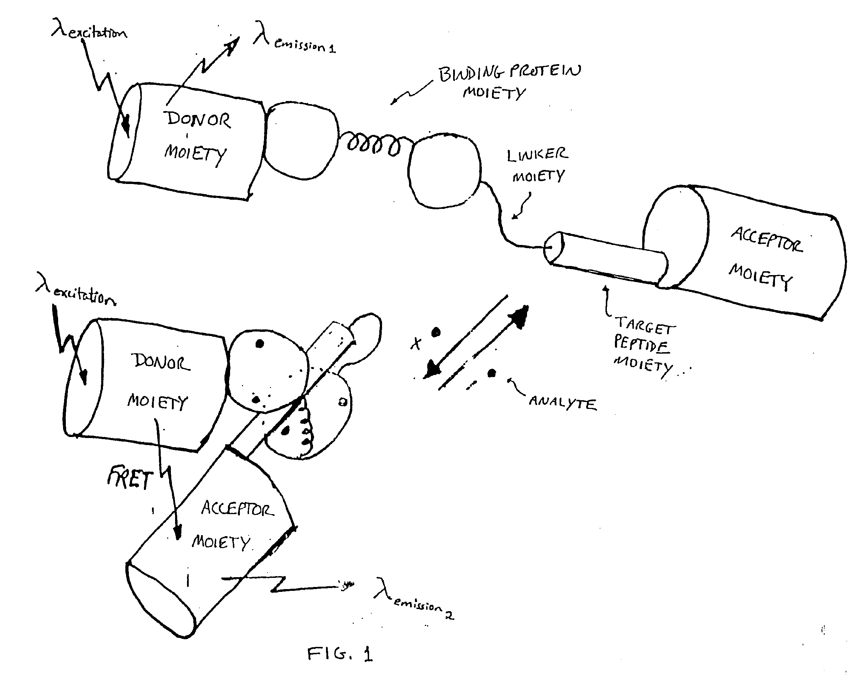

Referring to FIG. 1, the donor fluorescent protein moiety is covalently linked to a first region (e.g., the amino terminus) of the binding protein moiety, and the acceptor fluorescent protein moiety is covalently linked to a second region (e.g., the carboxy terminus) of the binding protein moiety such that the donor and acceptor moieties move closer together upon binding the analyte. Alternatively, the donor and acceptor moieties can move farther apart upon binding the analyte. In one embodiment, depicted in FIG. 1, the acceptor moiety is covalently bonded to a target peptide moiety that also binds to the binding protein moiety and the target peptide moiety is covalently bonded to the binding protein moiety by a linker moiety. The linker moiety is flexible enough to allow the target peptide moiety to bind to the binding protein moiety. The donor moiety is excited by light of appropriate intensity within the excitation spectrum of the donor moiety ($\Sigma_{excitation}$). The donor moiety emits .the absorbed energy as fluorescent light ($\Sigma_{emission\ 1}$). When the acceptor fluorescent protein moiety is positioned to quench the donor moiety in the excited state, the fluorescence energy is transferred to the acceptor moiety which can emit fluorescent light ($\Sigma_{emission\ 2}$). FRET can be manifested as a reduction in the intensity of the fluorescent signal from the donor moiety ($\Sigma_{emission\ 1}$), reduction in the lifetime of the excited state of the donor moiety, or emission of fluorescent light at the longer wavelengths (lower energies) characteristic of the acceptor moiety ($\Sigma_{emission\ 2}$). When the conformation of the binding protein moiety changes upon binding the analyte, the fluorescent protein moieties come closer together (or physically separate), and FRET is increased (or decreased) accordingly.

The efficiency of FRET depends on the separation distance and the orientation of the donor and acceptor fluorescent protein moieties. For example, the Forster equation describes the efficiency of excited state energy transfer, based in part on the fluorescence quantum yield of the donor moiety and the energetic overlap with the acceptor moiety. The Forster equation is:

$$E = (F_0 - F)/F_0 = R_0^6/(R^6 + R_0^6)$$

where E is the efficiency of FRET, F and $F_0$ are the fluorescence intensities of the donor moiety in the presence and absence of the acceptor, respectively, and R is the distance between the donor moiety and the acceptor moiety.

The characteristic distance $R_0$ at which FRET is 50% efficient depends on the quantum yield of the donor moiety (i.e., the shorter-wavelength fluorophore), the extinction coefficient of the acceptor moiety (i.e., the longer-wavelength fluorophore), and the overlap between the emission spectrum of the donor moiety and the excitation spectrum of the acceptor moiety. $R_0$ is given (in D) by

$$R_0 = 9.79 \times 10^3 (K^2 Q J n^{-4})^{1/6}$$

where $K^2$ is an orientation factor having an average value close to 0.67 for freely mobile donors and acceptors, Q is the quantum yield of the unquenched donor moiety, n is the refractive index of the medium separating the donor moiety and the acceptor moiety, and J is the overlap integral. J can be quantitatively expressed as the degree of spectral overlap between the donor moiety and the acceptor moiety according to the equation:

$$J = I^4_0 M_\Sigma F_\Sigma \Sigma^4 d\Sigma / I^4_0 F_\Sigma d\Sigma$$

where $M_\Sigma$ ($M^{-1}cm^{-1}$) is the molar absorptivity of the acceptor and $F_\Sigma$ is the donor moiety fluorescence intensity at wavelength E. See, for example, Forster, T. Ann.Physik 2:55-75 (1948). Tables of spectral overlap integrals are readily available to those working in the field (for example, Berlman, I.B. Energy transfer parameters of aromatic compounds, Academic Press, New York and London (1973)). FRET is a nondestructive spectroscopic method that can monitor proximity and relative angular orientation of fluorophores in living cells. See, for example, Adams, S.R., et al., Nature 349:694-697 (1991), and Gonzalez, J. & Tsien, R.Y. Biophy. J. 69:1272-1280 (1995).

These factors need to be balanced to optimize the efficiency and detectability of FRET from the fluorescent indicator. The emission spectrum of the donor fluorescent protein moiety should overlap as much as possible with the excitation spectrum of the acceptor fluorescent protein moiety to maximize the overlap integral J. Also, the quantum yield of the donor fluorescent protein moiety and the extinction coefficient of the acceptor fluorescent protein moiety should be as large as possible to maximize $R_0$. In addition, the excitation spectra of the donor and acceptor moieties should overlap as little as possible so that a wavelength region can be found at which the donor moiety can be excited selectively and efficiently without directly exciting the acceptor moiety. Direct excitation of the acceptor moiety should be avoided since it can be difficult to distinguish direct emission from fluorescence arising from FRET. Similarly, the emission spectra of the donor and acceptor moieties should have minimal overlap so that the two emissions can be distinguished. High fluorescence quantum yield of the acceptor moiety is desirable if the emission from the acceptor moiety is to be monitored to determine analyte concentration in a sample. In a preferred embodiment, the donor fluorescent protein moiety is excited by ultraviolet (<400 nm) and emits blue light (<500 nm), and the acceptor fluorescent protein moiety is efficiently excited by blue but not by ultraviolet light and emits green light (>500 nm), for example, P4-3 and S65T, respectively.

In another preferred embodiment, the donor fluorescent moiety is excited by violet (400-430 nm) and emits blue-green (450-500 nm) and the acceptor fluorescent moiety is efficiently excited by blue-green (450-500 nm) and emits yellow-green light (520 nm), for example WIB and 10C respectively.

The amount of analyte in a sample can be determined by determining the degree of FRET in the sample. Changes in analyte concentration can be determined by monitoring FRET at a first and second time after contact between the sample and the fluorescent indicator and determining the difference in the degree of FRET. The amount of analyte in the sample can be calculated by using a calibration curve established by titration.

The degree of FRET can be determined by any spectral or fluorescence lifetime characteristic of the excited donor moiety. For example, intensity of the fluorescent signal from the donor, the intensity of fluorescent signal from the acceptor, the ratio of the fluorescence amplitudes near the acceptor's emission maxima to the fluorescence amplitudes near the donor's emission maximum, or the excited state lifetime of the donor can be monitored.

Preferably, changes in the degree of FRET are determined as a function of the change in the ratio of the amount of fluorescence from the donor and acceptor moieties, a process referred to as "ratioing." Changes in the absolute amount of indicator, excitation intensity, and turbidity or other background absorbances in the sample at the excitation wavelength affect the intensities of fluorescence from both the donor and acceptor approximately in parallel. Therefore the ratio of the two emission intensities is a more robust and preferred measure of cleavage than either intensity alone.

Fluorescence in a sample is measured using a fluorometer. In general, excitation radiation, from an excitation source having a first wavelength, passes through excitation optics. The excitation optics cause the excitation radiation to excite the sample. In response, fluorescent proteins in the sample emit radiation which has a wavelength that is different from the excitation wavelength. Collection optics then collect the emission from the sample. The device can include a temperature controller to maintain the sample at a specific temperature while it is being scanned. According to one embodiment, a multi-axis translation stage moves a microtiter plate holding a plurality of samples in order to position different wells to be exposed. The multi-axis translation stage, temperature controller, auto-focusing feature, and electronics associated with imaging and data collection can be managed by an appropriately programmed digital computer. The computer also can transform the data collected during the assay into another format for presentation.

Methods of performing assays on fluorescent materials are well known in the art and are described in, *e.g.*, Lakowicz, J.R., Principles of Fluorescence Spectroscopy, New York:Plenum Press (1983); Herman, B., Resonance energy transfer microscopy, in: Fluorescence Microscopy of Living Cells in Culture, Part B, Methods in Cell Biology, vol. 30, ed. Taylor, D.L. & Wang, Y.-L., San Diego: Academic Press (1989), pp. 219-243; Turro, N.J., Modern Molecular Photochemistry, Menlo Park: Benjamin/Cummings Publishing Col, Inc. (1978), pp. 296-361.

The excited state lifetime of the donor moiety is, likewise, independent of the absolute amount of substrate, excitation intensity, or turbidity or other background absorbances. Its measurement requires equipment with nanosecond time resolution.

Quantum yields of wild-type GFP, S65T, and P4-1 mutants can be estimated by comparison with fluorescein in 0.1 N NaOH as a standard of quantum yield 0.91. J.N. Miller, ed., Standards in Fluorescence Spectrometry, New York: Chapman and Hall (1981). Mutants P4 and P4-3 were likewise compared to 9-aminoacridine in water (quantum yield 0.98).

Any fluorescent protein can be used in the invention, including proteins that fluoresce due intramolecular rearrangements or the addition of cofactors that promote fluorescence. For example, green fluorescent proteins of cnidarians, which act as their energy-transfer acceptors in bioluminescence, are suitable fluorescent proteins for use in the fluorescent indicators. A green fluorescent protein ("GFP") is a protein that emits green light, and a blue fluorescent protein ("BFP") is a protein that emits blue light. GFPs have been isolated from the Pacific Northwest jellyfish, *Aequorea victoria,* the sea pansy, *Renilla reniformis,* and *Phialidium gregarium.* See, Ward, W.W., et al., Photochem. Photobiol., 35:803-808 (1982); and Levine, L.D., et al., Comp. Biochem. Physiol., 72B:77-85 (1982).

A variety of *Aequorea*-related GFPs having useful excitation and emission spectra have been engineered by modifying the amino acid sequence of a naturally occurring GFP from *Aequorea victoria.* See, Prasher, D.C., et al., Gene, 111:229-233 (1992); Heim, R., et al., Proc. Natl. Acad Sci., USA, 91:12501-04 (1994); U.S. Ser. No. 08/337,915, filed November 10, 1994; International application PCT/US95/14692, filed 11/10/95; and U.S. Ser. No. 08/706,408, filed August 30, 1996. The cDNA of GFP can be concatenated with those encoding many other proteins; the resulting fusions often are fluorescent and retrain the biochemical features of the partner proteins. See, Cubitt, A.B., et al., Trends Biochem. Sci. 20:448-455 (1995). Mutagenesis studies have produced GFP mutants with shifted wavelengths of excitation or emission. See, Heim, R. & Tsien, R.Y. Current Biol. 6:178-182 (1996). Suitable pairs, for example a blue-shifted GFP mutant P4-3 (Y66H/Y145F) and an improved green mutant S65T can respectively serve as a donor and an acceptor for fluorescence resonance energy transfer (FRET). See, Tsien, R.Y., et al., Trends Cell Biol. 3:242-245 (1993). A fluorescent protein is an *Aequorea*-related fluorescent protein if any contiguous sequence of 150 amino acids of the fluorescent protein has at least 85% sequence identity with an amino acid sequence, either contiguous or non-contiguous, from the wild type *Aequorea* green fluorescent protein. More preferably, a fluorescent protein is an *Aequorea*-related fluorescent protein if any contiguous sequence of 200 amino acids of the fluorescent protein has at least 95% sequence identity with an amino acid sequence, either contiguous or non-contiguous, from the wild type *Aequorea* green fluorescent protein. Similarly, the fluorescent protein can be related to *Renilla* or *Phialidium* wild-type fluorescent proteins using the same standards. Some *Aequorea*-related engineered versions are described in Table I. Other variants or mutants are within the scope of the invention as described, for example, in the Examples.

TABLE I

| *Clone* | *Mutation(s)* | *Excitation max (nm)* | *Emission max (nm)* | *Extinction Coefficient (M⁻¹ cm⁻¹)* | *Quantum yield* |
|---|---|---|---|---|---|
| *Wild type* | none | 395 (475) | 508 | 21,000 (7,150) | 0.77 |
| *p4* | Y66H | 383 | 447 | 13.500 | 0.21 |
| *P4-3* | Y66H;Y145F | 381 | 445 | 14,000 | 0.38 |
| *W7* | Y66W;N1461 M153T V163A N212K | 433 (453) | 475(501) | 18,000 (17,100) | 0.67 |
| *W2* | Y66W;1123V Y145H H148R My V163A N212K | 432 (453) | 480 | 10,000 (9,600) | 0.72 |
| *S65T* | S65T | 489 | 511 | 39,200 | 0.68 |
| *p4-1* | S65T;M153A K238E | 504 (396) | 514 | 14,500 (8,600) | 0.53 |
| *S65A* | S65A | 471 | 504 | | |
| *S65C* | S65C | 479 | 507 | | |
| *S65L* | S65L | 484 | 510 | | |
| *Y66F* | Y66F | 360 | 442 | | |
| *Y66W* | Y66W | 458 | 480 | | |
| *10c* | S65G;V68L S72A: T203Y | 513 | 527 | | |
| *W1B* | F64L;S65T Y66W;N1461 M153T V163A N212K | 432 (453) | 476 (503) | | |
| *Emerald* | S65T;S72A N149K M153T 1167T | 487 | 508 | | |
| *Sapphire* | S72A;Y145F T2031 | 395 | 511 | | |

An additional clone, W1B1 included the following mutations: F64L; S65T; Y66W; F99S; and V163A.

Other fluorescent proteins can be used in the fluorescent indicators, such as, for example, yellow fluorescent protein from *Vibrio fischeri* strain Y-1, Peridinin-chlorophyll a binding protein from the dinoflagellate *Symbiodinium* sp., phycobiliproteins from marine cyanobacteria such as *Synechococcus,* e.g., phycoerythrin and phycocyanin, or oat phytochromes from oat reconstructed with phycoerythrobilin. These fluorescent proteins have been described in Baldwin, T.O., et al., Biochemistry 29:5509-5515 (1990), Morris, B.J., et al., Plant Molecular Biology, 24:673-677 (1994), and Wilbanks, S.M., et al., J. Biol. Chem. 268:1226-1235 (1993), and Li et al., Biochemistry 34:7923-7930 (1995).

The efficiency of FRET between the donor and acceptor fluorescent protein moieties can be adjusted by changing ability of the two fluorescent proteins to closely associate. The nature of the binding protein moiety, target peptide moiety, and linker moiety each affect the FRET and the response of the indicator to the analyte. Generally, large conformational changes in the binding protein moiety are desired along with a high affinity for the target peptide moiety.

The binding protein moiety is a protein, or part thereof, that changes conformation upon binding an analyte. Proteins that undergo useful conformation change upon binding an analyte include calmodulin (CaM), cGMP-dependent protein kinase, steroid hormone receptors (or ligand binding domain thereof), protein kinase C, inositol-1,4,5-triphosphate receptor, or recoverin. See, for example, Katzenellenbogen, J.A. & Katzenellenbogen, B.S. Chemistry & Biology 3: 529-536 (1996), and Ames, J.B., et al., Curr. Opin. Struct. Biol. 6:432-438 (1996). The binding protein moiety preferably binds target peptides in addition to the analyte. The $Ca^{2+}$-binding affinity of calmodulin can be tuned as reviewed, for example, in Falke, J.J., et al., Quart. Rev. Biophys. 27:219-290 (1994).

The target peptide moiety can contain any of the amino acid sequences in Table II, or a portion thereof with the proviso that the target peptide must bind to the binding protein moiety. The target peptide can be a subsequence of a calmodulin-binding domain. The target peptide moieties listed in Table II are recognized by the binding protein moiety CaM. See, for example, Crivici, A. & Ikura, M. Annu. Rev. Biophys Biomol. Struct. 24:84-116 (1995). The target peptide moiety can be modified to enhance the response of the fluorescent indicator to the analyte. Other target peptide moieties are known in the art for other binding proteins.

TABLE II

| Target[a] | Sequence |
|---|---|
| skMLCK (M13) | KRRWKKNFIAVSAANRFKKISSSGAL |
| smMLCK (smMLCKp) | ARRKWQKTGHAVRAIGRLSS |
| CAMKII, | ARRKLKGAILTTMLATRNFS |
| Caldesmon | GVRNIKSMWEKGNVFSS |
| Calspermin | ARRKLKAAVKAVVASSRLGS |
| PFK (M11) | FMNNWEVYKLLAHIRPPAPKSGSYTV |
| Calcineurin | ARKEVIRNKIRAIGKMARVFSVLR |
| PhK(PhK5) | LRRLIDAYAFRIYGHWVKKGQQQNRG |
| (PhK13) | RGKFKVICLTVLASVRIYYQYRRVKPG |
| $Ca^{2+}$-ATPase (C28W) | LRRGQILWFRGLNRIQTQIKVVNAFSSS |
| 59-kDa PDE | RRKHLQRPIFRLRCLVKQLEK |
| 60-kDa PDE | TEKMWQRLKGILRCLVKQLEK |
| NOS (NO-30) | KRRAIGFKKLAEAVKFSAKLMGQ |
| Type I AC (AC-28) | IKPAKRMKFKTVCYLLVQLMHCRKMFKA |
| *Borderella periussis* AC | IDLLWKIARAGARSAVGTEA |
| Neuromodulin | KAHKAATKIQASFRGHITRKKLKGEKK |
| Spectrin | KTASPWKSARLMVHTVATFNSIKE |
| MARCKS | KKKKKRFSFKKSFKLSGFSFKKSKK |
| F52 or MacMARKS | KKKKKFSFKKPFKLSGLSFKRNRX |
| β-Adducin | KQQKEKTRWLNTPNTYLRVNVADEVQRNMGS |
| HSP90a | KDQVANSAFQERLRKHGLEVI |
| HIV-1 gp160 | YHRLRDLLLIVKRIVELLGRR |
| BBMHBI | QQLATLIQKTYRGWRCRTHYQLM |
| Dilute MHC | RAACIRIQKTIRGWLLRKRYLCMQ |
| Mastoparan | INLKALAALAKKIL |
| Melittin | GIGAVLKVLTTGLPALISWIKRKRQQ |
| Glucagon | HSQGTFTTSDYSKYLDSRRAQDFVQWLMNT |

(continued)

| Target[a] | Sequence |
|---|---|
| Secretin | HSDGTFTSELSRLRDSARLQRLLQGLV |
| VIP | HSDAVFTDNYTRLRKQMAVKKYLNSILN |
| GIP | YADGTFISDYSAIMNKIRQQDFVNWLLAQQQKS |
| Model Peptide CBP2 | KLWKKLLKLLKKLLKLG |

[a] Abbreviations: AC, adenylyl cyclase; BBMHCI, brush-border myosin heavy chain-I; CaMKII, calmodulin kinase II; CBP2, calmodulin binding peptide-2; GIP, gastrin inhibitory peptide; HTV-1 gp160, human immunodeficiency virus envelope glycoprotein 160; HSP, heat-shock protein; MARCKS, myristoylated alaminte-rich C kinase substrate; MHC, myosin heavy chain; NOS, nitric oxide synthase; PDE, phosphodiesterase; PFK, phosphofructokinase; PhK, phosphorylase kinase; sk-, smMLCK, skeletal muscle- and smooth muscle-myosin light chain kinase; VIP, vasoactive intestinal peptide.

The length of the linker moiety is chosen to optimize both FRET and the kinetics and specificity of conformational changes induced by analyte binding. The linker moiety should be long enough and flexible enough to allow the binding protein moiety and target peptide moiety to freely interact and respond to analyte concentration. In order to optimize the FRET effect, the average distance between the donor and acceptor fluorescent protein moieties should become between about 1 nm and about 10 nm, preferably between about 1 nm and about 6 nm, and more preferably between about 1 nm and about 4 nm, when the analyte is bound (or released). If the linker is too short or too stiff, the donor and acceptor protein moieties cannot readily change position. If the linker moiety is too long, the target peptide moiety might not bind to the binding protein moiety effectively. The linker moiety is, preferably, a peptide moiety. The preferred linker moiety is a peptide between about one and 30 amino acid residues in length, preferably between about two and 15 amino acid residues. One preferred linker moiety is a -Gly-Gly- linker.

The linker moiety can include flexible spacer amino acid sequences, such as those known in single-chain antibody research. For example, the linker moiety can be GGGGS (GGGGS)$_n$, GKSSGSGSESKS, GSTSGSGKSSEG-KG, GSTSGSGKSSEGSGSTKG; GSTSGSGKSSEGKG, GSTSGSGKPGSGEGSTKG, or EGKSSGSGSESKEF. Linking moieties are described, for example, in Huston, J.S., et al., PNAS 85:5879-5883 (1988), Whitlow, M., et al., Protein Engineering 6:989-995 (1993), and Newton, D.L., et a/., Biochemistry 35:545-553 (1996).

The fluorescent indicators can also include a localization sequence to direct the indicator to particular cellular sites by fusion to appropriate organellar targeting signals or localized host proteins. A polynucleotide encoding a localization sequence, or signal sequence, can be ligated or fused at the 5' terminus of a polynucleotide encoding the fluorescence indicator such that the signal peptide is located at the amino terminal end of the resulting fusion polynucleotide/polypeptide. In the case of eukaryotes, the signal peptide is believed to function to transport the fusion polypeptide across the endoplasmic reticulum. The secretory protein is then transported through the Golgi apparatus, into secretory vesicles and into the extracellular space or, preferably, the external environment. Signal peptides which can be utilized according to the invention include pre-pro peptides which contain a proteolytic enzyme recognition site. Other signal peptides with similar properties to pro-calcitonin described herein are known to those skilled in the art, or can be readily ascertained without undue experimentation.

The localization sequence can be a nuclear localization sequence, an endoplasmic reticulum localization sequence, a peroxisome localization sequence, a mitochondrial localization sequence, or a localized protein. Localization sequences can be targeting sequences which are described, for example, in "Protein Targeting", chapter 35 of Stryer, L., Biochemistry (4th ed.). W.H. Freeman, 1995. The localization sequence can also be a localized protein. Some important localization sequences include those targeting the nucleus (KKKRK), mitochondrion (amino terminal MLRTSSLFTRRVQPSLFRNILRLQST-), endoplasmic reticulum (KDEL at C-terminus, assuming a signal sequence present at N-terminus), peroxisome (SKF at C-terminus), prenylation or insertion into plasma membrane (CaaX, CC, CXC, or CCXX at C-terminus), cytoplasmic side of plasma membrane (fusion to SNAP-25), or the Golgi apparatus (fusion to furin).

The fluorescent indicators can be produced as fusion proteins by recombinant DNA technology. Recombinant production of fluorescent proteins involves expressing nucleic acids having sequences that encode the proteins. Nucleic acids encoding fluorescent proteins can be obtained by methods known in the art. For example, a nucleic acid encoding the protein can be isolated by polymerase chain reaction of cDNA from *A. victoria* using primers based on the DNA sequence of *A. victoria* green fluorescent protein. PCR methods are described in, for example, U.S. Pat. No. 4,683,195; Mullis, et al. Cold Spring Harbor Symp. Quant. Biol. 51:263 (1987), and Erlich, ed., PCR Technology, (Stockton Press, NY, 1989). Mutant versions of fluorescent proteins can be made by site-specific mutagenesis of other nucleic acids encoding fluorescent proteins, or by random mutagenesis caused by increasing the error rate of PCR of the original polynucleotide with 0.1 mM $MnCl_2$ and unbalanced nucleotide concentrations. See, *e.g.*, U.S. patent application 08/337,915, filed November 10, 1994 or International application PCT/US95/14692, filed 11/10/95.

The construction of expression vectors and the expression of genes in transfected cells involves the use of molecular cloning techniques also well known in the art. Sambrook et al., Molecular Cloning - A Laboratory Manual, Cold Spring Harbor Laboratory, Cold Spring Harbor; Nay, (1989) and Current Protocols in Molecular Biology; F.M. Ausubel et al., eds., (Current Protocols, a joint venture between Greene Publishing Associates, Inc. and John Wiley & Sons, Inc., most recent Supplement).

Nucleic acids used to transfect cells with sequences coding for expression of the polypeptide of interest generally will be in the form of an expression vector including expression control sequences operatively linked to a nucleotide sequence coding for expression of the polypeptide. As used, the term "nucleotide sequence coding for expression of" a a polypeptide refers to a sequence that, upon transcription and translation of mRNA, produces the polypeptide. This can include sequences containing, e.g., introns. As used herein, the term "expression control sequences" refers to nucleic acid sequences that regulate the expression of a nucleic acid sequence to which it is operatively linked. Expression control sequences are operatively linked to a nucleic acid sequence when the expression control sequences control and regulate the transcription and, as appropriate, translation of the nucleic acid sequence. Thus, expression control sequences can include appropriate promoters, enhancers, transcription terminators, a start codon (i.e., ATG) in front of a protein-encoding gene, splicing signals for introns, maintenance of the correct reading frame of that gene to permit proper translation of the mRNA, and stop cordons.

Methods which are well known to those skilled in the art can be used to construct expression vectors containing the fluorescent indicator coding sequence and appropriate transcriptional/translational control signals. These methods include *in vitro* recombinant DNA techniques, synthetic techniques and *in vivo* recombination/genetic recombination. (See, for example, the techniques described in Maniatis, et al., Molecular Cloning A. Laboratory Manual, Cold Spring Harbor Laboratory, N.Y., 1989).

Transformation of a host cell with recombinant DNA may be carried out by conventional techniques as are well known to those skilled in the art. Where the host is prokaryotic, such as *E. coli,* competent cells which are capable of DNA uptake can be prepared from cells harvested after exponential growth phase and subsequently treated by the $CaCl_2$ method by procedures well known in the art. Alternatively, $MgCl_2$ or RbCl can be used. Transformation can also be performed after forming a protoplast of the host cell or by electroporation.

When the host is a non-human eukaryote, such methods of transfection of DNA as calcium phosphate co-precipitates, conventional mechanical procedures such as microinjection, electroporation, insertion of a plasmid encased in liposomes, or virus vectors may be used. Eukaryotic cells can also be cotransfected with DNA sequences encoding the fusion polypeptide of the invention, and a second foreign DNA molecule encoding a selectable phenotype, such as the herpes simplex thymidine kinase gene. Another method is to use a eukaryotic viral vector, such as simian virus 40 (SV40) or bovine papilloma virus, to transiently infect or transform eukaryotic cells and express the protein. (Eukaryotic Viral Vectors, Cold Spring Harbor Laboratory, Gluzman ed., 1982). Preferably, a non-human eukaryotic host is utilized as the host cell as described herein.

Techniques for the isolation and purification of either microbially or eukaryotically expressed polypeptides of the invention may be by any conventional means such as, for example, preparative chromatographic separations and immunological separations such as those involving the use of monoclonal or polyclonal antibodies or antigen.

A variety of non-human host-expression vector systems may be utilized to express fluorescent indicator coding sequence. These include but are not limited to microorganisms such as bacteria transformed with recombinant bacteriophage DNA, plasmid DNA or cosmid DNA expression vectors containing a fluorescent indicator coding sequence; yeast transformed with recombinant yeast expression vectors containing the fluorescent indicator coding sequence; plant cell systems infected with recombinant virus expression vectors (*e.g.*, cauliflower mosaic virus, CaMV; tobacco mosaic virus, TMV) or transformed with recombinant plasmid expression vectors (*e.g.*, Ti plasmid) containing a fluorescent indicator coding sequence; insect cell systems infected with recombinant virus expression vectors (*e.g.*, baculovirus) containing a fluorescent indicator coding sequence; or animal cell systems infected with recombinant virus expression vectors (*e.g.*, retroviruses, adenovirus, vaccinia virus) containing a fluorescent indicator coding sequence, or transformed animal cell systems engineered for stable expression.

Depending on the host/vector system utilized, any of a number of suitable transcription and translation elements, including constitutive and inducible promoters, transcription enhancer elements, transcription terminators, etc. may be used in the expression vector (see, *e.g.*, Bitter, et al., Methods in Enzymology 153:516-544, 1987). For example, when cloning in bacterial systems, inducible promoters such as pL of bacteriophage S, plac, ptrp, ptac (ptrp-lac hybrid promoter) and the like may be used. When cloning in mammalian cell systems, promoters derived from the genome of mammalian cells (*e.g.*, metallothionein promoter) or from mammalian viruses (*e.g.*, the retrovirus long terminal repeat; the adenovirus late promoter; the vaccinia virus 7.5K promoter) may be used. Promoters produced by recombinant DNA or synthetic techniques may also be used to provide for transcription of the inserted fluorescent indicator coding sequence.

In bacterial systems a number of expression vectors may be advantageously selected depending upon the use intended for the fluorescent indicator expressed. For example, when large quantities of the fluorescent indicator are to be produced, vectors which direct the expression of high levels of fusion protein products that are readily purified may be desirable. Those which are engineered to contain a cleavage site to aid in recovering fluorescent indicator are preferred.

In yeast, a number of vectors containing constitutive or inducible promoters may be used. For a review see, Current Protocols in Molecular Biology, Vol. 2, Ed. Ausubel, et al., Greene Publish. Assoc. & Wiley Interscience, Ch. 13, 1988; Grant, et al., Expression and Secretion Vectors for Yeast, in Methods in Enzymology, Eds. Wu & Grossman, 31987, Acad. Press, N.Y., Vol. 153, pp.516-544, 1987; Glover, DNA Cloning, Vol. II, IRL Press, Wash., D.C., Ch. 3, 1986; and Bitter, Heterologous Gene Expression in Yeast, Methods in Enzymology, Eds. Berger & Kimmel, Acad. Press, N.Y., Vol. 152, pp. 673-684, 1987; and The Molecular Biology of the Yeast Saccharomyces, Eds. Strathern et al., Cold Spring Harbor Press, Vols. I and II, 1982. A constitutive yeast promoter such as ADH or LEU2 or an inducible promoter such as GAL may be used (Cloning in Yeast, Ch. 3, R Rothstein In: DNA Cloning Vol. 11, A Practical Approach, Ed. DM Glover, IRL Press, Wash., D.C., 1986). Alternatively, vectors may be used which promote integration of foreign DNA sequences into the yeast chromosome.

In cases where plant expression vectors are used, the expression of a fluorescent indicator coding sequence may be driven by any of a number of promoters. For example, viral promoters such as the 35S RNA and 19S RNA promoters of CaMV (Brisson, et a/., Nature 310:511-514,1984), or the coat protein promoter to TMV (Takamatsu, et al., EMBO J. 6:307-311, 1987) may be used; alternatively, plant promoters such as the small subunit of RUBISCO (Coruzzi, et al., 1984, EMBO J. 3:1671-1680; Broglie, et al., Science 224:838-843, 1984); or heat shock promoters, e.g., soybean hsp17.5-E or hsp17.3-B (Gurley, et al., Mol. Cell. Biol. 6:559-565, 1986) may be used. These constructs can be introduced into plant cells using Ti plasmids, Ri plasmids, plant virus vectors, direct DNA transformation, microinjection, electroporation, *etc.* For reviews of such techniques see, for example, Weissbach &. Weissbach, Methods for Plant Molecular Biology, Academic Press, NY, Section VIII, pp. 421-463, 1988; and Grierson & Corey, Plant Molecular Biology, 2d Ed., Blackie, London, Ch. 7-9, 1988.

An alternative expression system which could be used to express fluorescent indicator is an insect system. In one such system, *Autographa californica* nuclear polyhedrosis virus (AcNPV) is used as a vector to express foreign genes. The virus grows in *Spodoptera frugiperda* cells. The fluorescent indicator coding sequence may be cloned into non-essential regions (for example, the polyhedrin gene) of the virus and placed under control of an AcNPV promoter (for example the polyhedrin promoter). Successful insertion of the fluorescent indicator coding sequence will result in inactivation of the polyhedrin gene and production of non-occluded recombinant virus (*i.e.*, virus lacking the proteinaceous coat coded for by the polyhedrin gene). These recombinant viruses are then used to infect *Spodoptera frugiperda* cells in which the inserted gene is expressed, see Smith, et al., J. Viol. 46:584, 1983; Smith, U.S. Patent No. 4,215,051.

Eukaryotic systems, and preferably mammalian expression systems, allow, for proper post-translational modifications of expressed mammalian proteins to occur. Eukaryotic cells which possess the cellular machinery for proper processing of the primary transcript, glycosylation, phosphorylation, and, advantageously secretion of the gene product should be used as host cells for the expression of fluorescent indicator. Such host cell lines may include but are not limited to CHO, VERO, BHK, HeLa, COS, MDCK, Jurkat, HEK-293, and WI38.

Mammalian cell systems which utilize recombinant viruses or viral elements to direct expression may be engineered. For example, when using adenovirus expression vectors, the fluorescent indicator coding sequence may be ligated to an adenovirus transcription/translation control complex, *e.g.*, the late promoter and tripartite leader sequence. This chimeric gene may then be inserted in the adenovirus genome by *in vitro* or *in vivo* recombination. Insertion in a non-essential region of the viral genome (*e.g.*, region E1 or E3) will result in a recombinant virus that is viable and capable of expressing the fluorescent indicator in infected hosts (*e.g.,* see Logan & Shenk, Proc. Natl. Acad Sci. USA, 81: 3655-3659, 1984). Alternatively, the vaccinia virus 7.5K promoter may be used. (*e.g.,* see, Mackett, et al., Proc. Natl. Acad Sci. USA, 79: 7415-7419, 1982; Mackett, et al., J. Virol. 49: 857-864, 1984; Panicali, et al., Proc. Natl. Acad Sci. USA 79: 4927-4931, 1982). Of particular interest are vectors based on bovine papilloma virus which have the ability to replicate as extrachromosomal elements (Sarver, et al., Mol. Cell. Biol. 1: 486,1981). Shortly after entry of this DNA into mouse cells, the plasmid replicates to about 100 to 200 copies per cell. Transcription of the inserted cDNA does not require integration of the plasmid into the host's chromosome, thereby yielding a high level of expression. These vectors can be used for stable expression by including a selectable marker in the plasmid, such as the *neo* gene. Alternatively, the retroviral genome can be modified for use as a vector capable of introducing and directing the expression of the fluorescent indicator gene in host cells (Cone & Mulligan, Proc. Natl. Acad Sci. USA, 81:6349-6353, 1984). High level expression may also be achieved using inducible promoters, including, but not limited to, the metallothionine IIA promoter and heat shock promoters.

For long-term, high-yield production of recombinant proteins, stable expression is preferred. Rather than using expression vectors which contain viral origins of replication, host cells can be transformed with the fluorescent indicator cDNA controlled by appropriate expression control elements (*e.g.*, promoter, enhancer sequences, transcription terminators, polyadenylation sites, *etc.*)*,* and a selectable marker. The selectable marker in the recombinant plasmid confers resistance to the selection and allows cells to stably integrate the plasmid into their chromosomes and grow to form foci which in turn can be cloned and expanded into cell lines. For example, following the introduction of foreign DNA, engineered cells may be allowed to grow for 1-2 days in an enriched media, and then are switched to a selective media. A number of selection systems may be used, including but not limited to the herpes simplex virus thymidine kinase (Wigler, et al., Cell, 11: 223, 1977), hypoxanthine-guanine phosphoribosyltransferase (Szybalska & Szybalski, Proc. Natl. Acad Sci. USA, 48:2026, 1962), and adenine phosphoribosyltransferase (Lowy, et al., Cell, 22: 817, 1980) genes which can be employed in tk-, hgprt- or aprt- cells, respectively. Also, antimetabolite resistance can be used as the basis of selection: dhfr, which confers resistance to methotrexate (Wigler, et al., Proc. Natl. Acad. Sci. USA, 77: 3567, 1980; O'Hare, et al., Proc. Natl. Acad. Sci. USA, 8: 1527, 1981); gpt, which confers resistance to mycophenolic acid (Mulligan & Berg, Proc. Natl. Acad. Sci. USA, 78: 2072, 1981); neo, which confers resistance to the aminoglycoside G-418 (Colberre-Garapin, et al., J. Mol. Biol., 150:1, 1981); and hygro, which confers resistance to hygromycin (Santerre, et al., Gene, 30: 147, 1984). Recently, additional selectable genes have been described, namely trpB, which allows cells to utilize indole in place of tryptophan; hisD, which allows cells to utilize histinol in place of histidine (Hartman & Mulligan, Proc. Natl. Acad. Sci. USA, 85:8047, 1988); and ODC (ornithine decarboxylase) which confers resistance to the ornithine decarboxylase inhibitor, 2-(difluoromethyl)-DL-ornithine, DFMO (McConlogue L., In: Current Communications in Molecular Biology, Cold Spring Harbor Laboratory, ed., 1987).

DNA sequences encoding the fluorescence indicator polypeptide of the invention can be expressed *in vitro* by DNA transfer into a suitable host cell. "Host cells" are cells in which a vector can be propagated and its DNA expressed. The term also includes any progeny of the subject host cell. It is understood that all progeny may not be identical to the parental cell since there may be mutations that occur during replication. However, such progeny are included when the term "host cell" is used.

Methods of stable transfer, in other words when the foreign DNA is continuously maintained in the host, are known in the art.

Recombinant fluorescent protein can be produced by expression of nucleic acid encoding the protein in prokaryotes, such as *E. coli* or in eukaryotes, such as yeast cells or mammalian cells. The fluorophore of *Aequorea*-related fluorescent proteins results from cyclization and oxidation of residues 65-67.

The construct can also contain a tag to simplify isolation of the fluorescent indicator. For example, a polyhistidine tag of, *e.g.*, six histidine residues, can be incorporated at the amino terminal end of the fluorescent protein. The polyhistidine tag allows convenient isolation of the protein in a single step by nickel-chelate chromatography.

In a preferred embodiment, the fluorescent indicator is a fusion protein produced by recombinant DNA technology in which a single polypeptide includes a donor moiety, a peptide linker moiety and an acceptor moiety. The donor moiety can be positioned at the amino-terminus relative to the acceptor moiety in the polypeptide. Such a fusion protein has the generalized structure: (amino terminus) donor fluorescent protein moiety --peptide linker moiety --acceptor fluorescent protein moiety (carboxy terminus). Alternatively, the donor moiety can be positioned at the carboxy-terminus relative to the acceptor moiety within the fusion protein. Such a fusion protein has the generalized structure: (amino terminus) acceptor fluorescent protein moiety --peptide linker moiety --donor fluorescent protein moiety (carboxy terminus). The invention also envisions fusion proteins that contain extra amino acid sequences at the amino and/or carboxy termini, for example, polyhistidine tags.

Thus, recombinant nucleic acids encoding the fluorescent indicator include sequences coding for expression of a donor fluorescent protein moiety, an acceptor fluorescent protein moiety and a peptide linker moiety. The elements are selected so that upon expression into a fusion protein, the donor and acceptor moieties exhibit FRET when the donor moiety is excited. The recombinant nucleic acid can be incorporated into an expression vector comprising expression control sequences operatively linked to the recombinant nucleic acid. The expression vector can be adapted for function in prokaryotes or eukaryotes by inclusion of appropriate promoters, replication sequences, markers, etc.

The expression vector can be transfected into a host cell for expression of the recombinant nucleic acid. Host cells can be selected for high levels of expression in order to purify the fluorescent indicator fusion protein. *E. coli* is useful for this purpose.

Alternatively, the host cell can be a prokaryotic or eukaryotic cell selected to study the activity of an enzyme produced by the cell. In this case, the linker peptide is selected to include an amino acid sequence recognized by the protease. The cell can be, *e.g.*, a cultured cell or a cell *in vivo*.

A primary advantage of fluorescent indicator fusion proteins is that they are prepared by normal protein biosynthesis, thus completely avoiding organic synthesis and the requirement for customized unnatural amino acid analogs. The constructs can be expressed in *E. coli* in large scale for *in vitro* assays. Purification from bacteria is simplified when the sequences include polyhistidine tags for one-step purification by nickel-chelate chromatography. Alternatively, the substrates can be expressed directly in a desired host cell for assays *in situ*.

A nucleic acid sequence which encodes the fluorescent indicator may be expressed in a transgenic non-human animal.

The "non-human animals" comprise any non-human animal having nucleic acid sequence which encodes a fluorescent indicator. Such non-human animals include vertebrates such as rodents, non-human primates, sheep, dog, cow, pig, amphibians, and reptiles. Preferred non-human animals are selected from the rodent family including rat and mouse, most preferably mouse. The "transgenic non-human animals" are produced by introducing "transgenes" into the germline of the non-human animal. Embryonal target cells at various developmental stages can be used to introduce transgenes. Different methods are used depending on the stage of development of the embryonal target cell. The zygote is the best target for micro-injection. In the mouse, the male pronucleus reaches the size of approximately 20 micrometers in diameter which allows reproducible injection of 1-2 pl of DNA solution. The use of zygotes as a target for gene transfer has a major advantage in that in most cases the injected DNA will be incorporated into the host gene before the first cleavage (Brinster et al., Proc. Natl. Acad. Sci. USA 82:4438-4442, 1985). As a consequence, all cells of the transgenic non-human animal will carry the incorporated transgene. This will in general also be reflected in the efficient transmission of the transgene to offspring of the founder since 50% of the germ cells will harbor the transgene. Microinjection of zygotes is the preferred method for incorporating transgenes.

The term "transgenic" is used to describe a non-human animal which includes exogenous genetic material within all of its cells. A "transgenic" non-human animal can be produced by cross-breeding two chimeric non-human animals which include exogenous genetic material within cells used in reproduction. Twenty-five percent of the resulting offspring will be transgenic *i.e.,* non-human animals which include the exogenous genetic material within all of their cells in both alleles. 50% of the resulting non-human animals will include the exogenous genetic material within one allele and 25% will include no exogenous genetic material.

Retroviral infection can also be used to introduce transgene into a non-human animal. The developing non-human embryo can be cultured *in vitro* to the blastocyst stage. During this time, the blastomeres can be targets for retro viral infection (Jaenich, R., Proc. Natl. Acad Sci USA 73:1260-1264, 1976). Efficient infection of the blastomeres is obtained by enzymatic treatment to remove the zona pellucida (Hogan, et al. (1986) in Manipulating the Mouse Embryo, Cold Spring Harbor Laboratory Press, Cold Spring Harbor, N.Y.). The viral vector system used to introduce the transgene is typically a replication-defective retro virus carrying the transgene (Jahner, et al., Proc. Natl. Acad Sci. USA 82: 6927-6931, 1985; Van der Putten, et al., Proc. Natl. Acad. Sci USA 82:6148-6152, 1985). Transfection is easily and efficiently obtained by culturing the blastomeres on a monolayer of virus-producing cells (Van der Putten, *supra;* Stewart, et al , EMBO J. 6:383-388,1987). Alternatively, infection can be performed at a later stage. Virus or virus-producing cells can be injected into the blastocoele (D. Jahner et al., Nature 298:623-628, 1982). Most of the founders will be mosaic for the transgene since incorporation occurs only in a subset of the cells which formed the transgenic nonhuman animal. Further, the founder may contain various retro viral insertions of the transgene at different positions in the genome which generally will segregate in the offspring. In addition, it is also possible to introduce transgenes into the germ line, albeit with low efficiency, by intrauterine retro viral infection of the midgestation embryo (D. Jahner *et al., supra).*

A third type of target cell for transgene introduction is the non-human embryonal stem cell (ES). Non-human ES cells are obtained from pre-implantation non-human embryos cultured *in vitro* and fused with non-human embryos (M. J. Evans et al. Nature 292:154-156, 1981; M.O. Bradley et al., Nature 309: 255-258, 1984; Gossler, et al., Proc. Natl. Acad. Sci USA 83: 9065-9069, 1986; and Robertson et al., Nature 322:445-448, 1986): Transgenes can be efficiently introduced into the non-human ES cells by DNA transfection or by retro virus-mediated transduction. Such transformed non-human ES cells can thereafter be combined with blastocysts from a nonhuman animal. The non-human ES cells thereafter colonize the non-human embryo and contribute to the germ line of the resulting chimeric non-human animal. (For review see Jaenisch, R., Science 240: 1468-1474, 1988).

"Transformed" means a cell into which (or into an ancestor of which) has been introduced, by means of recombinant nucleic acid techniques, a heterologous nucleic acid molecule. "Heterologous" refers to a nucleic acid sequence that either originates from another species or is modified from either its original form or the form primarily expressed in the cell.

"Transgene" means any piece of DNA which is inserted by artifice into a cell, and becomes part of the genome of the non-human organism (*i.e.*, either stably integrated or as a stable extrachromosomal element) which develops from that cell. Such a transgene may include a gene which is partly or entirely heterologous (*i.e.,* foreign) to the transgenic non-human organism, or may represent a gene homologous to an endogenous gene of the non-human organism. Included within this definition is a transgene created by the providing of an RNA sequence which is transcribed into DNA and then incorporated into the genome. The transgenes of the invention include DNA sequences which encode the fluorescent indicator which may be expressed in a transgenic non-human animal. The term "transgenic" as used herein additionally includes any non-human organism whose genome has been altered by *in vitro* manipulation of the early non-human embryo or non-human fertilized egg or by any transgenic technology to induce a specific gene knockout. The term "gene knockout" as used herein, refers to the targeted disruption of a gene *in vivo* with complete loss of function that has been achieved by any transgenic technology familiar to those in the art. In one embodiment, transgenic non-human animals having gene knockouts are those in which the target gene has been rendered nonfunctional by an insertion targeted to the gene to be rendered non-functional by homologous recombination. As used herein, the term "transgenic" includes any transgenic technology familiar to those in the art which can produce a non-human organism carrying an introduced transgene or one in which an endogenous gene has been rendered non-functional or "knocked out."

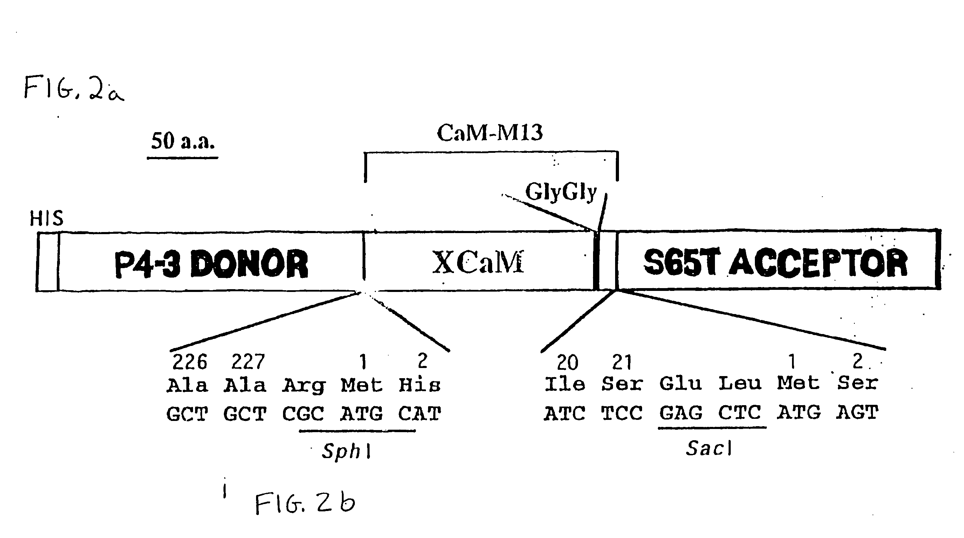

Without further elaboration, it is believed that one skilled in the art can, based on the description herein, utilize the present invention to its fullest extent. A fluorescent indicator for $Ca^{2+}$ was produced by sandwiching CaM-M 13 fusion, described in Porumb, T., et al., Prot.Engineering 7:109-115 (1994); between a blue (P4-3) and a green (S65T) GFP mutant, as illustrated in FIG. 2a. The chimeric cDNA was cloned at *Bam*HI/*Eco*RI sites of pRSETB (Invitrogen), HIS is the amino-terminal tag peptide containing the polyhistidine sequence and XCaM is Xenopus calmodulin. Chimeric proteins incorporating a polyhistidine tag were expressed in *Escherichia coli,* purified by nickel-chelate and size-exclusion chromatography, and their fluorescence characterized. Referring to FIG. 2a, the fluorescent CaM-based indicator ("cameleon-1") readily changes emission color by retracting and extending a long tongue (M13) into and out of the mouth of the CaM.

The amino acid composition of the boundary regions between the CaM-M13 hybrid and GFPs can be important to optimize protein folding and the $Ca^{2+}$-sensitivity of FRET. One particularly sensitive indicator is shown in FIG. 2b. Referring to FIG. 2b, the amino acid and nucleotide sequences of the boundaries between P4-3 and CaM and between M13 and S65T in cameleon-1 are shown. Cameleon-1 has an 11 amino acid deletion at the C-terminus of P4-3 and a 5 amino acid deletion at the C-terminus of M13. Two restriction sites *Sph*I and *Sac*I are underlined, which were utilized to connect the genes of P4-3 and S65T to the CaM-M13 gene, respectively. To facilitate the folding of the GFP that is fused to any other protein, a few glycine residues are usually inserted into the boundary. See, for example, Porumb, T., et al , Prot. Engineering 7:109-115 (1994). Further, glycine residues were not introduced so that P4-3 and S65T were fused rigidly to the CaM and M13, respectively. The rigid fusion leads to more effective transduction of the conformational change of CaM-M13, causing a greater change in FRET efficiency.

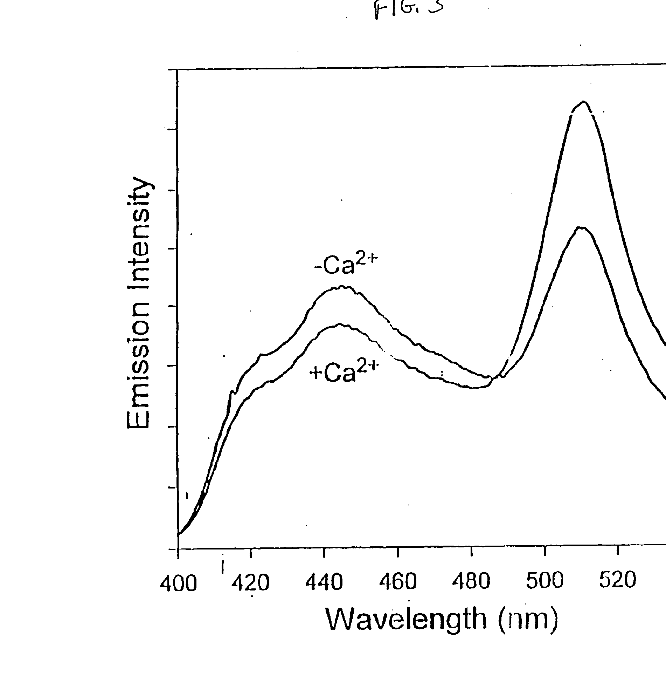

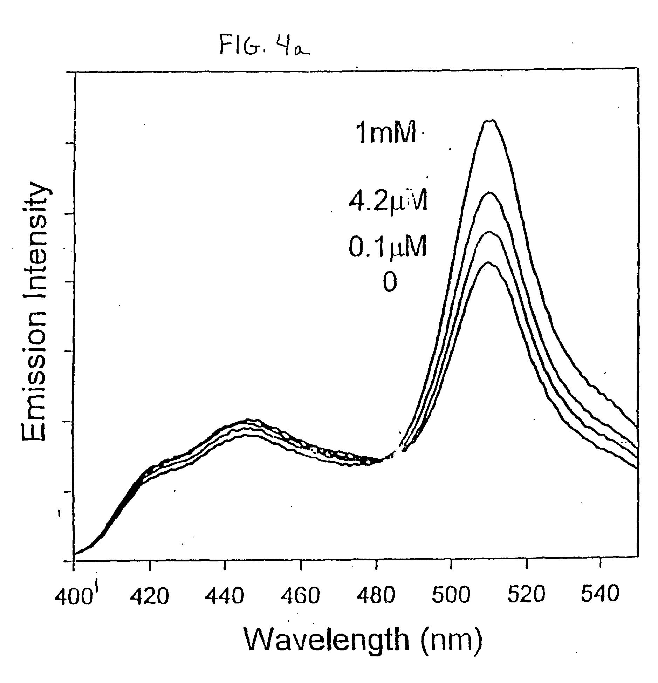

The fluorescent indicator was efficiently expressed and folded in bacteria and increased its ratio of UV-excited 510 nm to 445 nm emissions by 70% upon binding $Ca^{2+}$, as shown in FIG. 3. The emission spectra of cameleon-1 were measured in 100 mM KCl, 20 mM MOPS, 1 mM EDTA, KOH to pH 7.20, before and after addition of 2 mM $CaCl_2$ to give 1 mM free $Ca^{2+}$. The $Ca^{2+}$ binding to EDTA caused a local acidification of the solution, and a small fraction of the protein was denatured. Thus the spectrum after the $Ca^{2+}$ addition dropped down slightly (compare with FIG. 4a). The decrease in blue and increase in green emission indicated that $Ca^{2+}$ increased the efficiency of FRET from P4-3 to S65T, consistent with the expected decrease in distance between the two ends of the protein. The $Ca^{2+}$ response was fully reversible upon chelation of $Ca^{2+}$.

The $Ca^{2+}$-specificity of the response of cameleon-1 was examined. $Mg^{2+}$, pH, and ionic strength did not alter the emission spectra of either the $Ca^{2+}$-saturated and $Ca^{2+}$-unsaturated forms. The emission spectra of saturated and unsaturated cameleon-1 were also not affected by hydrophobic proteins such as bovine serum albumin. Isolated CaM saturated with $Ca^{2+}$ typically becomes sticky with hydrophobic amino acids exposed to the surface. The CaM in cameleon-1, on the other hand, appears to interact preferentially with its intramolecularly-adjacent M 13 peptide. The self-contained nature of the system minimizes the possibility that the protein might interact with endogenous CaM-binding sequences in eukaryotic cells.

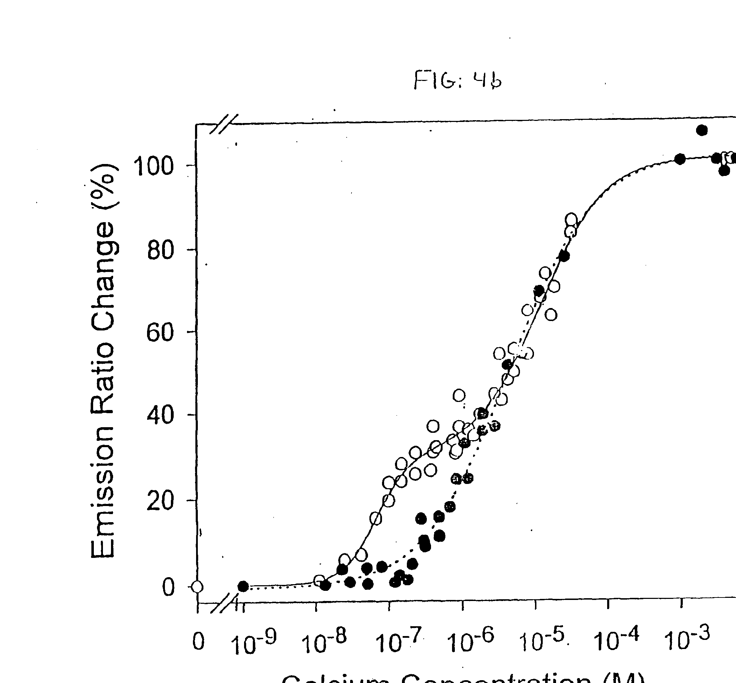

The $Ca^{2+}$ binding behavior of cameleon-1 was examined. The CaM-M13 hybrid protein without GFPs displayed a biphasic $Ca^{2+}$ binding with two dissociation constants (80 nM and 2 TM). See, Porumb, T., et al., Prot. Engineering 7:109-115 (1994). Titration experiments revealed that the emission ratio of cameleon-1 has a biphasic $Ca^{2+}$ dependency, as shown in FIGS. 4a and 4b. FIG. 4a shows the change in emission spectrum of cameleon-1 on titration with $Ca^{2+}$ when excited at 380 nm. The titration was done using KHHEDTA/KCaHEDTA solutions at pH 7.47. For clarity only two intermediate concentrations of $Ca^{2+}$ are shown.

FIG. 4b shows $Ca^{2+}$ titration curves of cameleon-1 (open circles) and cameleon-1/E104Q (solid circles). Data points were from 4 independent experiments at different pH using $Ca^{2+}$/EGTA and $Ca^{2+}$/HEEDTA systems for each protein. In each experiment, the emission ratio (510/445 nm) change was normalized to the value of full saturation with $Ca^{2+}$, which increased by 60-75% over the value of zero $Ca^{2+}$. The data of cameleon-1/E104Q were fitted to a four parameter logistic function curve (dotted line). The data of cameleon-1 were analysed using a linear combination of 2 four parameter logistic function fits (solid line). The apparent dissociation constants ($K'_d$s) for cameleon-1 were 68 nM and 11 TM, and the Hill coefficients were 1.8 and 1.0, respectively. The binding curve can be used to quantify the concentration of $Ca^{2+}$ present in the sample. Because of simulated negative cooperativity, cameleon-1 covers a very wide range of $Ca^{2+}$ concentration, from $< 10^{-7}$ to $-10^{-3}$ M .

The affinity of the CaM binding protein moiety can be modified. Many site-directed mutations have been studied for their effects on the $Ca^{2+}$ binding and $Ca^{2+}$-induced conformational changes of CaM. See, Maune, J.F., et al., J.Biol.Chem. 267:5286-5295 (1992), and Gao, Z.H., et al., J.Biol.Chem. 268:20096-20104 (1993). For example, a mutant chimera protein with the conserved bidentate glutamic acid at position 12 of the third $Ca^{2+}$ binding loop of the CaM mutated to glutamine (cameleon-1/E104Q) was constructed. The mutation eliminated the high-affinity response ofcameleon-1, as indicated in FIG. 4b, (solid circles). Cameleon-1/E104Q showed a monophasic response (K'd, 4.4 TM; Hill coefficient, 0.76), which corresponds closely to the low affinity component of the cameleon-1 FRET response. Other modifications of CaM can be made to tune the $Ca^{2+}$ affinities for particular applications of the fluorescent indicator.

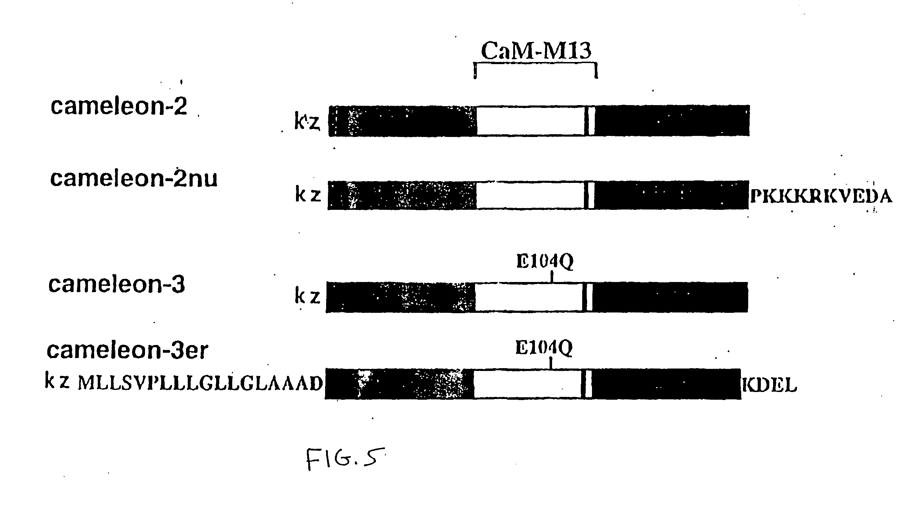

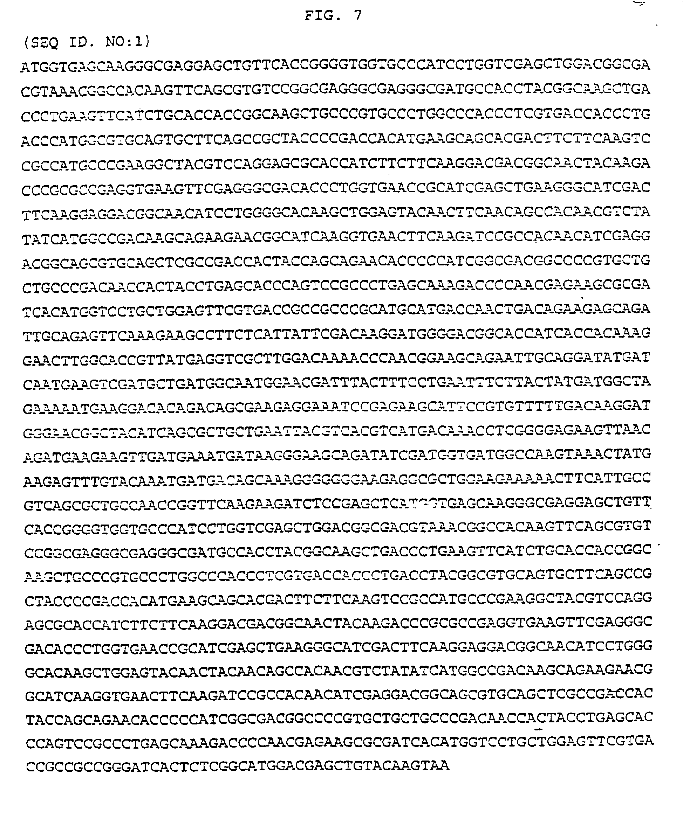

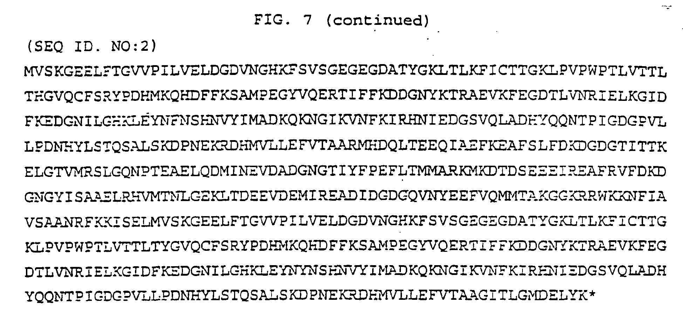

HeLa cells were transfected with the recombinant plasmid (cameleon-1/pCDNA3) to determine whether cameleon-1 can work as a $Ca^{2+}$ indicator in live cells. When the cells were excited with UV, however, the fluorescence of cameleon-1 was hardly detectable, mainly because of the dim fluorescence of the P4-3 component. Expression and folding of GFP at 37EC in mammalian cells was improved by introducing mammalian codon bias into the cDNA and mutating Phe64 to Leu, as in the commercially available construct "EGFP" (Clonetech), which encodes F64L/S65T with mammalian codons. The same changes were introduced into P4-3 (Y66H/Y145F), which did not change its final fluorescence properties but did improve expression in the HeLa cells. The improved blue mutant ("EBFP") and EGFP substituted P4-3 and S65T, respectively, in cameleon-1 to make cameleon-2, shown in FIG. 5, where Kz is Kozak's consensus sequence (M. Kozak, J. Cell Biol. 108:229-241 (1989)). The nucleotide sequence (SEQ ID NO:1) and amino acid sequence (SEQ ID NO:2) of cameleon-2 are shown in FIG. 7.

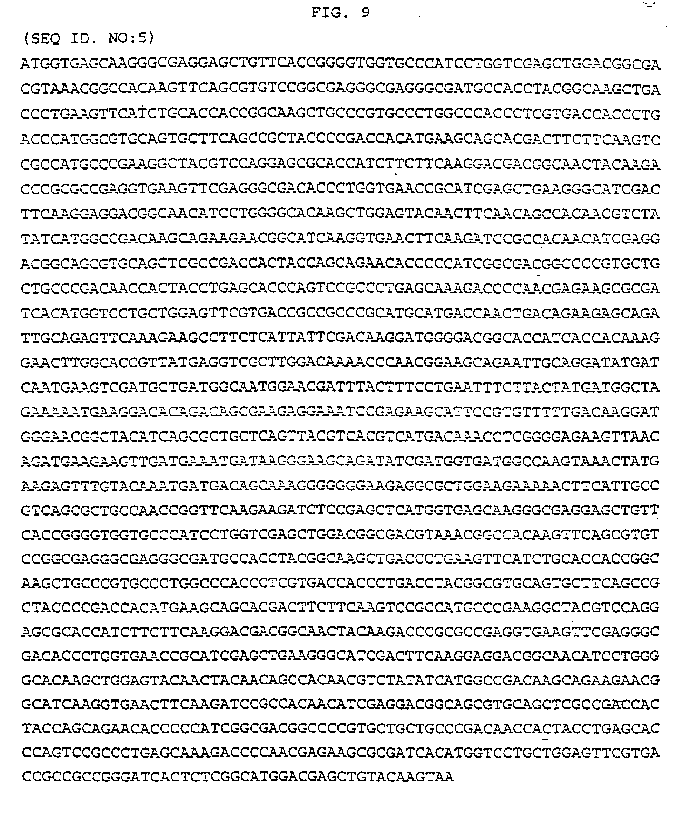

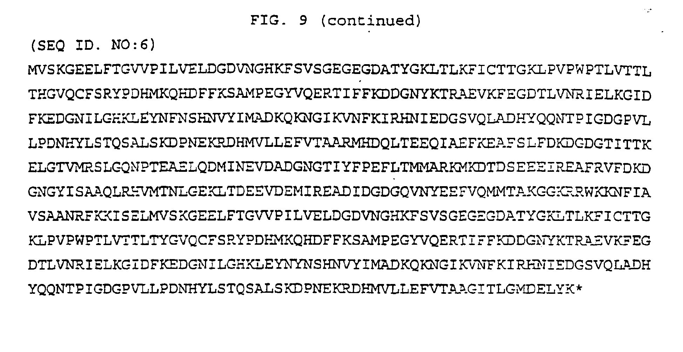

The E104Q mutation which afforded low $Ca^{2+}$ affinity in cameleon-1 was also introduced into cameleon-2; the resulting chimera protein (cameleon-2/E104Q) cameleon-3, also shown in FIG. 5. The nucleotide sequence (SEQ ID NO:5) and amino acid sequence (SEQ ID NO:6) of cameleon-3 are shown in FIG. 9.

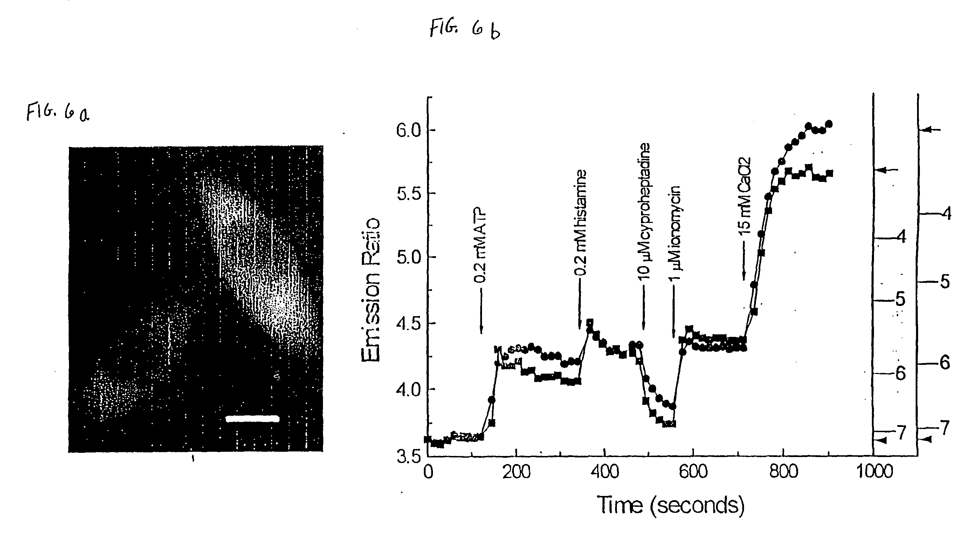

Significant emission signals were observed from both the EBFP and EGFP in the cells expressing cameleon-2 or cameleon-3. Referring to FIG. 6a, fluorescence of cameleon-2 localized to the cytosol of HeLa cells. The fluorescence was uniformly distributed in the cytosolic compartment but excluded from the nucleus, as expected for a protein of 74 kDa without localization sequences or targeting signals. The image was taken using a 330WB80 (excitation filter) and a 535DF45 (emission filter). The bar is 10 Tm.

FIG. 6b shows time courses of the spatially averaged green:blue emission ratios from two individual HeLa cells expressing cameleon-2. The two cells shown in FIG. 6a were excited by UV (330WB80) and monitored every 15 seconds by digital imaging microscopy. See, for example, Tsien, R.Y. & Harootunian, A.T. Cell Calcium, 11:93-109 (1990). The emission bands (440DF40 and 535DF45) over the cytoplasmic regions were alternately sampled. *In situ* calibration was performed for each of the cells. The pre-stimulation ratio (arrowhead) was assumed to be 50 nM, and $R_{max}$ (arrow) the value after saturation with $Ca^{2+}$. The calculated values for pCas 7, 6, 5, 4 are indicated by horizontal bars on the right side of the panel.

Elevation of cytosolic $Ca^{2+}$ concentration by saturating doses of ATP (as a purinergic agonist) and histamine produced significant increases in the emission ratio. Blockage of the histamine receptor by the antagonist cyproheptadine caused a rapid decrease in ratio, indicating the reversible behavior of the indicator. Addition of ionomycin followed by a high concentration (15 mM) of extracellular $Ca^{2+}$ gave a large increase of the ratio (70-80% increase of the initial ratio value), which should correspond to the maximal ratio $R_{max}$. Assuming the lowest ratio observed before stimulation represents the $R_{min}$, calibration for free $Ca^{2+}$ concentration can be performed. See, Adams, S.R., et al., in Fluorescent and Luminescent Probes for Biological Activity (ed. Mason, W.T.) (Academic Press, 1993).

By contrast, cameleon-3, which lacks the high affinity component of $Ca^{2+}$ binding to CaM, did not detect the changes in cytosolic $Ca^{2+}$ concentration signals due to ATP or histamine, but gave a similar $R_{max}$ in response to ionomycin and 20 mM extracellular $Ca^{2+}$. Cameleon-3 is less sensitive to and buffers cytosolic $Ca^{2+}$ to a lesser extent than does cameleon-2. The probable high $Ca^{2+}$ dissociation rate of cameleon-3 is advantageous for tracking rapid $Ca^{2+}$ release kinetics. The *in vitro* study revealed that the chameleon indicators show a relatively fast cellular response to $Ca^{2+}$ concentration changes.

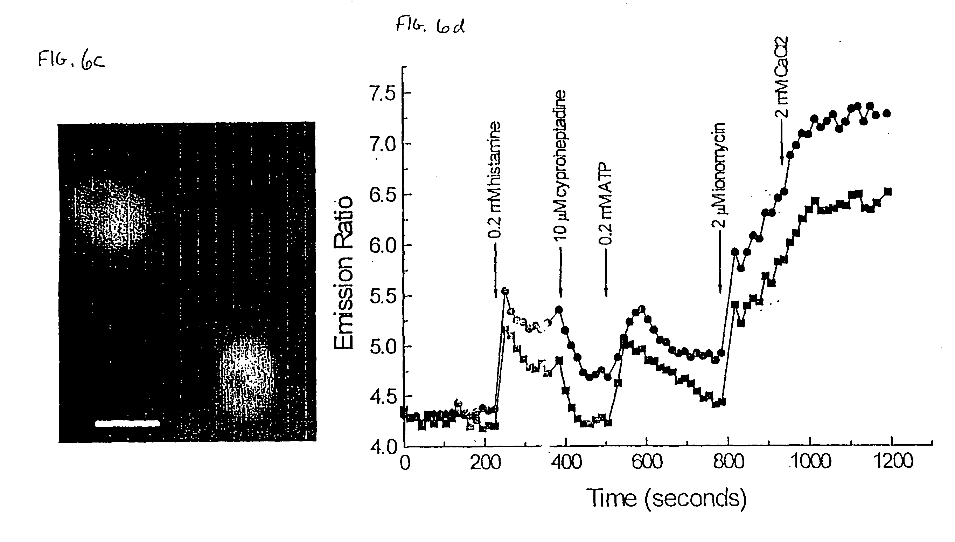

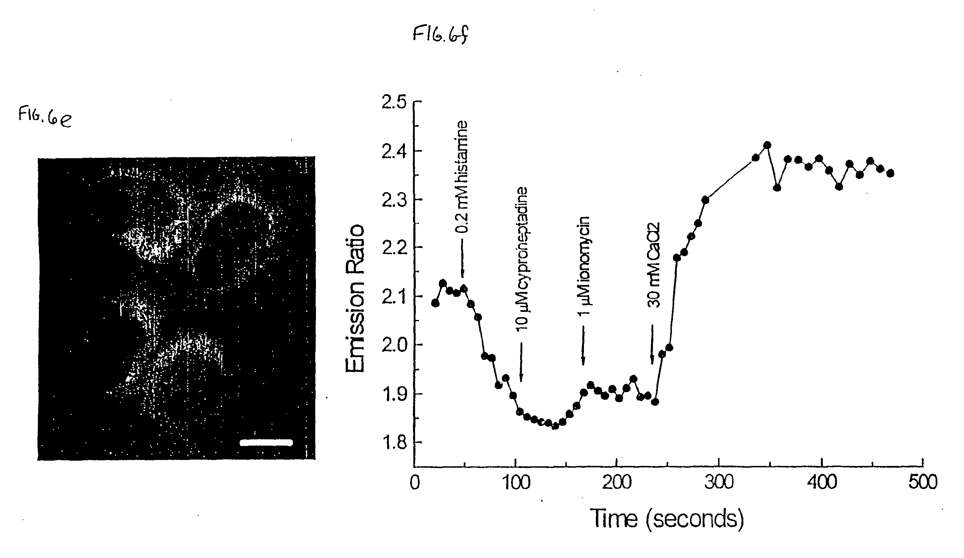

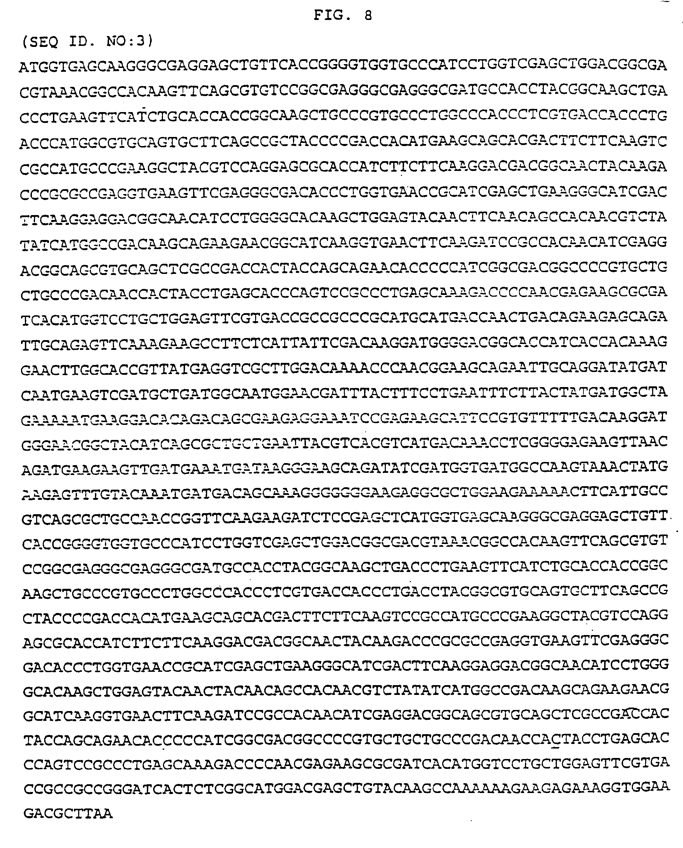

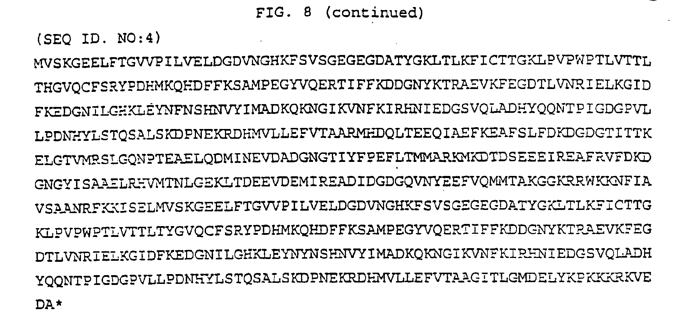

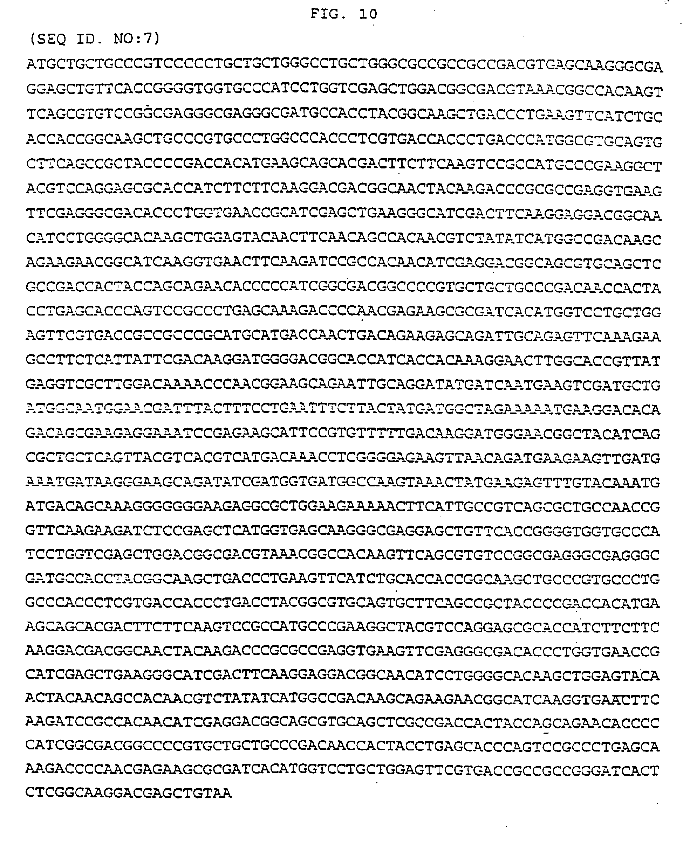

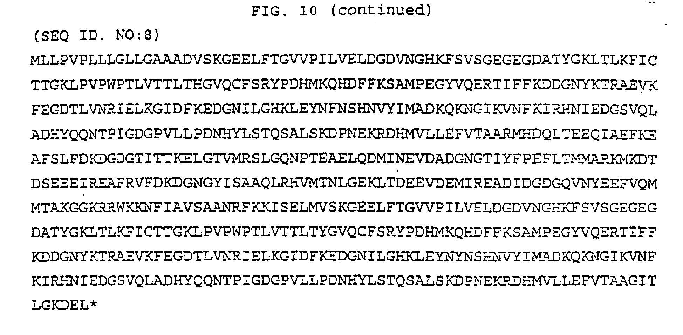

Addition of a nuclear localization sequence to cameleon-2 yielded a $Ca^{2+}$ indicator, cameleon-2nu, shown in FIG. 5. The nucleotide sequence (SEQ ID NO:3) and amino acid sequence (SEQ ID NO:4) of cameleon-2nu are shown in FIG. 8. The fluorescence of cameleon-2nu was localized to nuclei, as depicted in FIG. 6c. The time course of $Ca^{2+}$ concentrations in nuclei was followed (FIG. 6d) and was similar to the results obtained in the cytosol (FIG. 6b). Agonist-induced changes in the free $Ca^{2+}$ concentration inside the endoplasmic reticulum in intact cells were similarly monitored. The low-affinity indicator cameleon-3 was engineered to reside in the lumen of endoplasmic reticulum (ER) (cameleon-3er) (FIG. 5) by addition of a localization sequence at the amino terminus and a KDEL signal for ER retention at the carboxy terminus of the fluorescent indicator. The nucleotide sequence (SEQ ID NO:7) and amino acid sequence of cameleon-3er (SEQ ID NO:8) are shown in FIG. 10. Reticular patterns of fluorescence were seen in HeLa cells expressing the protein. FIG. 6e is a digital fluorescence image of cameleon-3er in transfected HeLa cells. The image was obtained with a cooled CCD camera system with a 480DF30 (excitation filter) and a 535DF45 (emission filter). The bar is 10 Tm.

FIG. 6f shows a time course of the average $Ca^{2+}_{cr}$ concentration of four cells obtained with a video-rate confocal microscope. Digital fluorescence images were the result of simultaneous acquisition of two confocal single-wavelength emission images at 450 nm (65 nm bandpass) and 535 nm (45 nm bandpass). After background subtraction, the ratio of the long wavelength image over the short wavelength one was calculated. Cells were illuminated for 66 msec (2 frames) for each time point. The interference filters and dichroics, and the sensitivity of the detectors are different between the CCD and confocal microscope systems. Therefore, the ratios obtained in the two systems differ quantitatively.

The pre-stimulus $Ca^{2+}_{cr}$ concentration reported by cameleon-3er was consistently higher than cytosolic or nuclear $Ca^{2+}$ concentrations reported by cameleon-2 and cameleon-2nu, respectively. Histamine reproducibly decreased the $Ca^{2+}_{cr}$ concentration in all of 15 cells, whereas it always increased the cytosolic and nuclear $Ca^{2+}$ concentrations.

Receptor blockade by cyproheptadine reversed the decrease $Ca^{2+}_{cr}$ concentration, indicating refill of the $Ca^{2+}$ pools. In FIG. 6f, the ratio did not reach the value of the resting state, whereas complete reversion was observed in five other experiments. Addition of ionomycin and 20 mM extracellular $Ca^{2+}$ increased $Ca^{2+}_{cr}$ concentration to a saturating value above the starting level. Changes in $Ca^{2+}_{cr}$ concentration were generally slower than those of the cytosol and nuclei.

The targetability of the fluorescent indicators can permit $Ca^{2+}$ measurements at previously inaccessible sites such as the immediate vicinity of synaptic vesicles or $Ca^{2+}$ channels, and in genetically tractable organisms that do not load well with esters of small-molecule indicators.

The Examples described below are illustrative of the disclosed method; however, many alternatives, modifications and variations will be clear to those skilled in the art.

## Examples

### Gene construction

The cDNA of the GFP mutant P4-3 was amplified by PCR with a sense primer containing a *Bam*HI site, and a reverse primer containing an *Sph*I site and eliminating the GFP stop codon. See, for example, Heim, R. & Tsien, R.Y. Current Biol. 6:178-182 (1996). Likewise, the cDNA of S65T was amplified with a *Sac*I site and an *Eco*RI site introduced to the 5' and 3' ends of the gene, respectively. Two restriction sites (*Sph*I and *Sac*I) were introduced by PCR into 5' and 3' ends of the CaM-M13 gene, respectively, using the pHY1 as a template. See, Porumb, T., et al. Prot. Engineering 7: 109-1:15 (1994). All the amplification reactions were done by Pfu polymerase (Stratagene). The restricted products were ligated and cloned in-frame into the *Bam*HI/*Eco*RI sites of pRSETB (Invitrogen). The modifications of the boundary regions between P4-3 and CaM and between M 13 and S65T were performed by PCR or by a combined use of restriction enzymes, Klenow fragment of DNA polymerase I, T4 DNA polymerase, mung bean exonuclease, and T4 DNA ligase as described, for example, in Molecular Cloning, A Laboratory Manual (eds. Sambrook, J., Fritsch, E.F. & Maniatis, T.) (CSH Laboratory Press, 1989). The phEGFP plasmid was commercially available from Clontech. Two amino acid substitutions (Y66H and Y145F) were made in hEGFP to construct EBFP. Oligonucleotide-directed mutageneses were carried out using the Muta-Gene Phagemid *in vitro* kit (Bio-Rad) at the codons for Y66H and Y145F of EGFP, and for E104Q of the calmodulin. The 5' end of the EBFP gene was modified by PCR to have a *Hind*III restriction site followed by a Kozak's consensus sequence (ACCGCC-ATG). The *Hind*III/*Eco*RI fragment encoding the entire chimeric protein was subcloned in the mammalian expression vector pCDNA3 (Invitrogen).

For cameleon-2nu, the cameleon-2 DNA was extended by PCR at the 3' end with the sequence encoding the nuclear localization sequence (PKKKRKVEDP). See, Forbes, D.J. Ann.Rev.Cell Biol. 8:495-527 (1992). Cameleon-3er DNA was likewise obtained by extending the cameleon-3 DNA at the 5' end with the sequence encoding the localization sequence peptide from calreticulin (MLLPVPLLLGLLGLAAAD), and at the 3' end with the sequence encoding the ER retention signal (KDEL). See, Kendall, J.M. et al., Biochem. Biophys. Res. Commun. 189:1008-1016 (1992).

### Protein expression and spectroscopy

The expression of chimera proteins in bacteria was performed using the T7 expression system (pRSETB/JM109 (DE3)). Cultures were grown at room temperature, and protein expression was induced by isopropyl B-D-thiogalactoside. Cells were lysed by a French press. The polyhistidine-tagged chimera proteins were purified from the cleared lysates on nickel-chelate columns (Qiagen). The protein samples in the cluates were concentrated by Centricon 30 (Amicon), and were further purified by gel-filtration column to remove abortive chimera proteins which resulted from proteolysis or misfolding. Emission spectra of the purified proteins were measured using a fluorometer (Spex Industries, Edison, NJ) at excitation 380 nm.

### $Ca^{2+}$ titration and calibration

The titration experiments were performed by the "pH-metric method" as described in Grzegorz, G., et al., J. Biol. Chem. 260:3440-3450 (1985). *In situ* calibration for cytosolic $Ca^{2+}$ concentration utilized the equation:

$$[Ca^{2+}]c = K'_d((R-R_{min})/(R_{max}-R))^{(1/nH)}$$

where $K'_d$ is the apparent dissociation constant corresponding to the $Ca^{2+}$ concentration at which R is midway between $R_{max}$ and $R_{min}$, and nH is the Hill coefficient.

**Imaging**

Two to five days after the cDNA transfection with lipofectin (Gibco BRL), the cells were imaged on a Zeiss Axiovert microscope with a cooled CCD camera (Photometrics, Tucson, AZ) interfaced to a personal computer. The program MetaFluor 2.75 (Universal Imaging) was used for controlling data acquisition and analysis. Dual-emission ratio imaging was carried out by manually switching the two emission filters (440DF40 for EBFP, 535DF45 for EGFP) in front of a single imaging camera. The excitation filter (330WB80) was used with a 420DRLP dichroic mirror. Digital fluorescence imaging with a video-rate confocal microscope was performed as described in Tsien, R.Y. & Backskai, B.J. Handbook of Biological Confocal Microscopy (ed. Pawley, J.B.) (Plenum Press, New York, 1995) p. 459-478. Cells were illuminated with wide band UV (351-364 nm) from an Ar$^+$ ion laser. The primary dichroic (380DRLP) reflects UV and transmits light emitted from the specimen, which is subsequently split by a secondary dichroic (505DRLP) into two broad bands: EBFP emission (450DF65) and EGFP emission (535DF45), and counted by photomultiplier tubes.

**Claims**

1. A fluorescent indicator fusion protein for detecting an analyte which is a ligand of a binding protein, wherein the fusion protein comprises:

   a binding protein moiety having the ligand binding region of the binding protein and which undergoes a conformational change upon the binding of the ligand;
   a donor fluorescent protein moiety wherein the donor fluorescent protein moiety is fused to the binding protein moiety;
   an acceptor fluorescent protein moiety wherein the acceptor fluorescent protein moiety is fused to the binding protein moiety; and
   wherein the donor fluorescent protein moiety has an emission spectrum which overlaps with the absorption spectrum of the acceptor fluorescent protein moiety and the excitation spectrum of the donor fluorescent moiety comprises a wavelength of excitation at which the donor fluorescent protein moiety can be excited effectively and selectively with respect to the acceptor fluorescent protein moiety; and the emission spectrum of the donor fluorescent protein moiety and the emission spectrum of the acceptor fluorescent protein moiety allow the two emissions to be distinguished, and fluorescence resonance energy transfer (FRET) between the donor fluorescent protein moiety and the acceptor fluorescent protein moiety can be detected when the donor fluorescent protein moiety is excited at the wavelength; and

   wherein the acceptor fluorescent protein moiety and the donor fluorescent protein moiety change position relative to each other when the ligand binds to the ligand-binding region, altering the FRET.

2. The fusion protein of claim 1, wherein the donor fluorescent protein moiety and the acceptor fluorescent protein moiety are Aequorea-related fluorescent protein moieties.

3. The fusion protein of claim 1, wherein the binding protein moiety additionally has a domain capable of binding a target peptide moiety and the target peptide moiety is fused to the binding protein moiety by a polypeptide linker and the binding of the ligand to the ligand binding region of the binding protein moiety alters the binding of the target peptide moiety to the target peptide binding domain of the binding protein moiety to alter the FRET.

4. The fusion protein of claim 1, wherein one of the donor fluorescent protein moiety or the acceptor protein moiety is fused at the carboxy terminus of the fusion protein and the other one of the donor fluorescent protein moiety or the acceptor protein moiety is fused at the amino terminus of the fusion protein.

5. The fusion protein of claim 1, wherein the binding protein moiety is calmodulin, a calmodulin-related protein moiety, cGMP-dependent protein kinase, a steroid hormone receptor, a ligand binding domain of a steroid hormone receptor, protein kinase C, inositol-1,4,5-triphosphate receptor, or recoverin.

**6.** The fusion protein of claim 3, wherein the binding protein moiety is calmodulin or a calmodulin-related protein moiety.

**7.** The fusion protein of claim 1, wherein the donor fluorescent protein moiety is a green fluorescent protein mutant selected from P4-3, EBFP, or W1B, and the acceptor fluorescent protein moiety is a green fluorescent protein mutant selected from S65T, EGFP, or 10c.

**8.** The fusion protein of claim 3, wherein the target peptide moiety is a calmodulin-binding domain of skMLCKp, smMLCK, CaMKII, Caldesmon, Calspermin, phosphofructokinase, calcineurin, phosphorylase kinase, $Ca^{2+}$-AT-Pase, 59 kDa PDE, 60 kDa PDE, nitric oxide synthase, type I adenylyl cyclase, *Bordetella pertussis* adenylyl cyclase, Neuromodulin, Spectrin, MARCKS, F52, β-Adducin, HSP90a, HIV-1 gp160, BBMHBI, Dilute MHC, Mastoparan, Melittin, Glucagon, Secretin, VIP, GIP, or Model Peptide CBP2.

**9.** The fusion protein of claim 3, wherein the target peptide moiety is M13.

**10.** The fusion protein of claim 9, wherein the polypeptide linker is -Gly-Gly-.

**11.** The fusion protein of claim 1, wherein the fusion protein further comprises a localization moiety.

**12.** The fusion protein of claim 11, wherein the localization moiety is a localization moiety for the nucleus, the endoplasmic reticulum, a peroxisome, the Golgi apparatus, the plasma membrane or the mitochondria.

**13.** The fusion protein of claim 1, wherein the binding protein moiety comprises a conformation of smaller volume when the ligand is bound to the ligand binding region of the binding protein moiety compared to the volume of the binding protein moiety when the ligand binding region is not bound to the ligand, whereby the FRET is increased by the binding of the ligand to the ligand binding region of the binding protein moiety.

**14.** The fusion protein of claim 3, wherein the binding protein moiety has increased affinity for the target peptide moiety when the ligand binding region of the binding protein moiety is bound by the ligand.

**15.** The fusion protein of claim 3, wherein the polypeptide linker is from one to 30 amino acids in length.

**16.** The fusion protein of claim 1, wherein the donor fluorescent protein moiety is capable of being excited by ultraviolet light to emit blue light and the acceptor fluorescent protein moiety is capable of being efficiently excited by the emitted blue light but not by the exciting ultraviolet light and of emitting green light when excited by the emitted blue light.

**17.** The fusion protein of claim 1, wherein the donor fluorescent protein moiety is capable of being excited by violet light to emit blue-green light and the acceptor fluorescent protein moiety is capable of being efficiently excited by the emitted blue-green light but not by the exciting violet light and of emitting yellow-green light when excited by the emitted blue-green light.

**18.** A fusion protein of claim 1, wherein the donor and acceptor fluorescent protein moieties are selected from among the green fluorescent proteins of cnidarians.

**19.** A fusion protein of claim 18, wherein the donor and acceptor fluorescent protein moieties are selected from or related to the fluorescent proteins of *Renilla reniformis, Phialidium gregarium,* and *Aequoria victoria.*

**20.** A fusion protein of claim 1, wherein the donor and acceptor fluorescent protein moieties are selected from the green fluorescent proteins of cnidarians; the yellow fluorescent proteins of *Vibrio fischeri* strain Y-1; peridinin-chlorophyll a binding protein from the dinoflagellate *Symbiodinium* sp.; phycobiliproteins from marine cyanobacteria, or oat phytochromes from oat reconstructed with phycoerythrobilin.

**21.** An isolated nucleic acid molecule encoding a fluorescent indicator fusion protein of any one of claims 1 to 20.

**22.** An expression vector comprising a nucleic acid encoding a fluorescent indicator fusion protein of any one of claims 1 to 20.

**23.** The expression vector of claim 22, wherein the nucleic acid encoding the fluorescent indicator fusion protein is operatively linked to an expression control sequence.

24. The expression vector of claim 23, wherein the vector is adapted for function in a prokaryotic cell or a eukaryotic cell.

25. A host cell transfected with an expression vector of claim 22.

26. The cell of claim 25, wherein the cell is a prokaryote.

27. The cell of claim 25, wherein the cell is a eukaryote.

28. The cell of claim 25, wherein the cell is a yeast cell.

29. The cell of claim 25, wherein the cell is a mammalian cell.

30. A method for determining the concentration in a sample of an analyte which is a ligand of a binding protein, comprising:

contacting the sample with a fusion protein of any one of claims 1 to 20;
exciting the donor fluorescent protein moiety; and
determining the degree of FRET in the sample by measuring light emitted by the acceptor fluorescent protein moiety.

31. A method of claim 30, wherein the analyte is calcium.

32. A method for determining the concentration of an analyte in a cell of claim 25, wherein the analyte is a ligand of the binding protein, comprising:

expressing the fusion protein in the cell;
exciting the donor fluorescent protein moiety; and
determining the degree of FRET in the sample by measuring light emitted by the acceptor fluorescent protein moiety.

33. A method of claim 32, wherein the cell is a mammalian cell.

34. A method of claim 32, wherein the analyte is calcium.

35. A method of claim 32, wherein the fusion protein comprises a localization moiety.


**Patentansprüche**

1. Fluoreszierendes Indikator-Fusionsprotein zum Nachweis eines Analyten, der ein Ligand eines Bindeproteins ist, wobei das Fusionsprotein umfasst:

einen Bindeprotein-Anteil mit der Liganden-Binderegion des Bindeproteins, der eine konformelle Änderung bei Bindung des Liganden durchläuft,
einen Donor-Fluoreszenzprotein-Anteil, wobei der Donor-Fluoreszenzprotein-Anteil an den Bindeprotein-Anteil fusioniert ist,
einen Akzeptor-Fluoreszenzprotein-Anteil, wobei der Akzeptor-Fluoreszenzprotein-Anteil an den Bindeprotein-Anteil fusioniert ist, und
wobei der Donor-Fluoreszenzprotein-Anteil ein Emissionsspektrum aufweist, das mit dem Absorptionsspektrum des Akzeptor-Fluoreszenzprotein-Anteils überlappt, und das Anregungsspektrum des Donor-Fluoreszenzprotein-Anteils eine Anregungswellenlänge umfasst, bei der der Donor-Fluoreszenzprotein-Anteil wirksam und selektiv im Hinblick auf den Akezptor-Fluoreszenzprotein-Anteil angeregt werden kann, das Emissionsspektrum des Donor-Fluoreszenzprotein-Anteils und das Emissionsspektrum des Akzeptor-Fluoreszenzprotein-Anteils eine Unterscheidung der zwei Emissionen ermöglichen und Fluoreszenzresonanzenergietransfer (FRET) zwischen dem Donor-Fluoreszenzprotein-Anteil und dem Akzeptor-Fluoreszenzprotein-Anteil nachgewiesen werden kann, wenn der Donor-Fluoreszenzprotein-Anteil bei der Wellenlänge angeregt wird, und

wobei der Akzeptor-Fluoreszenzprotein-Anteil und der Donor-Fluoreszenzprotein-Anteil ihre Position relativ zueinander verändern wenn der Ligand an die Liganden-Binderegion bindet, wodurch der FRET verändert wird.

2. Fusionsprotein nach Anspruch 1, wobei der Donor-Fluoreszenzportein-Anteil und der Akzeptor-Fluoreszenzprotein-Anteil mit Aequorea in Beziehung stehende Fluoreszenzprotein-Anteile sind.

3. Fusionsprotein nach Anspruch 1, wobei der Bindeprotein-Anteil weiterhin eine Domäne aufweist, die in der Lage ist, an einen Zielpeptid-Anteil zu binden, und der Zielpeptid-Anteil an den Bindeprotein-Anteil durch einen Polypeptid-Linker fusioniert ist und die Bindung des Liganden an die Liganden-Binderegion des Bindeprotein-Anteils die Bindung des Zielpeptid-Anteils an die Zielpeptid-Bindedomäne des Bindeprotein-Anteils verändert, um den FRET zu verändern.

4. Fusionsprotein nach Anspruch 1, wobei der Donor-Fluoreszenzprotein-Anteil oder der Akzeptor-Fluoreszenzprotein-Anteil an dem Carboxyterminus des Fusionsproteins fusioniert ist und der jeweils andere des Donor-Fluoreszenzprotein-Anteils oder des Akzeptor-Fluoreszenzprotein-Anteils an dem Aminoterminus des Fusionsproteins fusioniert ist.

5. Fusionsprotein nach Anspruch 1, wobei der Bindeprotein-Anteil Calmodulin, ein mit Calmodulin in Beziehung stehender Proteinanteil, cGMP-abhängige Proteinkinase, ein Steroidhormon-Rezeptor, eine Liganden-Bindedomäne eines Steroidhormon-Rezeptors, Proteinkinase C, Inositol-1,4,5-Triphosphat-Rezeptor oder Recoverin ist.

6. Fusionsprotein nach Anspruch 3, wobei der Bindeprotein-Anteil Calmodulin oder ein mit Calmodulin in Beziehung stehender Proteinanteil ist.

7. Fusionsprotein nach Anspruch 1, wobei der Donor-Fluoreszenzprotein-Anteil eine Mutante des grün-fluoreszierenden Proteins ist, die ausgewählt ist aus P4-3, EBFP oder W1B, und der Akzeptor-Fluoreszenzprotein-Anteil eine Mutante des grün-fluoreszierenden Proteins ist, die ausgewählt ist aus S65T, EGFP oder 10c.

8. Fusionsprotein nach Anspruch 3, wobei der Zielpeptid-Anteil eine Calmodulin-bindende Domäne von skMLCKp, smMLCK, CaMKII, Caldesmon, Calspermin, Phosphofructokinase, Calcineurin, Phosphorylasekinase, $Ca^{2+}$-ATPase, 59 kDa PDE, 60 kDa PDE, Stickstoffmonoxid-Synthase, Typ I-Adenylylcyclase, Bordetella pertussis-Adenylylcyclase, Neuromodulin, Spectrin, MARCKS, F52, β-Adducin, HSP90a, HIV-1 gp160, BBMHBI, Dilute MHC, Mastoparan, Melittin, Glucagon, Secretin, VIP, GIP oder Modelpeptid CBP2 ist.

9. Fusionsprotein nach Anspruch 3, wobei der Zielpeptid-Anteil M 13 ist.

10. Fusionsprotein nach Anspruch 9, wobei der Polypeptidlinker -Gly-Glyist.

11. Fusionsprotein nach Anspruch 1, wobei das Fusionsprotein ferner einen Lokalisierungsanteil umfasst.

12. Fusionsprotein nach Anspruch 11, wobei der Lokalisierungsanteil ein Lokalisierungsanteil für den Nucleus, das endoplasmatische Reticulum, ein Peroxisom, den Golgi-Apparat, die Plasmamembran oder die Mitochondrien ist.

13. Fusionsprotein nach Anspruch 1, wobei der Bindprotein-Anteil eine Konformation eines kleineren Volumens, wenn der Ligand an die Liganden-Binderegion des Bindeprotein-Anteils gebunden ist, im Vergleich zu dem Volumen des Bindeprotein-Anteils, wenn die Liganden-Binderegion nicht an den Liganden gebunden ist, umfasst, wobei der FRET durch die Bindung des Liganden an die Liganden-Binderegion des Bindeprotein-Anteils zunimmt.

14. Fusionsprotein nach Anspruch 3, wobei der Bindeprotein-Anteil eine erhöhte Affinität für den Zielpeptid-Anteil aufweist, wenn die Liganden-Binderegion des Bindeprotein-Anteils durch den Liganden gebunden ist.

15. Fusionsprotein nach Anspruch 3, wobei der Polypeptid-Linker eine bis 30 Aminosäuren lang ist.

16. Fusionsprotein nach Anspruch 1, wobei der Donor-Fluoreszenzprotein-Anteil durch ultraviolette Strahlung angeregt werden kann, um blaue Strahlung zu emittieren, und der Akzeptor-Fluoreszenzprotein-Anteil wirksam durch die emittierte blaue Strahlung, jedoch nicht durch die anregende ultraviolette Strahlung angeregt werden kann und grüne Strahlung bei einer Anregung durch die emittierte blaue Strahlung emittieren kann.

17. Fusionsprotein nach Anspruch 1, wobei der Donor-Fluoreszenzprotein-Anteil durch violette Strahlung angeregt werden kann, um blau-grüne Strahlung zu emittieren, und der Akzeptor-Fluoreszenzprotein-Anteil wirksam durch die emittierte blau-grüne Strahlung, jedoch nicht durch die anregende violette Strahlung angeregt werden kann und gelb-grüne Strahlung bei einer Anregung durch die emittierte blau-grüne Strahlung emittieren kann.

18. Fusionsprotein nach Anspruch 1, wobei die Donor- und Akzeptor-Fluoreszenzprotein-Anteile aus den grün-fluoreszierenden Proteinen von Cnidariern ausgewählt sind.

19. Fusionsprotein nach Anspruch 18, wobei die Donor- und Akzeptor-Fluoreszenzprotein-Anteile aus den Fluoreszenzproteinen von Renilla reniformis, Phialidium gregarium und Aequoria victoria ausgewählt sind oder damit in Beziehung stehen.

20. Fusionsprotein nach Anspruch 1, wobei die Donor- und Akzeptor-Fluoreszenzprotein-Anteile aus den grün-fluoreszierenden Proteinen von Cnidariern, den gelb-fluoreszierenden Proteinen von Vibrio fischeri, Stamm Y-1, Peridinin-Chlorophyll a-bindendem Protein des Dinoflagellaten Symbiodinium sp., Phycobiliproteinen von marinen Cyanobakterien oder Hafer-Phytochromen aus Hafer, die mit Phycoerythrobilin rekonstruiert wurden, ausgewählt sind.

21. Isoliertes Nukleinsäuremolekül, das für ein fluoreszierendes Indikator-Fusionsprotein nach einem der Ansprüche 1 bis 20 kodiert.

22. Expressionsvektor, der eine Nukleinsäure umfasst, die für ein fluoreszierendes Indikator-Fusionsprotein nach einem der Ansprüche 1 bis 20 kodiert.

23. Expressionsvektor nach Anspruch 22, wobei die Nukleinsäure, die für das fluoreszierendes Indikator-Fusionsprotein kodiert, mit einer Expressionskontrollsequenz operabel verbunden ist.

24. Expressionsvektor nach Anspruch 23, wobei der Vektor für eine Funktion in einer prokaryontischen Zelle oder einer eukaryontischen Zelle angepasst ist.

25. Wirtszelle, die mit einem Expressionsvektor nach Anspruch 22 transfiziert ist.

26. Zelle nach Anspruch 25, wobei die Zelle ein Prokaryont ist.

27. Zelle nach Anspruch 25, wobei die Zelle ein Eukaryont ist.

28. Zelle nach Anspruch 25, wobei die Zelle eine Hefezelle ist.

29. Zelle nach Anspruch 25, wobei die Zelle eine Säugerzelle ist.

30. Verfahren zum Bestimmen der Konzentration eines Analyten, der ein Ligand eines Bindeproteins ist, in einer Probe, umfassend:

In Kontakt-Bringen der Probe mit einem Fusionsprotein nach einem der Ansprüche 1 bis 20,
Anregen des Donor-Fluoreszenzprotein-Anteils und
Bestimmen des Grads an FRET in der Probe durch Messen von durch den Akzeptor-Fluoreszenzprotein-Anteil emittierter Strahlung.

31. Verfahren nach Anspruch 30, wobei der Analyt Calcium ist.

32. Verfahren zum Bestimmen der Konzentration eines Analyten in einer Zelle nach Anspruch 25, wobei der Analyt ein Ligand des Bindeproteins ist, umfassend:

Expression des Fusionsproteins in der Zelle,
Anregen des Donor-Fluoreszenzprotein-Anteils und
Bestimmen des Grads an FRET in der Probe durch Messen von durch den Akzeptor-Fluoreszenzprotein-Anteil emittierter Strahlung.

33. Verfahren nach Anspruch 32, wobei die Zelle eine Säugerzelle ist.

**34.** Verfahren nach Anspruch 32, wobei der Analyt Calcium ist.

**35.** Verfahren nach Anspruch 32, wobei das Fusionsprotein einen Lokalisierungsanteil umfasst.

**Revendications**

**1.** Protéine de fusion indicatrice fluorescente pour la détection d'un analyte qui est un ligand d'une protéine de liaison, dans laquelle la protéine de fusion comprend:

une portion protéique de liaison ayant la région de liaison de ligand de la protéine de liaison et qui subit une modification conformationnelle lors de la liaison du ligand;
une portion protéique fluorescente donneur, tandis que la portion protéique fluorescente donneur est fusionnée à la portion de protéine de liaison;
une portion protéique fluorescente accepteur, tandis que la portion protéique fluorescente accepteur est fusionnée à la portion de protéine de liaison; et
tandis que la portion protéique fluorescente donneur a un spectre d'émission qui se chevauche avec le spectre d'absorption de la portion protéique fluorescente accepteur, et le spectre d'excitation de la portion fluorescente donneur comprend une longueur d'onde d'excitation à laquelle la portion protéique fluorescente donneur peut être excitée efficacement et sélectivement vis-à-vis de la portion protéique fluorescente accepteur; et le spectre d'émission de la portion protéique fluorescente donneur et le spectre d'émission de la portion protéique fluorescente accepteur permettent que les deux émissions soient distinguées, et le transfert d'énergie de résonance de fluorescence (FRET) entre la portion protéique fluorescente donneur et la portion protéique fluorescente accepteur peut être détecté lorsque la portion protéique fluorescente donneur est excitée à la longueur d'onde; et

tandis que la portion protéique fluorescente accepteur et la portion protéique fluorescente donneur changent de position l'une par rapport à l'autre quand le ligand se lie à la région de liaison de ligand, en modifiant le FRET.

**2.** Protéine de fusion selon la revendication 1, dans laquelle la portion protéique fluorescente donneur et la portion protéique fluorescente accepteur sont des portions protéiques fluorescentes apparentées à Aequorea.

**3.** Protéine de fusion selon la revendication 1, dans laquelle la portion protéique de liaison présente en outre un domaine capable de se lier à une portion peptidique cible et la portion peptidique cible est fusionnée à la portion de protéine de liaison par un lieur polypeptidique et la liaison du ligand à la région de liaison de ligand de la portion protéique de liaison modifie la liaison de la portion peptidique cible au domaine de liaison peptidique cible de la portion protéique de liaison pour modifier le FRET.

**4.** Protéine de fusion selon la revendication 1, dans laquelle l'une parmi la portion protéique fluorescente donneur ou la portion protéique accepteur est fusionnée à l'extrémité carboxy-terminale de la protéine de fusion et l'autre parmi la portion protéique fluorescente donneur ou la portion protéique accepteur est fusionnée à l'extrémité amino-terminale de la protéine de fusion.

**5.** Protéine de fusion selon la revendication 1, dans laquelle la portion protéique de liaison est la calmoduline, une entité protéique apparentée à la calmoduline, une protéine kinase dépendante de cGMP, un récepteur d'hormone stéroïdienne, un domaine de liaison de ligand d'un récepteur d'hormone stéroïdienne, la protéine kinase C, le récepteur d'inositol-1,4,5-triphosphate, ou la recovérine.

**6.** Protéine de fusion selon la revendication 3, dans laquelle la portion protéique de liaison est la calmoduline ou une entité protéique apparentée à la calmoduline.

**7.** Protéine de fusion selon la revendication 1, dans laquelle la portion protéique fluorescente donneur est un mutant de protéine fluorescente verte choisi parmi P4-3, EBFP, ou W1B, et la portion protéique fluorescente accepteur est un mutant de protéine fluorescente verte choisi parmi S65T, EGFP, ou 10c.

**8.** Protéine de fusion selon la revendication 3, dans laquelle la portion peptidique cible est un domaine de liaison à la calmoduline de skMLCKp, smMLCK, CaMKII, Caldesmone, Calspermine, phosphofructokinase, calcineurine, phosphorylase kinase, $Ca^{2+}$-ATPase, PDE de 59 kDa, PDE de 60 kDa, oxyde nitrique synthase, adénylyl cyclase de type I, adénylyl cyclase de *Bordetella pertussis,* Neuromoduline, Spectrine, MARCKS, F52, β-Adducine, HSP90a, gp160 de VIH-1, BBMHBI, CMH dilué, Mastoparan, Mélittine, Glucagon, Sécrétine, VIP, GIP, ou peptide modèle CBP2.

9. Protéine de fusion selon la revendication 3, dans laquelle la portion peptidique cible est M13.

10. Protéine de fusion selon la revendication 9, dans laquelle le lieur polypeptidique est -Gly-Gly-.

11. Protéine de fusion selon la revendication 1, dans laquelle la protéine de fusion comprend en outre une portion de localisation.

12. Protéine de fusion selon la revendication 11, dans laquelle la portion de localisation est une portion de localisation pour le noyau, le réticulum endoplasmique, un peroxysome, l'appareil de Golgi, la membrane plasmatique ou les mitochondries.

13. Protéine de fusion selon la revendication 1, dans laquelle la portion protéique de liaison comporte une conformation de plus faible volume lorsque le ligand est lié à la région de liaison de ligand de la portion protéique de liaison par comparaison avec le volume de la portion protéique de liaison lorsque la région de liaison de ligand n'est pas liée au ligand, tandis que le FRET est augmenté par la liaison du ligand à la région de liaison de ligand de la portion protéique de liaison.

14. Protéine de fusion selon la revendication 3, dans laquelle la portion protéique de liaison a une affinité accrue pour la portion peptidique cible lorsque la région de liaison de ligand de la portion protéique de liaison est liée par le ligand.

15. Protéine de fusion selon la revendication 3, dans laquelle le lieur polypeptidique a une longueur de un à 30 acides aminés.

16. Protéine de fusion selon la revendication 1, dans laquelle la portion protéique fluorescente donneur est capable d'être excitée par la lumière ultraviolette pour émettre une lumière bleue et la portion protéique fluorescente accepteur est capable d'être efficacement excitée par la lumière bleue émise, mais pas par la lumière ultraviolette d'excitation et d'émettre une lumière verte lorsqu'elle est excitée par la lumière bleue émise.

17. Protéine de fusion selon la revendication 1, dans laquelle la portion protéique fluorescente donneur est capable d'être excitée par la lumière violette pour émettre une lumière bleu-vert et la portion protéique fluorescente accepteur est capable d'être efficacement excitée par la lumière bleu-vert émise, mais pas par la lumière violette d'excitation et d'émettre une lumière jaune-vert lorsqu'elle est excitée par la lumière bleu-vert émise.

18. Protéine de fusion selon la revendication 1, dans laquelle les portions protéiques fluorescentes donneur et accepteur sont choisies parmi les protéines fluorescentes vertes de cnidaires.

19. Protéine de fusion selon la revendication 18, dans laquelle les portions protéiques fluorescentes donneur et accepteur sont choisies parmi les ou apparentées aux protéines fluorescentes vertes de *Renilla reniformis, Phialidium gregarium,* et *Aequoria victoria.*

20. Protéine de fusion selon la revendication 1, dans laquelle les portions protéiques fluorescentes donneur et accepteur sont choisies parmi les protéines fluorescentes vertes de cnidaires, les protéines fluorescentes jaunes de souche Y-1 de *Vibrio fischeri,* la protéine de liaison à la chlorophylle a de péridinine provenant de dinoflagellé *Symbiodinium* sp., de phycobiliprotéines de cyanobactéries marines, ou de phytochromes d'avoine provenant d'avoine reconstruite avec de la phycoérythrobiline.

21. Molécule d'acide nucléique isolée codant pour une protéine de fusion indicatrice fluorescente selon l'une quelconque des revendications 1 à 20.

22. Vecteur d'expression comprenant un acide nucléique codant pour une protéine de fusion indicatrice fluorescente selon l'une quelconque des revendications 1 à 20.

23. Vecteur d'expression selon la revendication 22, dans lequel l'acide nucléique codant pour la protéine de fusion indicatrice fluorescente est lié de manière opérationnelle à une séquence de contrôle d'expression.

24. Vecteur d'expression selon la revendication 23, dans lequel le vecteur est adapté pour fonctionner dans une cellule procaryote ou une cellule eucaryote.

25. Cellule hôte transfectée avec un vecteur d'expression selon la revendication 22.

26. Cellule selon la revendication 25, dans laquelle la cellule est un procaryote.

27. Cellule selon la revendication 25, dans laquelle la cellule est un eucaryote.

28. Cellule selon la revendication 25, dans laquelle la cellule est une cellule de levure.

29. Cellule selon la revendication 25, dans laquelle la cellule est une cellule mammalienne.

30. Procédé pour la détermination de la concentration dans un échantillon d'un analyte qui est un ligand d'une protéine de liaison, comprenant :

la mise en contact de l'échantillon avec une protéine de fusion selon l'une quelconque des revendications 1 à 20;
l'excitation de la portion protéique fluorescente donneur; et
la détermination du degré de FRET dans l'échantillon par mesure de la lumière émise par la portion protéique fluorescente accepteur.

31. Procédé selon la revendication 30, dans lequel l'analyte est le calcium.

32. Procédé pour la détermination de la concentration d'un analyte dans une cellule selon la revendication 25, tandis que l'analyte est un ligand de la protéine de liaison, comprenant:

l'expression de la protéine de fusion dans la cellule;
l'excitation de la portion protéique fluorescente donneur; et
la détermination du degré de FRET dans l'échantillon par mesure de la lumière émise par la portion protéique fluorescente accepteur.

33. Procédé selon la revendication 32, dans lequel la cellule est une cellule mammalienne.

34. Procédé selon la revendication 32, dans lequel l'analyte est le calcium.

35. Procédé selon la revendication 32, dans lequel la protéine de fusion comprend une portion de localisation.

FIG. 1

FIG. 2a

50 a.a.

CaM-M13

GlyGly

HIS

| P4-3 DONOR | XCaM | | S65T ACCEPTOR |

EP 0 970 199 B1

| 226 | 227 | | 1 | 2 | | 20 | 21 | | | 1 | 2 |
| Ala | Ala | Arg | Met | His | | Ile | Ser | Glu | Leu | Met | Ser |
| GCT | GCT | CGC | ATG | CAT | | ATC | TCC | GAG | CTC | ATG | AGT |

*Sph*I

*Sac*I

FIG. 2b

FIG. 3

FIG. 4a

FIG. 4b

EP 0 970 199 B1

CaM-M13

**cameleon-2**　　　kz

**cameleon-2nu**　　kz　　　　　　　　　　　　　PKKKRKVEDA

**cameleon-3**　　　kz　　　　　　　E104Q

**cameleon-3er**

kz MLLSVPLLLGLLGLAAAD　　E104Q　　　　KDEL

FIG. 5

EP 0 970 199 B1

FIG. 6 a

FIG. 6 b

Emission Ratio

0.2 mMATP

0.2 mM histamine

10 μM cyproheptadine

1 μM ionomycin

15 mM CaCl2

Time (seconds)

FIG. 6c

FIG. 6d

Time (seconds)

Emission Ratio

0.2 mM histamine
10 µM cyproheptadine
0.2 mM ATP
2 µM ionomycin
2 mM CaCl2

FIG. 6e

FIG. 6f

FIG. 7

(SEQ ID. NO:1)

ATGGTGAGCAAGGGCGAGGAGCTGTTCACCGGGGTGGTGCCCATCCTGGTCGAGCTGGACGGCGA
CGTAAACGGCCACAAGTTCAGCGTGTCCGGCGAGGGCGAGGGCGATGCCACCTACGGCAAGCTGA
CCCTGAAGTTCATCTGCACCACCGGCAAGCTGCCCGTGCCCTGGCCCACCCTCGTGACCACCCTG
ACCCATGGCGTGCAGTGCTTCAGCCGCTACCCCGACCACATGAAGCAGCACGACTTCTTCAAGTC
CGCCATGCCCGAAGGCTACGTCCAGGAGCGCACCATCTTCTTCAAGGACGACGGCAACTACAAGA
CCCGCGCCGAGGTGAAGTTCGAGGGCGACACCCTGGTGAACCGCATCGAGCTGAAGGGCATCGAC
TTCAAGGAGGACGGCAACATCCTGGGGCACAAGCTGGAGTACAACTTCAACAGCCACAACGTCTA
TATCATGGCCGACAAGCAGAAGAACGGCATCAAGGTGAACTTCAAGATCCGCCACAACATCGAGG
ACGGCAGCGTGCAGCTCGCCGACCACTACCAGCAGAACACCCCCATCGGCGACGGCCCCGTGCTG
CTGCCCGACAACCACTACCTGAGCACCCAGTCCGCCCTGAGCAAAGACCCCAACGAGAAGCGCGA
TCACATGGTCCTGCTGGAGTTCGTGACCGCCGCCCGCATGCATGACCAACTGACAGAAGAGCAGA
TTGCAGAGTTCAAAGAAGCCTTCTCATTATTCGACAAGGATGGGGACGGCACCATCACCACAAAG
GAACTTGGCACCGTTATGAGGTCGCTTGGACAAAACCCAACGGAAGCAGAATTGCAGGATATGAT
CAATGAAGTCGATGCTGATGGCAATGGAACGATTTACTTTCCTGAATTTCTTACTATGATGGCTA
GAAAAATGAAGGACACAGACAGCGAAGAGGAAATCCGAGAAGCATTCCGTGTTTTTGACAAGGAT
GGGAACGGCTACATCAGCGCTGCTGAATTACGTCACGTCATGACAAACCTCGGGGAGAAGTTAAC
AGATGAAGAAGTTGATGAAATGATAAGGGAAGCAGATATCGATGGTGATGGCCAAGTAAACTATG
AAGAGTTTGTACAAATGATGACAGCAAAGGGGGGGAAGAGGCGCTGGAAGAAAAACTTCATTGCC
GTCAGCGCTGCCAACCGGTTCAAGAAGATCTCCGAGCTCATGGTGAGCAAGGGCGAGGAGCTGTT
CACCGGGGTGGTGCCCATCCTGGTCGAGCTGGACGGCGACGTAAACGGCCACAAGTTCAGCGTGT
CCGGCGAGGGCGAGGGCGATGCCACCTACGGCAAGCTGACCCTGAAGTTCATCTGCACCACCGGC
AAGCTGCCCGTGCCCTGGCCCACCCTCGTGACCACCCTGACCTACGGCGTGCAGTGCTTCAGCCG
CTACCCCGACCACATGAAGCAGCACGACTTCTTCAAGTCCGCCATGCCCGAAGGCTACGTCCAGG
AGCGCACCATCTTCTTCAAGGACGACGGCAACTACAAGACCCGCGCCGAGGTGAAGTTCGAGGGC
GACACCCTGGTGAACCGCATCGAGCTGAAGGGCATCGACTTCAAGGAGGACGGCAACATCCTGGG
GCACAAGCTGGAGTACAACTACAACAGCCACAACGTCTATATCATGGCCGACAAGCAGAAGAACG
GCATCAAGGTGAACTTCAAGATCCGCCACAACATCGAGGACGGCAGCGTGCAGCTCGCCGACCAC
TACCAGCAGAACACCCCCATCGGCGACGGCCCCGTGCTGCTGCCCGACAACCACTACCTGAGCAC
CCAGTCCGCCCTGAGCAAAGACCCCAACGAGAAGCGCGATCACATGGTCCTGCTGGAGTTCGTGA
CCGCCGCCGGGATCACTCTCGGCATGGACGAGCTGTACAAGTAA

FIG. 7 (continued)

(SEQ ID. NO:2)

MVSKGEELFTGVVPILVELDGDVNGHKFSVSGEGEGDATYGKLTLKFICTTGKLPVPWPTLVTTL

THGVQCFSRYPDHMKQHDFFKSAMPEGYVQERTIFFKDDGNYKTRAEVKFEGDTLVNRIELKGID

FKEDGNILGHKLEYNFNSHNVYIMADKQKNGIKVNFKIRHNIEDGSVQLADHYQQNTPIGDGPVL

LPDNHYLSTQSALSKDPNEKRDHMVLLEFVTAARMHDQLTEEQIAEFKEAFSLFDKDGDGTITTK

ELGTVMRSLGQNPTEAELQDMINEVDADGNGTIYFPEFLTMMARKMKDTDSEEEIREAFRVFDKD

GNGYISAAELRHVMTNLGEKLTDEEVDEMIREADIDGDGQVNYEEFVQMMTAKGGKRRWKKNFIA

VSAANRFKKISELMVSKGEELFTGVVPILVELDGDVNGHKFSVSGEGEGDATYGKLTLKFICTTG

KLPVPWPTLVTTLTYGVQCFSRYPDHMKQHDFFKSAMPEGYVQERTIFFKDDGNYKTRAEVKFEG

DTLVNRIELKGIDFKEDGNILGHKLEYNYNSHNVYIMADKQKNGIKVNFKIRHNIEDGSVQLADH

YQQNTPIGDGPVLLPDNHYLSTQSALSKDPNEKRDHMVLLEFVTAAGITLGMDELYK*

FIG. 8

(SEQ ID. NO:3)

ATGGTGAGCAAGGGCGAGGAGCTGTTCACCGGGGTGGTGCCCATCCTGGTCGAGCTGGACGGCGA
CGTAAACGGCCACAAGTTCAGCGTGTCCGGCGAGGGCGAGGGCGATGCCACCTACGGCAAGCTGA
CCCTGAAGTTCATCTGCACCACCGGCAAGCTGCCCGTGCCCTGGCCCACCCTCGTGACCACCCTG
ACCCATGGCGTGCAGTGCTTCAGCCGCTACCCCGACCACATGAAGCAGCACGACTTCTTCAAGTC
CGCCATGCCCGAAGGCTACGTCCAGGAGCGCACCATCTTCTTCAAGGACGACGGCAACTACAAGA
CCCGCGCCGAGGTGAAGTTCGAGGGCGACACCCTGGTGAACCGCATCGAGCTGAAGGGCATCGAC
TTCAAGGAGGACGGCAACATCCTGGGGCACAAGCTGGAGTACAACTTCAACAGCCACAACGTCTA
TATCATGGCCGACAAGCAGAAGAACGGCATCAAGGTGAACTTCAAGATCCGCCACAACATCGAGG
ACGGCAGCGTGCAGCTCGCCGACCACTACCAGCAGAACACCCCCATCGGCGACGGCCCCGTGCTG
CTGCCCGACAACCACTACCTGAGCACCCAGTCCGCCCTGAGCAAAGACCCCAACGAGAAGCGCGA
TCACATGGTCCTGCTGGAGTTCGTGACCGCCGCCCGCATGCATGACCAACTGACAGAAGAGCAGA
TTGCAGAGTTCAAAGAAGCCTTCTCATTATTCGACAAGGATGGGGACGGCACCATCACCACAAAG
GAACTTGGCACCGTTATGAGGTCGCTTGGACAAAACCCAACGGAAGCAGAATTGCAGGATATGAT
CAATGAAGTCGATGCTGATGGCAATGGAACGATTTACTTTCCTGAATTTCTTACTATGATGGCTA
GAAAAATGAAGGACACAGACAGCGAAGAGGAAATCCGAGAAGCATTCCGTGTTTTTGACAAGGAT
GGGAACGGCTACATCAGCGCTGCTGAATTACGTCACGTCATGACAAACCTCGGGGAGAAGTTAAC
AGATGAAGAAGTTGATGAAATGATAAGGGAAGCAGATATCGATGGTGATGGCCAAGTAAACTATG
AAGAGTTTGTACAAATGATGACAGCAAAGGGGGGGAAGAGGCGCTGGAAGAAAAACTTCATTGCC
GTCAGCGCTGCCAACCGGTTCAAGAAGATCTCCGAGCTCATGGTGAGCAAGGGCGAGGAGCTGTT
CACCGGGGTGGTGCCCATCCTGGTCGAGCTGGACGGCGACGTAAACGGCCACAAGTTCAGCGTGT
CCGGCGAGGGCGAGGGCGATGCCACCTACGGCAAGCTGACCCTGAAGTTCATCTGCACCACCGGC
AAGCTGCCCGTGCCCTGGCCCACCCTCGTGACCACCCTGACCTACGGCGTGCAGTGCTTCAGCCG
CTACCCCGACCACATGAAGCAGCACGACTTCTTCAAGTCCGCCATGCCCGAAGGCTACGTCCAGG
AGCGCACCATCTTCTTCAAGGACGACGGCAACTACAAGACCCGCGCCGAGGTGAAGTTCGAGGGC
GACACCCTGGTGAACCGCATCGAGCTGAAGGGCATCGACTTCAAGGAGGACGGCAACATCCTGGG
GCACAAGCTGGAGTACAACTACAACAGCCACAACGTCTATATCATGGCCGACAAGCAGAAGAACG
GCATCAAGGTGAACTTCAAGATCCGCCACAACATCGAGGACGGCAGCGTGCAGCTCGCCGACCAC
TACCAGCAGAACACCCCCATCGGCGACGGCCCCGTGCTGCTGCCCGACAACCACTACCTGAGCAC
CCAGTCCGCCCTGAGCAAAGACCCCAACGAGAAGCGCGATCACATGGTCCTGCTGGAGTTCGTGA
CCGCCGCCGGGATCACTCTCGGCATGGACGAGCTGTACAAGCCAAAAAAGAAGAGAAAGGTGGAA
GACGCTTAA

FIG. 8 (continued)

(SEQ ID. NO:4)

MVSKGEELFTGVVPILVELDGDVNGHKFSVSGEGEGDATYGKLTLKFICTTGKLPVPWPTLVTTL

THGVQCFSRYPDHMKQHDFFKSAMPEGYVQERTIFFKDDGNYKTRAEVKFEGDTLVNRIELKGID

FKEDGNILGHKLEYNFNSHNVYIMADKQKNGIKVNFKIRHNIEDGSVQLADHYQQNTPIGDGPVL

LPDNHYLSTQSALSKDPNEKRDHMVLLEFVTAARMHDQLTEEQIAEFKEAFSLFDKDGDGTITTK

ELGTVMRSLGQNPTEAELQDMINEVDADGNGTIYFPEFLTMMARKMKDTDSEEEIREAFRVFDKD

GNGYISAAELRHVMTNLGEKLTDEEVDEMIREADIDGDGQVNYEEFVQMMTAKGGKRRWKKNFIA

VSAANRFKKISELMVSKGEELFTGVVPILVELDGDVNGHKFSVSGEGEGDATYGKLTLKFICTTG

KLPVPWPTLVTTLTYGVQCFSRYPDHMKQHDFFKSAMPEGYVQERTIFFKDDGNYKTRAEVKFEG

DTLVNRIELKGIDFKEDGNILGHKLEYNYNSHNVYIMADKQKNGIKVNFKIRHNIEDGSVQLADH

YQQNTPIGDGPVLLPDNHYLSTQSALSKDPNEKRDHMVLLEFVTAAGITLGMDELYKPKKKRKVE

DA*

FIG. 9

(SEQ ID. NO:5)

```
ATGGTGAGCAAGGGCGAGGAGCTGTTCACCGGGGTGGTGCCCATCCTGGTCGAGCTGGACGGCGA
CGTAAACGGCCACAAGTTCAGCGTGTCCGGCGAGGGCGAGGGCGATGCCACCTACGGCAAGCTGA
CCCTGAAGTTCATCTGCACCACCGGCAAGCTGCCCGTGCCCTGGCCCACCCTCGTGACCACCCTG
ACCCATGGCGTGCAGTGCTTCAGCCGCTACCCCGACCACATGAAGCAGCACGACTTCTTCAAGTC
CGCCATGCCCGAAGGCTACGTCCAGGAGCGCACCATCTTCTTCAAGGACGACGGCAACTACAAGA
CCCGCGCCGAGGTGAAGTTCGAGGGCGACACCCTGGTGAACCGCATCGAGCTGAAGGGCATCGAC
TTCAAGGAGGACGGCAACATCCTGGGGCACAAGCTGGAGTACAACTTCAACAGCCACAACGTCTA
TATCATGGCCGACAAGCAGAAGAACGGCATCAAGGTGAACTTCAAGATCCGCCACAACATCGAGG
ACGGCAGCGTGCAGCTCGCCGACCACTACCAGCAGAACACCCCCATCGGCGACGGCCCCGTGCTG
CTGCCCGACAACCACTACCTGAGCACCCAGTCCGCCCTGAGCAAAGACCCCAACGAGAAGCGCGA
TCACATGGTCCTGCTGGAGTTCGTGACCGCCGCCCGCATGCATGACCAACTGACAGAAGAGCAGA
TTGCAGAGTTCAAAGAAGCCTTCTCATTATTCGACAAGGATGGGGACGGCACCATCACCACAAAG
GAACTTGGCACCGTTATGAGGTCGCTTGGACAAAACCCAACGGAAGCAGAATTGCAGGATATGAT
CAATGAAGTCGATGCTGATGGCAATGGAACGATTTACTTTCCTGAATTTCTTACTATGATGGCTA
GAAAAATGAAGGACACAGACAGCGAAGAGGAAATCCGAGAAGCATTCCGTGTTTTTGACAAGGAT
GGGAACGGCTACATCAGCGCTGCTCAGTTACGTCACGTCATGACAAACCTCGGGGAGAAGTTAAC
AGATGAAGAAGTTGATGAAATGATAAGGGAAGCAGATATCGATGGTGATGGCCAAGTAAACTATG
AAGAGTTTGTACAAATGATGACAGCAAAGGGGGGGAAGAGGCGCTGGAAGAAAAACTTCATTGCC
GTCAGCGCTGCCAACCGGTTCAAGAAGATCTCCGAGCTCATGGTGAGCAAGGGCGAGGAGCTGTT
CACCGGGGTGGTGCCCATCCTGGTCGAGCTGGACGGCGACGTAAACGGCCACAAGTTCAGCGTGT
CCGGCGAGGGCGAGGGCGATGCCACCTACGGCAAGCTGACCCTGAAGTTCATCTGCACCACCGGC
AAGCTGCCCGTGCCCTGGCCCACCCTCGTGACCACCCTGACCTACGGCGTGCAGTGCTTCAGCCG
CTACCCCGACCACATGAAGCAGCACGACTTCTTCAAGTCCGCCATGCCCGAAGGCTACGTCCAGG
AGCGCACCATCTTCTTCAAGGACGACGGCAACTACAAGACCCGCGCCGAGGTGAAGTTCGAGGGC
GACACCCTGGTGAACCGCATCGAGCTGAAGGGCATCGACTTCAAGGAGGACGGCAACATCCTGGG
GCACAAGCTGGAGTACAACTACAACAGCCACAACGTCTATATCATGGCCGACAAGCAGAAGAACG
GCATCAAGGTGAACTTCAAGATCCGCCACAACATCGAGGACGGCAGCGTGCAGCTCGCCGACCAC
TACCAGCAGAACACCCCCATCGGCGACGGCCCCGTGCTGCTGCCCGACAACCACTACCTGAGCAC
CCAGTCCGCCCTGAGCAAAGACCCCAACGAGAAGCGCGATCACATGGTCCTGCTGGAGTTCGTGA
CCGCCGCCGGGATCACTCTCGGCATGGACGAGCTGTACAAGTAA
```

FIG. 9 (continued)

(SEQ ID. NO:6)

MVSKGEELFTGVVPILVELDGDVNGHKFSVSGEGEGDATYGKLTLKFICTTGKLPVPWPTLVTTL
THGVQCFSRYPDHMKQHDFFKSAMPEGYVQERTIFFKDDGNYKTRAEVKFEGDTLVNRIELKGID
FKEDGNILGHKLEYNFNSHNVYIMADKQKNGIKVNFKIRHNIEDGSVQLADHYQQNTPIGDGPVL
LPDNHYLSTQSALSKDPNEKRDHMVLLEFVTAARMHDQLTEEQIAEFKEAFSLFDKDGDGTITTK
ELGTVMRSLGQNPTEAELQDMINEVDADGNGTIYFPEFLTMMARKMKDTDSEEEIREAFRVFDKD
GNGYISAAQLRHVMTNLGEKLTDEEVDEMIREADIDGDGQVNYEEFVQMMTAKGGKRRWKKNFIA
VSAANRFKKISELMVSKGEELFTGVVPILVELDGDVNGHKFSVSGEGEGDATYGKLTLKFICTTG
KLPVPWPTLVTTLTYGVQCFSRYPDHMKQHDFFKSAMPEGYVQERTIFFKDDGNYKTRAEVKFEG
DTLVNRIELKGIDFKEDGNILGHKLEYNYNSHNVYIMADKQKNGIKVNFKIRHNIEDGSVQLADH
YQQNTPIGDGPVLLPDNHYLSTQSALSKDPNEKRDHMVLLEFVTAAGITLGMDELYK*

FIG. 10

(SEQ ID. NO:7)

ATGCTGCTGCCCGTCCCCCTGCTGCTGGGCCTGCTGGGCGCCGCCGCCGACGTGAGCAAGGGCGA
GGAGCTGTTCACCGGGGTGGTGCCCATCCTGGTCGAGCTGGACGGCGACGTAAACGGCCACAAGT
TCAGCGTGTCCGGCGAGGGCGAGGGCGATGCCACCTACGGCAAGCTGACCCTGAAGTTCATCTGC
ACCACCGGCAAGCTGCCCGTGCCCTGGCCCACCCTCGTGACCACCCTGACCCATGGCGTGCAGTG
CTTCAGCCGCTACCCCGACCACATGAAGCAGCACGACTTCTTCAAGTCCGCCATGCCCGAAGGCT
ACGTCCAGGAGCGCACCATCTTCTTCAAGGACGACGGCAACTACAAGACCCGCGCCGAGGTGAAG
TTCGAGGGCGACACCCTGGTGAACCGCATCGAGCTGAAGGGCATCGACTTCAAGGAGGACGGCAA
CATCCTGGGGCACAAGCTGGAGTACAACTTCAACAGCCACAACGTCTATATCATGGCCGACAAGC
AGAAGAACGGCATCAAGGTGAACTTCAAGATCCGCCACAACATCGAGGACGGCAGCGTGCAGCTC
GCCGACCACTACCAGCAGAACACCCCCATCGGCGACGGCCCCGTGCTGCTGCCCGACAACCACTA
CCTGAGCACCCAGTCCGCCCTGAGCAAAGACCCCAACGAGAAGCGCGATCACATGGTCCTGCTGG
AGTTCGTGACCGCCGCCCGCATGCATGACCAACTGACAGAAGAGCAGATTGCAGAGTTCAAAGAA
GCCTTCTCATTATTCGACAAGGATGGGGACGGCACCATCACCACAAAGGAACTTGGCACCGTTAT
GAGGTCGCTTGGACAAAACCCAACGGAAGCAGAATTGCAGGATATGATCAATGAAGTCGATGCTG
ATGGCAATGGAACGATTTACTTTCCTGAATTTCTTACTATGATGGCTAGAAAAATGAAGGACACA
GACAGCGAAGAGGAAATCCGAGAAGCATTCCGTGTTTTTGACAAGGATGGGAACGGCTACATCAG
CGCTGCTCAGTTACGTCACGTCATGACAAACCTCGGGGAGAAGTTAACAGATGAAGAAGTTGATG
AAATGATAAGGGAAGCAGATATCGATGGTGATGGCCAAGTAAACTATGAAGAGTTTGTACAAATG
ATGACAGCAAAGGGGGGGAAGAGGCGCTGGAAGAAAAACTTCATTGCCGTCAGCGCTGCCAACCG
GTTCAAGAAGATCTCCGAGCTCATGGTGAGCAAGGGCGAGGAGCTGTTCACCGGGGTGGTGCCCA
TCCTGGTCGAGCTGGACGGCGACGTAAACGGCCACAAGTTCAGCGTGTCCGGCGAGGGCGAGGGC
GATGCCACCTACGGCAAGCTGACCCTGAAGTTCATCTGCACCACCGGCAAGCTGCCCGTGCCCTG
GCCCACCCTCGTGACCACCCTGACCTACGGCGTGCAGTGCTTCAGCCGCTACCCCGACCACATGA
AGCAGCACGACTTCTTCAAGTCCGCCATGCCCGAAGGCTACGTCCAGGAGCGCACCATCTTCTTC
AAGGACGACGGCAACTACAAGACCCGCGCCGAGGTGAAGTTCGAGGGCGACACCCTGGTGAACCG
CATCGAGCTGAAGGGCATCGACTTCAAGGAGGACGGCAACATCCTGGGGCACAAGCTGGAGTACA
ACTACAACAGCCACAACGTCTATATCATGGCCGACAAGCAGAAGAACGGCATCAAGGTGAACTTC
AAGATCCGCCACAACATCGAGGACGGCAGCGTGCAGCTCGCCGACCACTACCAGCAGAACACCCC
CATCGGCGACGGCCCCGTGCTGCTGCCCGACAACCACTACCTGAGCACCCAGTCCGCCCTGAGCA
AAGACCCCAACGAGAAGCGCGATCACATGGTCCTGCTGGAGTTCGTGACCGCCGCCGGGATCACT
CTCGGCAAGGACGAGCTGTAA

FIG. 10 (continued)

(SEQ ID. NO:8)

MLLPVPLLLGLLGAAADVSKGEELFTGVVPILVELDGDVNGHKFSVSGEGEGDATYGKLTLKFIC

TTGKLPVPWPTLVTTLTHGVQCFSRYPDHMKQHDFFKSAMPEGYVQERTIFFKDDGNYKTRAEVK

FEGDTLVNRIELKGIDFKEDGNILGHKLEYNFNSHNVYIMADKQKNGIKVNFKIRHNIEDGSVQL

ADHYQQNTPIGDGPVLLPDNHYLSTQSALSKDPNEKRDHMVLLEFVTAARMHDQLTEEQIAEFKE

AFSLFDKDGDGTITTKELGTVMRSLGQNPTEAELQDMINEVDADGNGTIYFPEFLTMMARKMKDT

DSEEEIREAFRVFDKDGNGYISAAQLRHVMTNLGEKLTDEEVDEMIREADIDGDGQVNYEEFVQM

MTAKGGKRRWKKNFIAVSAANRFKKISELMVSKGEELFTGVVPILVELDGDVNGHKFSVSGEGEG

DATYGKLTLKFICTTGKLPVPWPTLVTTLTYGVQCFSRYPDHMKQHDFFKSAMPEGYVQERTIFF

KDDGNYKTRAEVKFEGDTLVNRIELKGIDFKEDGNILGHKLEYNYNSHNVYIMADKQKNGIKVNF

KIRHNIEDGSVQLADHYQQNTPIGDGPVLLPDNHYLSTQSALSKDPNEKRDHMVLLEFVTAAGIT

LGKDEL*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5439797 A
- US 4603209 A
- US 5049673 A
- US 4868103 A
- US 08337915 B
- US 9514692 W
- US 08706408 B

- US 4683195 A
- US 33791594 A
- US 4215051 A, Smith
- US 08818252 B
- US 08818253 B
- US 08919143 B

### Non-patent literature cited in the description

- **PORUMB, T. et al.** *Prot.Engineering,* 1994, vol. 7, 109-115
- **SPATOLA, A.F.** Chemistry and Biochemistry of Amino Acids, Peptides and Proteins. Marcel Dekker, 1983, 267
- **ORMO, M. et al.** *Science,* 1996, vol. 273, 1392-1395
- **FORSTER, T.** *Ann.Physik,* 1948, vol. 2, 55-75
- **BERLMAN, I.B.** Energy transfer parameters of aromatic compounds. Academic Press, 1973
- **ADAMS, S.R. et al.** *Nature,* 1991, vol. 349, 694-697
- **GONZALEZ, J. ; TSIEN, R.Y.** *Biophy. J.,* 1995, vol. 69, 1272-1280
- **LAKOWICZ, J.R.** Principles of Fluorescence Spectroscopy. Plenum Press, 1983
- Resonance energy transfer microscopy, in: Fluorescence Microscopy of Living Cells in Culture. **HERMAN, B.** Methods in Cell Biology. Academic Press, 1989, vol. 30, 219-243
- **TURRO, N.J.** Modern Molecular Photochemistry. Benjamin/Cummings Publishing Col, Inc, 1978, 296-361
- Standards in Fluorescence Spectrometry. Chapman and Hall, 1981
- **WARD, W.W. et al.** *Photochem. Photobiol.,* 1982, vol. 35, 803-808
- **LEVINE, L.D. et al.** *Comp. Biochem. Physiol.,* 1982, vol. 72B, 77-85
- **PRASHER, D.C. et al.** *Gene,* 1992, vol. 111, 229-233
- **HEIM, R. et al.** *Proc. Natl. Acad Sci., USA,* 1994, vol. 91, 12501-04
- **CUBITT, A.B. et al.** *Trends Biochem. Sci.,* 1995, vol. 20, 448-455
- **HEIM, R. ; TSIEN, R.Y.** *Current Biol.,* 1996, vol. 6, 178-182

- **TSIEN, R.Y. et al.** *Trends Cell Biol.,* 1993, vol. 3, 242-245
- **BALDWIN, T.O. et al.** *Biochemistry,* 1990, vol. 29, 5509-5515
- **MORRIS, B.J. et al.** *Plant Molecular Biology,* 1994, vol. 24, 673-677
- **WILBANKS, S.M. et al.** *J. Biol. Chem.,* 1993, vol. 268, 1226-1235
- **LI et al.** *Biochemistry,* 1995, vol. 34, 7923-7930
- **KATZENELLENBOGEN, J.A. ; KATZENELLENBOGEN, B.S.** *Chemistry & Biology,* 1996, vol. 3, 529-536
- **AMES, J.B. et al.** *Curr. Opin. Struct. Biol.,* 1996, vol. 6, 432-438
- **FALKE, J.J. et al.** *Quart. Rev. Biophys.,* 1994, vol. 27, 219-290
- **CRIVICI, A. ; IKURA, M.** *Annu. Rev. Biophys Biomol. Struct.,* 1995, vol. 24, 84-116
- **HUSTON, J.S. et al.** *PNAS,* 1988, vol. 85, 5879-5883
- **WHITLOW, M. et al.** *Protein Engineering,* 1993, vol. 6, 989-995
- **NEWTON, D.L.** *Biochemistry,* 1996, vol. 35, 545-553
- Protein Targeting. **STRYER, L.** Biochemistry. W.H. Freeman, 1995
- **MULLIS et al.** *Cold Spring Harbor Symp. Quant. Biol.,* 1987, vol. 51, 263
- PCR Technology. Stockton Press, 1989
- **SAMBROOK et al.** Molecular Cloning - A Laboratory Manual. Cold Spring Harbor Laboratory, 1989
- Current Protocols in Molecular Biology. Greene Publishing Associates, Inc. and John Wiley & Sons, Inc,
- **MANIATIS et al.** Molecular Cloning A. Laboratory Manual. Cold Spring Harbor Laboratory, 1989

- Eukaryotic Viral Vectors. Cold Spring Harbor Laboratory, 1982
- **BITTER et al.** *Methods in Enzymology,* 1987, vol. 153, 516-544
- Current Protocols in Molecular Biology. Greene Publish. Assoc. & Wiley Interscience, 1988, vol. 2
- Expression and Secretion Vectors for Yeast. **GRANT et al.** Methods in Enzymology. Acad. Press, 1987, vol. 153, 516-544
- **GLOVER.** DNA Cloning. IRL Press, 1986, vol. II
- Heterologous Gene Expression in Yeast. **BITTER.** Methods in Enzymology. Acad. Press, 1987, vol. 152, 673-684
- The Molecular Biology of the Yeast Saccharomyces. Cold Spring Harbor Press, 1982, vol. I,II
- A Practical Approach. **R ROTHSTEIN.** DNA Cloning. IRL Press, 1986, vol. 1
- **BRISSON.** *Nature,* 1984, vol. 310, 511-514
- **TAKAMATSU et al.** *EMBO J.,* 1987, vol. 6, 307-311
- **CORUZZI et al.** *EMBO J.,* 1984, vol. 3, 1671-1680
- **BROGLIE et al.** *Science,* 1984, vol. 224, 838-843
- **GURLEY et al.** *Mol. Cell. Biol.,* 1986, vol. 6, 559-565
- **WEISSBACH ; WEISSBACH.** Methods for Plant Molecular Biology. Academic Press, 1988, vol. VIII, 421-463
- **GRIERSON ; COREY.** Plant Molecular Biology. Blackie, 1988
- **SMITH et al.** *J. Viol.,* 1983, vol. 46, 584
- **LOGAN ; SHENK.** *Proc. Natl. Acad Sci. USA,* 1984, vol. 81, 3655-3659
- **MACKETT et al.** *Proc. Natl. Acad Sci. USA,* 1982, vol. 79, 7415-7419
- **MACKETT et al.** *J. Virol.,* 1984, vol. 49, 857-864
- **PANICALI et al.** *Proc. Natl. Acad Sci. USA,* 1982, vol. 79, 4927-4931
- **SARVER et al.** *Mol. Cell. Biol.,* 1981, vol. 1, 486
- **CONE ; MULLIGAN.** *Proc. Natl. Acad Sci. USA,* 1984, vol. 81, 6349-6353
- **WIGLER et al.** *Cell,* 1977, vol. 11, 223
- **SZYBALSKA ; SZYBALSKI.** *Proc. Natl. Acad Sci. USA,* 1962, vol. 48, 2026
- **LOWY et al.** *Cell,* 1980, vol. 22, 817
- **WIGLER et al.** *Proc. Natl. Acad. Sci. USA,* 1980, vol. 77, 3567
- **O'HARE et al.** *Proc. Natl. Acad. Sci. USA,* 1981, vol. 8, 1527
- **MULLIGAN ; BERG.** *Proc. Natl. Acad. Sci. USA,* 1981, vol. 78, 2072
- **COLBERRE-GARAPIN et al.** *J. Mol. Biol.,* 1981, vol. 150, 1
- **SANTERRE et al.** *Gene,* 1984, vol. 30, 147
- **HARTMAN ; MULLIGAN.** *Proc. Natl. Acad. Sci. USA,* 1988, vol. 85, 8047
- **MCCONLOGUE L.** Current Communications in Molecular Biology. Cold Spring Harbor Laboratory, 1987
- **BRINSTER et al.** *Proc. Natl. Acad. Sci. USA,* 1985, vol. 82, 4438-4442
- **JAENICH, R.** *Proc. Natl. Acad Sci USA,* 1976, vol. 73, 1260-1264
- **HOGAN et al.** Manipulating the Mouse Embryo. Cold Spring Harbor Laboratory Press, 1986
- **JAHNER et al.** *Proc. Natl. Acad Sci. USA,* 1985, vol. 82, 6927-6931
- **VAN DER PUTTEN et al.** *Proc. Natl. Acad Sci USA,* 1985, vol. 82, 6148-6152
- **STEWART et al.** *EMBO J.,* 1987, vol. 6, 383-388
- **D. JAHNER et al.** *Nature,* 1982, vol. 298, 623-628
- **M. J. EVANS et al.** *Nature,* 1981, vol. 292, 154-156
- **M.O. BRADLEY et al.** *Nature,* 1984, vol. 309, 255-258
- **GOSSLER et al.** *Proc. Natl. Acad Sci USA,* 1986, vol. 83, 9065-9069
- **ROBERTSON et al.** *Nature,* 1986, vol. 322, 445-448
- **JAENISCH, R.** *Science,* 1988, vol. 240, 1468-1474
- **PORUMB, T. et al.** *Prot. Engineering,* 1994, vol. 7, 109-115
- **MAUNE, J.F. et al.** *J.Biol.Chem.,* 1992, vol. 267, 5286-5295
- **GAO, Z.H. et al.** *J.Biol.Chem.,* 1993, vol. 268, 20096-20104
- **M. KOZAK.** *J. Cell Biol.,* 1989, vol. 108, 229-241
- **TSIEN, R.Y. ; HAROOTUNIAN, A.T.** *Cell Calcium,* 1990, vol. 11, 93-109
- **ADAMS, S.R. et al.** Fluorescent and Luminescent Probes for Biological Activity. Academic Press, 1993
- **PORUMB, T. et al.** *Prot. Engineering,* 1994, vol. 7 (109-1), 15
- Molecular Cloning, A Laboratory Manual. CSH Laboratory Press, 1989
- **FORBES, D.J.** *Ann.Rev.Cell Biol.,* 1992, vol. 8, 495-527
- **KENDALL, J.M. et al.** *Biochem. Biophys. Res. Commun.,* 1992, vol. 189, 1008-1016
- **GRZEGORZ, G. et al.** *J. Biol. Chem.,* 1985, vol. 260, 3440-3450
- **TSIEN, R.Y. ; BACKSKAI, B.J.** Handbook of Biological Confocal Microscopy. Plenum Press, 1995, 459-478